# EUROPEAN PATENT APPLICATION

(11) **EP 1 705 248 A1**
(43) Date of publication of application: **27.09.2006**
(21) Application number: 05019787.0
(22) Date of filing: 30.09.1998
(51) Int. Cl.: C12N 15/62, C12N 9/68, C07K 14/56, C07K 14/78, C07K 14/52, A61K 48/00, A61K 38/17, A61K 38/19

(54) **Fusion proteins comprising an angiostatin moiety and their use in anti-tumour treatment**

(62) Divisional of application: 98950775.1
(71) Applicant: G.D. Searle LLC., Chicago, Illinois 60680-5110 (US)
(72) Inventor: Bolanowski, Mark A., St. Charles, MO 63304 (US); Caparon, Maire H., Chesterfield, MO 63017 (US); Casperson, Gerald F., Ballwin, MO 63011 (US); Gregory, Susan A., University City, MO 63130 (US); Klein, Barbara K., St. Louis, MO 63131 (US); McKearn, John P., Glencoe, MO 63038 (US)
(74) Representative: Strych, Werner Maximilian Josef

(57) **Abstract**

The present invention relates to the development of novel multi-functional proteins (MFPs) of the formula: R₁-L₁-R₂, R₂-L₁-R₁, R₁-L₁-R₁, R₁-R₂, R₂-R₁, or R₁-R₁ where R₁ is angiostatin, R₂ is selected from endostatin, human type I interferon, thrombospondin, interferon-inducible protein 10, platelet factor 4, and L₁ is a linker capable of linking R₁ to R₂. The invention is also drawn to nucleic acids that encode MFPs, procedures for manufacturing MFPs, methods of using MFPs, methods of treating angiogenic-mediated disease with MFPs including methods of treating tumors with MFPs, and treating tumors by gene therapy using vectors that encode MFPs.

SEQUENCE LISTING

<110> Bolanowski, Mark A.
Caparon, Maire H.
Casperson, Gerald F.
Gregory, Susan A.
Klein, Barbara K.
McKearn, John P.

<120> Multi-functional Proteins

<130> C-3026

<150> 60/060,609
<151> 1997-10-02

<160> 113

<170> FastSEQ for Windows Version 3.0

<210> 1
<211> 32
<212> DNA
<213> human

<400> 1
gatcgaccat ggcttgtgat ctgcctcaaa cc 32

<210> 2
<211> 36
<212> DNA
<213> human

<400> 2
cgatcgaagc ttattattcc ttacttctta aacttt 36

<210> 3
<211> 115
<212> DNA
<213> human

<400> 3
gatcgaccat ggcttgtgat ctgccgcaaa ctcatagcct gggtagccgt cgcaccctga 60
tgctgctggc tcagatgcgc cgtatctctc ttttctcctg cttgaaggac agaca 115

<210> 4
<211> 61
<212> DNA
<213> human

<400> 4
ccggggagcc tccaccgccc tctacgtatt ccttacgacg cagacgttct tgcaggttgg 60
t 61

<210> 5
<211> 79
<212> DNA
<213> human

<400> 5
ccggggagcc tccaccgccc tctacgtatt ccttacttct taaactttct tgcaagtttg 60
ttgacaaaga aaaagatct 79

<210> 6
<211> 79
<212> DNA
<213> human

<400> 6
catgagatct ttttctttgt caacaaactt gcaagaaagt ttaagaagta aggaatacgt 60
agagggcggt ggaggctcc 79

<210> 7
<211> 38
<212> DNA
<213> human

<400> 7
ccggggagcc tccaccgccc tctacgtatt cctcttca 38

<210> 8
<211> 37
<212> DNA
<213> human

<400> 8
gtgtgaagag gaatacgtag agggcggtgg aggctcc 37

<210> 9
<211> 32
<212> DNA
<213> human

<400> 9
ccggggagcc tccaccgccc tctacgtaag cc 32

<210> 10
<211> 32
<212> DNA
<213> human

<400> 10
aattggctta cgtagagggc ggtggaggct cc 32

<210> 11
<211> 80
<212> DNA
<213> human

<400> 11
gatcttcaac ctgtttacca ccaaagattc ttctgctgct tgggatgagg acctgctgga 60
caaattctgc accgaactgt 80

<210> 12
<211> 84
<212> DNA
<213> human

<400> 12
ctgctggtac agttcggtgc agaatttgtc cagcaggtcc tcatcccaag cagcagaaga 60
atctttggtg gtaaacaggt tgaa 84

<210> 13
<211> 80
<212> DNA
<213> human

<400> 13
accagcagct gaacgacctc gaagcctgtg tgatgcagga ggagcgtgtg ggtgaaactc 60
cgctgatgaa cgtggactcc 80

<210> 14
<211> 80
<212> DNA
<213> human

<400> 14
gccaggatgg agtccacgtt catcagcgga gtttcaccca cacgctcctc ctgcatcaca 60
caggcttcga ggtcgttcag 80

<210> 15
<211> 80
<212> DNA
<213> human

<400> 15
atcctggctg tgaagaaata cttccgtcgt atcactctgt atctgactga gaagaaatac 60
agcccgtgtg cttgggaggt 80

<210> 16
<211> 80
<212> DNA
<213> human

<400> 16
cacgaacaac ctcccaagca cacgggctgt atttcttctc agtcagatac agagtgatac 60
gacggaagta tttcttcaca 80

<210> 17
<211> 75
<212> DNA
<213> human

<400> 17
tgttcgtgct gaaatcatgc gttccctgtc tctgtctacc aacctgcaag aacgtctgcg 60
tcgtaaggaa taata 75

<210> 18
<211> 71
<212> DNA
<213> human

<400> 18
agcttattat tccttacgac gcagacgttc ttgcaggttg gtagacagag acagggaacg 60
catgatttca g 71

<210> 19
<211> 29
<212> DNA
<213> human

<400> 19
ggaaaaaggg ccccttggtg ccatacaac 29

<210> 20
<211> 29
<212> DNA
<213> human

<400> 20
   gttgtatggc accaaggggc cctttttcc 29

<210> 21
<211> 30
<212> DNA
<213> human

<400> 21
catgccatgg cagtttatct ttcagagtgt 30

<210> 22
<211> 34
<212> DNA

<213> human

<400> 22
gatcaagctt ttattaagcc aattgttccg tgga                                34

<210> 23
<211> 32
<212> DNA
<213> human

<400> 23
aattggctta cgtagagggc ggtggaggct cc                                  32

<210> 24
<211> 32
<212> DNA
<213> human

<400> 24
ccggggagcc tccaccgccc tctacgtaag cc                                  32

<210> 25
<211> 59
<212> DNA
<213> human

<400> 25
ctaccaacct gcaagaacgt ctgcgtcgta aggaatacgt agagggcggt ggaggctcc     59

<210> 26
<211> 61
<212> DNA
<213> human

<400> 26
ccggggagcc tccaccgccc tctacgtatt ccttacgacg cagacgttct tgcaggttgg    60
t                                                                    61

<210> 27
<211> 507
<212> DNA
<213> human

<400> 27
atggcttgtg atctgcctca aacccacagc ctgggtagca ggaggacctt gatgctcctg    60
gcacagatga ggagaatctc tcttttctcc tgcttgaagg acagacatga ctttggattt   120
ccccaggagg agtttggcaa ccagttccaa aaggctgaaa ccatccctgt cctccatgag   180
atgatccagc agatcttcaa tctcttcagc acaaaggact catctgctgc ttgggatgag   240
accctcctag acaaattcta cactgaactc taccagcagc tgaatgacct ggaagcctgt   300
gtgatacagg gggtgggggt gacagagact cccctgatga aggaggactc cattctggct   360
gtgaggaaat acttccaaag aatcactctc tatctgaaag agaagaaata cagcccttgt   420
gcctgggagg ttgtcagagc agaaatcatg agatcttttt ctttgtcaac aaacttgcaa   480
gaaagtttaa gaagtaagga ataataa                                       507

<210> 28
<211> 507
<212> DNA
<213> human

<400> 28
atggcttgtg atctgccgca aactcatagc ctgggtagcc gtcgcaccct gatgctgctg    60
gctcagatgc gccgtatctc tcttttctcc tgcttgaagg acagacatga ctttggattt   120
ccccaggagg agtttggcaa ccagttccaa aaggctgaaa ccatccctgt cctccatgag   180
atgatccagc agatcttcaa tctcttcagc acaaaggact catctgctgc ttgggatgag   240
accctcctag acaaattcta cactgaactc taccagcagc tgaatgacct ggaagcctgt   300
gtgatacagg gggtgggggt gacagagact cccctgatga aggaggactc cattctggct   360
gtgaggaaat acttccaaag aatcactctc tatctgaaag agaagaaata cagcccttgt   420
gcctgggagg ttgtcagagc agaaatcatg agatcttttt ctttgtcaac aaacttgcaa   480

gaaagtttaa gaagtaagga ataataa                                          507

<210> 29
<211> 822
<212> DNA
<213> human

<400> 29
atggcttgtg atctgccgca aactcatagc ctgggtagcc gtcgcaccct gatgctgctg      60
gctcagatgc gccgtatctc tcttttctcc tgcttgaagg acagacatga ctttggattt     120
ccccaggagg agtttggcaa ccagttccaa aaggctgaaa ccatccctgt cctccatgag     180
atgatccagc agatcttcaa cctgtttacc accaaagatt cttctgctgc ttgggatgag     240
gacctgctgg acaaattctg caccgaactg taccagcagc tgaacgacct cgaagcctgt     300
gtgatgcagg aggagcgtgt gggtgaaact ccgctgatga acgtggactc catcctggct     360
gtgaagaaat acttccgtcg tatcactctg tatctgactg agaagaaata cagcccgtgt     420
gcttgggagg ttgttcgtgc tgaaatcatg cgttccctgt ctctgtctac caacctgcaa     480
gaacgtctgc gtcgtaagga atacgtagag ggcggtggag gctccccggg tggtggttct     540
ggcggcggct ccaacatggc agtttatctt tcagagtgta agactgggaa tggaaagaat     600
tacagaggga cgatgtccaa aacaaaaaat ggcatcacct gtcaaaaatg gagttccact     660
tctccccaca gacctagatt ctcacctgct acacacccct cagagggact ggaggagaac     720
tactgcagga atccagacaa cgatccgcag gggccctggt gctatactac tgatccagaa     780
aagagatatg actactgcga cattcttgag tgtgaagagg aa                        822

<210> 30
<211> 1359
<212> DNA
<213> human

<400> 30
atggcttgtg atctgccgca aactcatagc ctgggtagcc gtcgcaccct gatgctgctg      60
gctcagatgc gccgtatctc tcttttctcc tgcttgaagg acagacatga ctttggattt     120
ccccaggagg agtttggcaa ccagttccaa aaggctgaaa ccatccctgt cctccatgag     180
atgatccagc agatcttcaa cctgtttacc accaaagatt cttctgctgc ttgggatgag     240
gacctgctgg acaaattctg caccgaactg taccagcagc tgaacgacct cgaagcctgt     300
gtgatgcagg aggagcgtgt gggtgaaact ccgctgatga acgtggactc catcctggct     360
gtgaagaaat acttccgtcg tatcactctg tatctgactg agaagaaata cagcccgtgt     420
gcttgggagg ttgttcgtgc tgaaatcatg cgttccctgt ctctgtctac caacctgcaa     480
gaacgtctgc gtcgtaagga atacgtagag ggcggtggag gctccccggg tggtggttct     540
ggcggcggct ccaacatggc agtttatctt tcagagtgta agactgggaa tggaaagaat     600
tacagaggga cgatgtccaa aacaaaaaat ggcatcacct gtcaaaaatg gagttccact     660
tctccccaca gacctagatt ctcacctgct acacacccct cagagggact ggaggagaac     720
tactgcagga atccagacaa cgatccgcag gggccctggt gctatactac tgatccagaa     780
aagagatatg actactgcga cattcttgag tgtgaagagg aatgtatgca ttgcagtgga     840
gaaaactatg acggcaaaat ttccaagacc atgtctggac tggaatgcca ggcctgggac     900
tctcagagcc cacacgctca tggatacatt ccttccaaat ttccaaacaa gaacctgaag     960
aagaattact gtcgtaaccc cgatagggag ctgcggcctt ggtgtttcac caccgacccc    1020
aacaagcgct gggaactttg tgacatcccc cgctgcacaa cacctccacc atcttctggt    1080
cccacctacc agtgtctgaa gggaacaggt gaaaactatc gcgggaatgt ggctgttacc    1140
gtgtccgggc acacctgtca gcactggagt gcacagaccc ctcacacaca taacaggaca    1200
ccagaaaact tcccctgcaa aaatttggat gaaaactact gccgcaatcc tgacggaaaa    1260
agggcccctt ggtgccatac aaccaacagc caagtgcggt gggagtactg taagataccg    1320
tcctgtgact cctccccagt atccacggaa caattggct                           1359

<210> 31
<211> 822
<212> DNA
<213> human

<400> 31
atggcttgtg atctgccgca aactcatagc ctgggtagcc gtcgcaccct gatgctgctg      60
gctcagatgc gccgtatctc tcttttctcc tgcttgaagg acagacatga ctttggattt     120
ccccaggagg agtttggcaa ccagttccaa aaggctgaaa ccatccctgt cctccatgag     180
atgatccagc agatcttcaa tctcttcagc acaaaggact catctgctgc ttgggatgag     240
accctcctag acaaattcta cactgaactc taccagcagc tgaatgacct ggaagcctgt     300
gtgatacagg gggtgggggt gacagagact cccctgatga aggaggactc cattctggct     360
gtgaggaaat acttccaaag aatcactctc tatctgaaag agaagaaata cagcccttgt     420
gcctgggagg ttgtcagagc agaaatcatg agatcttttt ctttgtcaac aaacttgcaa     480
gaaagtttaa gaagtaagga atacgtagag ggcggtggag gctccccggg tggtggttct     540

ggcggcggct ccaacatggc agtttatctt tcagagtgta agactgggaa tggaaagaat 600
tacagaggga cgatgtccaa aacaaaaaat ggcatcacct gtcaaaaatg gagttccact 660
tctccccaca gacctagatt ctcacctgct acacacccct cagagggact ggaggagaac 720
tactgcagga atccagacaa cgatccgcag gggccctggt gctatactac tgatccagaa 780
aagagatatg actactgcga cattcttgag tgtgaagagg aa 822

<210> 32
<211> 1359
<212> DNA
<213> human

<400> 32
atggcttgtg atctgccgca aactcatagc ctgggtagcc gtcgcaccct gatgctgctg 60
gctcagatgc gccgtatctc tcttttctcc tgcttgaagg acagacatga ctttggattt 120
ccccaggagg agtttggcaa ccagttccaa aaggctgaaa ccatccctgt cctccatgag 180
atgatccagc agatcttcaa tctcttcagc acaaaggact catctgctgc ttgggatgag 240
accctcctag acaaattcta cactgaactc taccagcagc tgaatgacct ggaagcctgt 300
gtgatacagg gggtgggggt gacagagact cccctgatga aggaggactc cattctggct 360
gtgaggaaat acttccaaag aatcactctc tatctgaaag agaagaaata cagcccttgt 420
gcctgggagg ttgtcagagc agaaatcatg agatcttttt ctttgtcaac aaacttgcaa 480
gaaagtttaa gaagtaagga atacgtagag ggcggtggag gctccccggg tggtggttct 540
ggcggcggct ccaacatggc agtttatctt tcagagtgta agactgggaa tggaaagaat 600
tacagaggga cgatgtccaa aacaaaaaat ggcatcacct gtcaaaaatg gagttccact 660
tctccccaca gacctagatt ctcacctgct acacacccct cagagggact ggaggagaac 720
tactgcagga atccagacaa cgatccgcag gggccctggt gctatactac tgatccagaa 780
aagagatatg actactgcga cattcttgag tgtgaagagg aatgtatgca ttgcagtgga 840
gaaaactatg acggcaaaat ttccaagacc atgtctggac tggaatgcca ggcctgggac 900
tctcagagcc cacacgctca tggatacatt ccttccaaat ttccaaacaa gaacctgaag 960
aagaattact gtcgtaaccc cgatagggag ctgcggcctt ggtgtttcac caccgacccc 1020
aacaagcgct gggaactttg tgacatcccc cgctgcacaa cacctccacc atcttctggt 1080
cccacctacc agtgtctgaa gggaacaggt gaaaactatc gcgggaatgt ggctgttacc 1140
gtgtccgggc acacctgtca gcactggagt gcacagaccc ctcacacaca taacaggaca 1200
ccagaaaact tcccctgcaa aaatttggat gaaaactact gccgcaatcc tgacggaaaa 1260
agggcccctt ggtgccatac aaccaacagc caagtgcggt gggagtactg taagataccg 1320
tcctgtgact cctccccagt atccacggaa caattggct 1359

<210> 33
<211> 1359
<212> DNA
<213> human

<400> 33
atggcagttt atctttcaga gtgtaagact gggaatggaa agaattacag agggacgatg 60
tccaaaacaa aaaatggcat cacctgtcaa aaatggagtt ccacttctcc ccacagacct 120
agattctcac ctgctacaca cccctcagag ggactggagg agaactactg caggaatcca 180
gacaacgatc cgcaggggcc ctggtgctat actactgatc cagaaaagag atatgactac 240
tgcgacattc ttgagtgtga agaggaatgt atgcattgca gtggagaaaa ctatgacggc 300
aaaatttcca agaccatgtc tggactggaa tgccaggcct gggactctca gagcccacac 360
gctcatggat acattccttc caaatttcca aacaagaacc tgaagaagaa ttactgtcgt 420
aaccccgata gggagctgcg gccttggtgt ttcaccaccg accccaacaa gcgctgggaa 480
ctttgtgaca tcccccgctg cacaacacct ccaccatctt ctggtcccac ctaccagtgt 540
ctgaagggaa caggtgaaaa ctatcgcggg aatgtggctg ttaccgtgtc cgggcacacc 600
tgtcagcact ggagtgcaca gacccctcac acacataaca ggacaccaga aaacttcccc 660
tgcaaaaatt tggatgaaaa ctactgccgc aatcctgacg gaaaaagggc cccttggtgc 720
catacaacca acagccaagt gcggtgggag tactgtaaga taccgtcctg tgactcctcc 780
ccagtatcca cggaacaatt ggcttacgta gagggcggtg gaggctcccc gggtggtggt 840
tctggcggcg gctccaacat ggcttgtgat ctgccgcaaa ctcatagcct gggtagccgt 900
cgcaccctga tgctgctggc tcagatgcgc cgtatctctc ttttctcctg cttgaaggac 960
agacatgact ttggatttcc ccaggaggag tttggcaacc agttccaaaa ggctgaaacc 1020
atccctgtcc tccatgagat gatccagcag atcttcaatc tcttcagcac aaaggactca 1080
tctgctgctt gggatgagac cctcctagac aaattctaca ctgaactcta ccagcagctg 1140
aatgacctgg aagcctgtgt gatacagggg gtgggggtga cagagactcc cctgatgaag 1200
gaggactcca ttctggctgt gaggaaatac ttccaaagaa tcactctcta tctgaaagag 1260
aagaaataca gcccttgtgc ctgggaggtt gtcagagcag aaatcatgag atctttttct 1320
ttgtcaacaa acttgcaaga aagtttaaga agtaaggaa 1359

<210> 34
<211> 822

<212> DNA
<213> human

<400> 34
atggcagttt atctttcaga gtgtaagact gggaatggaa agaattacag agggacgatg 60
tccaaaacaa aaaatggcat cacctgtcaa aaatggagtt ccacttctcc ccacagacct 120
agattctcac ctgctacaca cccctcagag ggactggagg agaactactg caggaatcca 180
gacaacgatc cgcaggggcc ctggtgctat actactgatc cagaaaagag atatgactac 240
tgcgacattc ttgagtgtga agaggaatac gtagagggcg gtggaggctc cccgggtggt 300
ggttctggcg gcggctccaa catggcttgt gatctgccgc aaactcatag cctgggtagc 360
cgtcgcaccc tgatgctgct ggctcagatg cgccgtatct ctcttttctc ctgcttgaag 420
gacagacatg actttggatt tccccaggag gagtttggca accagttcca aaaggctgaa 480
accatccctg tcctccatga gatgatccag cagatcttca acctgtttac caccaaagat 540
tcttctgctg cttgggatga ggacctgctg gacaaattct gcaccgaact gtaccagcag 600
ctgaacgacc tcgaagcctg tgtgatgcag gaggagcgtg tgggtgaaac tccgctgatg 660
aacgtggact ccatcctggc tgtgaagaaa tacttccgtc gtatcactct gtatctgact 720
gagaagaaat acagcccgtg tgcttgggag gttgttcgtg ctgaaatcat gcgttccctg 780
tctctgtcta ccaacctgca agaacgtctg cgtcgtaagg aa 822

<210> 35
<211> 822
<212> DNA
<213> human

<400> 35
atggcagttt atctttcaga gtgtaagact gggaatggaa agaattacag agggacgatg 60
tccaaaacaa aaaatggcat cacctgtcaa aaatggagtt ccacttctcc ccacagacct 120
agattctcac ctgctacaca cccctcagag ggactggagg agaactactg caggaatcca 180
gacaacgatc cgcaggggcc ctggtgctat actactgatc cagaaaagag atatgactac 240
tgcgacattc ttgagtgtga agaggaatac gtagagggcg gtggaggctc cccgggtggt 300
ggttctggcg gcggctccaa catggcttgt gatctgccgc aaactcatag cctgggtagc 360
cgtcgcaccc tgatgctgct ggctcagatg cgccgtatct ctcttttctc ctgcttgaag 420
gacagacatg actttggatt tccccaggag gagtttggca accagttcca aaaggctgaa 480
accatccctg tcctccatga gatgatccag cagatcttca atctcttcag cacaaaggac 540
tcatctgctg cttgggatga gaccctccta gacaaattct acactgaact ctaccagcag 600
ctgaatgacc tggaagcctg tgtgatacag ggggtggggg tgacagagac tcccctgatg 660
aaggaggact ccattctggc tgtgaggaaa tacttccaaa gaatcactct ctatctgaaa 720
gagaagaaat acagcccttg tgcctgggag gttgtcagag cagaaatcat gagatctttt 780
tctttgtcaa caaacttgca agaaagttta agaagtaagg aa 822

<210> 36
<211> 1359
<212> DNA
<213> human

<400> 36
atggcagttt atctttcaga gtgtaagact gggaatggaa agaattacag agggacgatg 60
tccaaaacaa aaaatggcat cacctgtcaa aaatggagtt ccacttctcc ccacagacct 120
agattctcac ctgctacaca cccctcagag ggactggagg agaactactg caggaatcca 180
gacaacgatc cgcaggggcc ctggtgctat actactgatc cagaaaagag atatgactac 240
tgcgacattc ttgagtgtga agaggaatgt atgcattgca gtggagaaaa ctatgacggc 300
aaaatttcca agaccatgtc tggactggaa tgccaggcct gggactctca gagcccacac 360
gctcatggat acattccttc caaatttcca aacaagaacc tgaagaagaa ttactgtcgt 420
aaccccgata gggagctgcg gccttggtgt ttcaccaccg accccaacaa gcgctgggaa 480
ctttgtgaca tcccccgctg cacaacacct ccaccatctt ctggtcccac ctaccagtgt 540
ctgaagggaa caggtgaaaa ctatcgcggg aatgtggctg ttaccgtgtc cgggcacacc 600
tgtcagcact ggagtgcaca gacccctcac acacataaca ggacaccaga aaacttcccc 660
tgcaaaaatt tggatgaaaa ctactgccgc aatcctgacg gaaaaagggc cccttggtgc 720
catacaacca acagccaagt gcggtgggag tactgtaaga taccgtcctg tgactcctcc 780
ccagtatcca cggaacaatt ggcttacgta gagggcggtg gaggctcccc gggtggtggt 840
tctggcggcg gctccaacat ggcttgtgat ctgccgcaaa ctcatagcct gggtagccgt 900
cgcaccctga tgctgctggc tcagatgcgc cgtatctctc ttttctcctg cttgaaggac 960
agacatgact ttggatttcc ccaggaggag tttggcaacc agttccaaaa ggctgaaacc 1020
atccctgtcc tccatgagat gatccagcag atcttcaacc tgtttaccac caaagattct 1080
tctgctgctt gggatgagga cctgctggac aaattctgca ccgaactgta ccagcagctg 1140
aacgacctcg aagcctgtgt gatgcaggag gagcgtgtgg gtgaaactcc gctgatgaac 1200
gtggactcca tcctggctgt gaagaaatac ttccgtcgta tcactctgta tctgactgag 1260
aagaaataca gcccgtgtgc ttgggaggtt gttcgtgctg aaatcatgcg ttccctgtct 1320

ctgtctacca acctgcaaga acgtctgcgt cgtaaggaa 1359

<210> 37
<211> 1719
<212> DNA
<213> human

<400> 37
ggatccgcca ccatggaaca taaggaagtg gttcttctac ttcttttatt tctgaaatca 60
ggtcaaggag agcctctgga tgactatgtg aatacccagg gggcttcact gttcagtgtc 120
actaagaagc agctgggagc aggaagtata gaagaatgtg cagcaaaatg tgaggaggac 180
gaagaattca cctgcagggc attccaatat cacagtaaag agcaacaatg tgtgataatg 240
gctgaaaaca ggaagtcctc cataatcatt aggatgagag atgtagtttt atttgaaaag 300
aaagtgtatc tctcagagtg caagactggg aatggaaaga attacagagg gacgatgtcc 360
aaaacaaaaa atggcatcac ctgtcaaaaa tggagttcca cttctcccca cagacctaga 420
ttctcacctg ctacacaccc ctcagaggga ctggaggaga actactgcag gaatccagac 480
aacgatccgc aggggccctg gtgctatact actgatccag aaaagagata tgactactgc 540
gacattcttg agtgtgaaga ggaatgtatg cattgcagtg gagaaaacta tgacggcaaa 600
atttccaaga ccatgtctgg actggaatgc caggcctggg actctcagag cccacacgct 660
catggataca ttccttccaa atttccaaac aagaacctga agaagaatta ctgtcgtaac 720
cccgataggg agctgcggcc ttggtgtttc accaccgacc ccaacaagcg ctgggaactt 780
tgtgacatcc cccgctgcac aacacctcca ccatcttctg gtcccaccta ccagtgtctg 840
aagggaacag gtgaaaacta tcgcgggaat gtggctgtta ccgtgtccgg gcacacctgt 900
cagcactgga gtgcacagac ccctcacaca cataacagga caccagaaaa cttcccctgc 960
aaaaatttgg atgaaaacta ctgccgcaat cctgacggaa aaagggcccc atggtgccat 1020
acaaccaaca gccaagtgcg gtgggagtac tgtaagatac cgtcctgtga ctcctcccca 1080
gtatccacgg aacagttggc tcccacagca ccacctgagc taacccctgt ggtccaggac 1140
tgctaccatg gtgatggaca gagctaccga ggcacatcct ccaccaccac cacaggaaag 1200
aagtgtcagt cttggtcatc tatgacacca caccggcacc agaagacccc agaaaactac 1260
ccaaatgctg gcctgacaat gaactactgc aggaatccag atgccgataa aggcccctgg 1320
tgttttacca cagaccccag cgtcaggtgg gagtactgca acctgaaaaa atgctcagga 1380
acagaagcga gtgttgtagc acctccgcct gttgtcctgc ttccagatgt agagactcct 1440
tccgaagaag actgtatgtt tgggaatggg aaaggatacc gaggcaagag ggcgaccact 1500
gttactggga cgccatgcca ggactgggct gcccaggagc cccatagaca cagcattttc 1560
actccagaga caaatccacg ggcgggtctg gaaaaaaatt actgccgtaa ccctgatggt 1620
gatgtaggtg gtccctggtg ctacacgaca aatccaagaa aactttacga ctactgtgat 1680
gtccctcagt gtgcggcccc ttcatttgat taaggatcc 1719

<210> 38
<211> 1167
<212> DNA
<213> human

<400> 38
ggatccacca tgagccgcct gcccgtcctg ctcctgctcc aactcctggt ccgccccgcc 60
atgtctgtgt atctctcaga gtgcaagact gggaatggaa agaattacag agggacgatg 120
tccaaaacaa aaaatggcat cacctgtcaa aaatggagtt ccacttctcc ccacagacct 180
agattctcac ctgctacaca cccctcagag ggactggagg agaactactg caggaatcca 240
gacaacgatc cgcaggggcc ctggtgctat actactgatc cagaaaagag atatgactac 300
tgcgacattc ttgagtgtga agaggaatgt atgcattgca gtggagaaaa ctatgacggc 360
aaaatttcca agaccatgtc tggactggaa tgccaggcct gggactctca gagcccacac 420
gctcatggat acattccttc caaatttcca aacaagaacc tgaagaagaa ttactgtcgt 480
aaccccgata gggagctgcg gccttggtgt ttcaccaccg accccaacaa gcgctgggaa 540
ctttgtgaca tcccccgctg cacaacacct ccaccatctt ctggtcccac ctaccagtgt 600
ctgaagggaa caggtgaaaa ctatcgcggg aatgtggctg ttaccgtgtc cgggcacacc 660
tgtcagcact ggagtgcaca gacccctcac acacataaca ggacaccaga aaacttcccc 720
tgcaaaaatt tggatgaaaa ctactgccgc aatcctgacg gaaaaagggc cccatggtgc 780
catacaacca acagccaagt gcggtgggag tactgtaaga taccgtcctg tgactcctcc 840
ccagtatcca cggaacagtt ggctcccaca gcaccacctg agctaacccc tgtggtccag 900
gactgctacc atggtgatgg acagagctac cgaggcacat cctccaccac caccacagga 960
aagaagtgtc agtcttggtc atctatgaca ccacaccggc accagaagac cccagaaaac 1020
tacccaaatg ctggcctgac aatgaactac tgcaggaatc cagatgccga taaaggcccc 1080
tggtgtttta ccacagaccc cagcgtcagg tgggagtact gcaacctgaa aaaatgctca 1140
ggaacagaag cgtgaaagct tggatcc 1167

<210> 39
<211> 879
<212> DNA

<213> human

<400> 39
ggatccacca tgagccgcct gcccgtcctg ctcctgctcc aactcctggt ccgccccgcc 60
atgtctgtgt atctctcaga gtgcaagact gggaatggaa agaattacag agggacgatg 120
tccaaaacaa aaaatggcat cacctgtcaa aaatggagtt ccacttctcc ccacagacct 180
agattctcac ctgctacaca cccctcagag ggactggagg agaactactg caggaatcca 240
gacaacgatc cgcaggggcc ctggtgctat actactgatc cagaaaagag atatgactac 300
tgcgacattc ttgagtgtga agaggaatgt atgcattgca gtggagaaaa ctatgacggc 360
aaaatttcca agaccatgtc tggactggaa tgccaggcct gggactctca gagcccacac 420
gctcatggat acattccttc caaatttcca aacaagaacc tgaagaagaa ttactgtcgt 480
aaccccgata gggagctgcg gccttggtgt ttcaccaccg accccaacaa gcgctgggaa 540
ctttgtgaca tcccccgctg cacaacacct ccaccatctt ctggtcccac ctaccagtgt 600
ctgaagggaa caggtgaaaa ctatcgcggg aatgtggctg ttaccgtgtc cgggcacacc 660
tgtcagcact ggagtgcaca gacccctcac acacataaca ggacaccaga aaacttcccc 720
tgcaaaaatt tggatgaaaa ctactgccgc aatcctgacg gaaaaagggc cccatggtgc 780
catacaacca acagccaagt gcggtgggag tactgtaaga taccgtcctg tgactcctcc 840
ccagtatcca cggaacagtt ggcttgaaag cttggatcc 879

<210> 40
<211> 915
<212> DNA
<213> human

<400> 40
ggatccacca tgagccgcct gcccgtcctg ctcctgctcc aactcctggt ccgccccgcc 60
atgtctgaag aggaatgtat gcattgcagt ggagaaaact atgacggcaa aatttccaag 120
accatgtctg gactggaatg ccaggcctgg gactctcaga gcccacacgc tcatggatac 180
attccttcca aatttccaaa caagaacctg aagaagaatt actgtcgtaa ccccgatagg 240
gagctgcggc cttggtgttt caccaccgac cccaacaagc gctgggaact ttgtgacatc 300
ccccgctgca caacacctcc accatcttct ggtcccacct accagtgtct gaagggaaca 360
ggtgaaaact atcgcgggaa tgtggctgtt accgtgtccg ggcacacctg tcagcactgg 420
agtgcacaga cccctcacac acataacagg acaccagaaa acttcccctg caaaaatttg 480
gatgaaaact actgccgcaa tcctgacgga aaaagggccc catggtgcca tacaaccaac 540
agccaagtgc ggtgggagta ctgtaagata ccgtcctgtg actcctcccc agtatccacg 600
gaacagttgg ctcccacagc accacctgag ctaacccctg tggtccagga ctgctaccat 660
ggtgatggac agagctaccg aggcacatcc tccaccacca ccacaggaaa gaagtgtcag 720
tcttggtcat ctatgacacc acaccggcac cagaagaccc cagaaaacta cccaaatgct 780
ggcctgacaa tgaactactg caggaatcca gatgccgata aaggcccctg gtgttttacc 840
acagacccca gcgtcaggtg ggagtactgc aacctgaaaa aatgctcagg aacagaagcg 900
tgaaagcttg gatcc 915

<210> 41
<211> 627
<212> DNA
<213> human

<400> 41
ggatccacca tgagccgcct gcccgtcctg ctcctgctcc aactcctggt ccgccccgcc 60
atgtctgaag aggaatgtat gcattgcagt ggagaaaact atgacggcaa aatttccaag 120
accatgtctg gactggaatg ccaggcctgg gactctcaga gcccacacgc tcatggatac 180
attccttcca aatttccaaa caagaacctg aagaagaatt actgtcgtaa ccccgatagg 240
gagctgcggc cttggtgttt caccaccgac cccaacaagc gctgggaact ttgtgacatc 300
ccccgctgca caacacctcc accatcttct ggtcccacct accagtgtct gaagggaaca 360
ggtgaaaact atcgcgggaa tgtggctgtt accgtgtccg ggcacacctg tcagcactgg 420
agtgcacaga cccctcacac acataacagg acaccagaaa acttcccctg caaaaatttg 480
gatgaaaact actgccgcaa tcctgacgga aaaagggccc catggtgcca tacaaccaac 540
agccaagtgc ggtgggagta ctgtaagata ccgtcctgtg actcctcccc agtatccacg 600
gaacaattgg cttgaaagct tggatcc 627

<210> 42
<211> 1154
<212> DNA
<213> human

<400> 42
ccatgtcact gtttgaaaaa aaagtttatc tctcagagtg caagactggg aatggaaaga 60
attacagagg gacgatgtcc aaaacaaaaa atggcatcac ctgtcaaaaa tggagttcca 120

cttctcccca cagacctaga ttctcacctg ctacacaccc ctcagaggga ctggaggaga 180
actactgcag gaatccagac aacgatccgc aggggccctg gtgctatact actgatccag 240
aaaagagata tgactactgc gacattcttg agtgtgaaga ggaatgtatg cattgcagtg 300
gagaaaacta tgacggcaaa atttccaaga ccatgtctgg actggaatgc caggcctggg 360
actctcagag cccacacgct catggataca ttccttccaa atttccaaac aagaacctga 420
agaagaatta ctgtcgtaac cccgataggg agctgcggcc ttggtgtttc accaccgacc 480
ccaacaagcg ctgggaactt tgtgacatcc cccgctgcac aacacctcca ccatcttctg 540
gtcccaccta ccagtgtctg aagggaacag gtgaaaacta tcgcgggaat gtggctgtta 600
ccgtgtccgg gcacacctgt cagcactgga gtgcacagac ccctcacaca cataacagga 660
caccagaaaa cttcccctgc aaaaatttgg atgaaaacta ctgccgcaat cctgacggaa 720
aaagggcccc atggtgccat acaaccaaca gccaagtgcg gtgggagtac tgtaagatac 780
cgtcctgtga ctcctcccca gtatccacgg aacagttggc tcccacagca ccacctgagc 840
taacccctgt ggtccaggac tgctaccatg gtgatggaca gagctaccga ggcacatcct 900
ccaccaccac cacaggaaag aagtgtcagt cttggtcatc tatgacacca caccggcacc 960
agaagacccc agaaaactac ccaaatgctg gcctgacaat gaactactgc aggaatccag 1020
atgccgataa aggcccctgg tgttttacca cagaccccag cgtcaggtgg gagtactgca 1080
acctgaaaaa atgctcagga acagaagcga gtgttgtagc acctccgcct gttgtcctgc 1140
tttaataaaa gctt 1154

<210> 43
<211> 1106
<212> DNA
<213> human

<400> 43
ccatgtcagt gtatctgtcc gagtgtaaga ctgggaatgg aaagaattac agagggacga 60
tgtccaaaac aaaaaatggc atcacctgtc aaaaatggag ttccacttct ccccacagac 120
ctagattctc acctgctaca cacccctcag agggactgga ggagaactac tgcaggaatc 180
cagacaacga tccgcagggg ccctggtgct atactactga tccagaaaag agatatgact 240
actgcgacat tcttgagtgt gaagaggaat gtatgcattg cagtggagaa aactatgacg 300
gcaaaatttc caagaccatg tctggactgg aatgccaggc ctgggactct cagagcccac 360
acgctcatgg atacattcct tccaaatttc caaacaagaa cctgaagaag aattactgtc 420
gtaaccccga tagggagctg cggccttggt gtttcaccac cgaccccaac aagcgctggg 480
aactttgtga catcccccgc tgcacaacac ctccaccatc ttctggtccc acctaccagt 540
gtctgaaggg aacaggtgaa aactatcgcg ggaatgtggc tgttaccgtg tccgggcaca 600
cctgtcagca ctggagtgca cagacccctc acacacataa caggacacca gaaaacttcc 660
cctgcaaaaa tttggatgaa aactactgcc gcaatcctga cggaaaaagg gccccatggt 720
gccatacaac caacagccaa gtgcggtggg agtactgtaa gataccgtcc tgtgactcct 780
ccccagtatc cacggaacag ttggctccca cagcaccacc tgagctaacc cctgtggtcc 840
aggactgcta ccatggtgat ggacagagct accgaggcac atcctccacc accaccacag 900
gaaagaagtg tcagtcttgg tcatctatga caccacaccg gcaccagaag accccagaaa 960
actacccaaa tgctggcctg acaatgaact actgcaggaa tccagatgcc gataaaggcc 1020
cctggtgttt taccacagac cccagcgtca ggtgggagta ctgcaacctg aaaaaatgct 1080
caggaacaga agcgtaataa aagctt 1106

<210> 44
<211> 566
<212> DNA
<213> human

<400> 44
ccatgtctga agaagaatgc atgcactgca gtggagaaaa ctatgacggc aaaatttcca 60
agaccatgtc tggactggaa tgccaggcct gggactctca gagcccacac gctcatggat 120
acattccttc caaatttcca aacaagaacc tgaagaagaa ttactgtcgt aaccccgata 180
gggagctgcg gccttggtgt ttcaccaccg accccaacaa gcgctgggaa ctttgtgaca 240
tcccccgctg cacaacacct ccaccatctt ctggtcccac ctaccagtgt ctgaagggaa 300
caggtgaaaa ctatcgcggg aatgtggctg ttaccgtgtc cgggcacacc tgtcagcact 360
ggagtgcaca gacccctcac acacataaca ggacaccaga aaacttcccc tgcaaaaatt 420
tggatgaaaa ctactgccgc aatcctgacg gaaaaagggc cccatggtgc catacaacca 480
acagccaagt gcggtgggag tactgtaaga taccgtcctg tgactcctcc ccagtatcca 540
cggaacaatt ggcttaataa aagctt 566

<210> 45
<211> 854
<212> DNA
<213> human

<400> 45

ccatgtctga ggaagaatgc atgcactgca gtggagaaaa ctatgacggc aaaatttcca 60
agaccatgtc tggactggaa tgccaggcct gggactctca gagcccacac gctcatggat 120
acattccttc caaatttcca aacaagaacc tgaagaagaa ttactgtcgt aaccccgata 180
gggagctgcg gccttggtgt ttcaccaccg accccaacaa gcgctgggaa ctttgtgaca 240
tcccccgctg cacaacacct ccaccatctt ctggtcccac ctaccagtgt ctgaagggaa 300
caggtgaaaa ctatcgcggg aatgtggctg ttaccgtgtc cgggcacacc tgtcagcact 360
ggagtgcaca gacccctcac acacataaca ggacaccaga aaacttcccc tgcaaaaatt 420
tggatgaaaa ctactgccgc aatcctgacg gaaaaagggc cccatggtgc catacaacca 480
acagccaagt gcggtgggag tactgtaaga taccgtcctg tgactcctcc ccagtatcca 540
cggaacagtt ggctcccaca gcaccacctg agctaacccc tgtggtccag gactgctacc 600
atggtgatgg acagagctac cgaggcacat cctccaccac caccacagga aagaagtgtc 660
agtcttggtc atctatgaca ccacaccggc accagaagac cccagaaaac tacccaaatg 720
ctggcctgac aatgaactac tgcaggaatc cagatgccga taaaggcccc tggtgtttta 780
ccacagaccc cagcgtcagg tgggagtact gcaacctgaa aaaatgctca ggaacagaag 840
cgtaataaaa gctt 854

<210> 46
<211> 818
<212> DNA
<213> human

<400> 46
ccatgtcagt ttatctttca gagtgtaaga ctgggaatgg aaagaattac agagggacga 60
tgtccaaaac aaaaaatggc atcacctgtc aaaaatggag ttccacttct ccccacagac 120
ctagattctc acctgctaca cacccctcag agggactgga ggagaactac tgcaggaatc 180
cagacaacga tccgcagggg ccctggtgct atactactga tccagaaaag agatatgact 240
actgcgacat tcttgagtgt gaagaggaat gtatgcattg cagtggagaa aactatgacg 300
gcaaaatttc caagaccatg tctggactgg aatgccaggc ctgggactct cagagcccac 360
acgctcatgg atacattcct tccaaatttc caaacaagaa cctgaagaag aattactgtc 420
gtaaccccga tagggagctg cggccttggt gtttcaccac cgaccccaac aagcgctggg 480
aactttgtga catcccccgc tgcacaacac ctccaccatc ttctggtccc acctaccagt 540
gtctgaaggg aacaggtgaa aactatcgcg ggaatgtggc tgttaccgtg tccgggcaca 600
cctgtcagca ctggagtgca cagacccctc acacacataa caggacacca gaaaacttcc 660
cctgcaaaaa tttggatgaa aactactgcc gcaatcctga cggaaaaagg gccccatggt 720
gccatacaac caacagccaa gtgcggtggg agtactgtaa gataccgtcc tgtgactcct 780
ccccagtatc cacggaacaa ttggcttaat aaaagctt 818

<210> 47
<211> 281
<212> DNA
<213> human

<400> 47
ccatgtccgt gtacctgtca gaatgtaaga ctgggaatgg aaagaattac agagggacga 60
tgtccaaaac aaaaaatggc atcacctgtc aaaaatggag ttccacttct ccccacagac 120
ctagattctc acctgctaca cacccctcag agggactgga ggagaactac tgcaggaatc 180
cagacaacga tccgcagggg ccctggtgct atactactga tccagaaaag agatatgact 240
actgcgacat tcttgagtgt gaagaggaat aataaaagct t 281

<210> 48
<211> 345
<212> DNA
<213> human

<400> 48
ggatccacca tgagccgcct gcccgtcctg ctcctgctcc aactcctggt ccgccccgcc 60
atggccgtgt acctgtcaga atgtaagact gggaatggaa agaattacag agggacgatg 120
tccaaaacaa aaaatggcat cacctgtcaa aaatggagtt ccacttctcc ccacagacct 180
agattctcac ctgctacaca cccctcagag ggactggagg agaactactg caggaatcca 240
gacaacgatc cgcaggggcc ctggtgctat actactgatc cagaaaagag atatgactac 300
tgcgacattc ttgagtgtga agaggaataa taaaagcttg gattc 345

<210> 49
<211> 1436
<212> DNA
<213> human

<400> 49

ggatccacca tgagccgcct gcccgtcctg ctcctgctcc aactcctggt ccgccccgcc 60
atggcagttt atctttcaga gtgtaagact gggaatggaa agaattacag agggacgatg 120
tccaaaacaa aaaatggcat cacctgtcaa aaatggagtt ccacttctcc ccacagacct 180
agattctcac ctgctacaca cccctcagag ggactggagg agaactactg caggaatcca 240
gacaacgatc cgcaggggcc ctggtgctat actactgatc cagaaaagag atatgactac 300
tgcgacattc ttgagtgtga agaggaatgt atgcattgca gtggagaaaa ctatgacggc 360
aaaatttcca agaccatgtc tggactggaa tgccaggcct gggactctca gagcccacac 420
gctcatggat acattccttc caaatttcca aacaagaacc tgaagaagaa ttactgtcgt 480
aaccccgata gggagctgcg gccttggtgt ttcaccaccg accccaacaa gcgctgggaa 540
ctttgtgaca tcccccgctg cacaacacct ccaccatctt ctggtcccac ctaccagtgt 600
ctgaagggaa caggtgaaaa ctatcgcggg aatgtggctg ttaccgtgtc cgggcacacc 660
tgtcagcact ggagtgcaca gacccctcac acacataaca ggacaccaga aaacttcccc 720
tgcaaaaatt tggatgaaaa ctactgccgc aatcctgacg gaaaaagggc cccttggtgc 780
catacaacca acagccaagt gcggtgggag tactgtaaga taccgtcctg tgactcctcc 840
ccagtatcca cggaacagtt ggcttacgta gagggcggtg gaggctcccc gggtggtggt 900
tctggcggcg gctccaacat ggcttgtgat ctgccgcaaa ctcatagcct gggtagccgt 960
cgcaccctga tgctgctggc tcagatgcgc cgtatctctc ttttctcctg cttgaaggac 1020
agacatgact ttggatttcc ccaggaggag tttggcaacc agttccaaaa ggctgaaacc 1080
atccctgtcc tccatgagat gatccagcag atcttcaacc tgtttaccac caaagattct 1140
tctgctgctt gggatgagga cctgctggac aaattctgca ccgaactgta ccagcagctg 1200
aacgacctcg aagcctgtgt gatgcaggag gagcgtgtgg gtgaaactcc gctgatgaac 1260
gtggactcca tcctggctgt gaagaaatac ttccgtcgta tcactctgta tctgactgag 1320
aagaaataca gcccgtgtgc ttgggaggtt gttcgtgctg aaatcatgcg ttccctgtct 1380
ctgtctacca acctgcaaga acgtctgcgt cgtaaggaat aataaagctt ggatcc 1436

<210> 50
<211> 552
<212> DNA
<213> human

<400> 50
cacagccacc gcgacttcca gccggtgctc cacctggttg cgctcaacag ccccctgtca 60
ggcggcatgc ggggcatccg cggggccgac ttccagtgct tccagcaggc gcgggccgtg 120
gggctggcgg gcaccttccg cgccttcctg tcctcgcgcc tgcaggacct gtacagcatc 180
gtgcgccgtg ccgaccgcgc agccgtgccc atcgtcaacc tcaaggacga gctgctgttt 240
cccagctggg aggctctgtt ctcaggctct gagggtccgc tgaagcccgg ggcacgcatc 300
ttctcctttg acggcaagga cgtcctgagg caccccacct ggccccagaa gagcgtgtgg 360
catggctcgg accccaacgg gcgcaggctg accgagagct actgtgagac gtggcggacg 420
gaggctccct cggccacggg ccaggcctcc tcgctgctgg ggggcaggct cctggggcag 480
agtgccgcga gctgccatca cgcctacatc gtgctctgca ttgagaacag cttcatgact 540
gcctccaagt ag 552

<210> 51
<211> 2732
<212> DNA
<213> human

<400> 51
aacaacatcc tgggattggg acccactttc tgggcactgc tggccagtcc caaaatggaa 60
cataaggaag tggttcttct acttctttta tttctgaaat caggtcaagg agagcctctg 120
gatgactatg tgaataccca gggggcttca ctgttcagtg tcactaagaa gcagctggga 180
gcaggaagta tagaagaatg tgcagcaaaa tgtgaggagg acgaagaatt cacctgcagg 240
gcattccaat atcacagtaa agagcaacaa tgtgtgataa tggctgaaaa caggaagtcc 300
tccataatca ttaggatgag agatgtagtt ttatttgaaa agaaagtgta tctctcagag 360
tgcaagactg ggaatggaaa gaattacaga gggacgatgt ccaaaacaaa aaatggcatc 420
acctgtcaaa aatggagttc cacttctccc cacagaccta gattctcacc tgctacacac 480
ccctcagagg gactggagga gaactactgc aggaatccag acaacgatcc gcaggggccc 540
tggtgctata ctactgatcc agaaaagaga tatgactact gcgacattct tgagtgtgaa 600
gaggaatgta tgcattgcag tggagaaaac tatgacggca aaatttccaa gaccatgtct 660
ggactggaat gccaggcctg ggactctcag agcccacacg ctcatggata cattccttcc 720
aaatttccaa acaagaacct gaagaagaat tactgtcgta accccgatag ggagctgcgg 780
ccttggtgtt tcaccaccga ccccaacaag cgctgggaac tttgtgacat cccccgctgc 840
acaacacctc caccatcttc tggtcccacc taccagtgtc tgaagggaac aggtgaaaac 900
tatcgcggga atgtggctgt taccgtgtcc gggcacacct gtcagcactg gagtgcacag 960
acccctcaca cacataacag gacaccagaa aacttcccct gcaaaaattt ggatgaaaac 1020
tactgccgca atcctgacgg aaaaagggcc ccatggtgcc atacaaccaa cagccaagtg 1080
cggtgggagt actgtaagat accgtcctgt gactcctccc cagtatccac ggaacagttg 1140
gctcccacag caccacctga gctaacccct gtggtccagg actgctacca tggtgatgga 1200

cagagctacc gaggcacatc ctccaccacc accacaggaa agaagtgtca gtcttggtca 1260
tctatgacac cacaccggca ccagaagacc ccagaaaact acccaaatgc tggcctgaca 1320
atgaactact gcaggaatcc agatgccgat aaaggcccct ggtgttttac cacagacccc 1380
agcgtcaggt gggagtactg caacctgaaa aaatgctcag gaacagaagc gagtgttgta 1440
gcacctccgc ctgttgtcct gcttccagat gtagagactc cttccgaaga agactgtatg 1500
tttgggaatg ggaaaggata ccgaggcaag agggcgacca ctgttactgg gacgccatgc 1560
caggactggg ctgcccagga gccccataga cacagcattt tcactccaga gacaaatcca 1620
cgggcgggtc tggaaaaaaa ttactgccgt aaccctgatg gtgatgtagg tggtccctgg 1680
tgctacacga caaatccaag aaaactttac gactactgtg atgtccctca gtgtgcggcc 1740
ccttcatttg attgtgggaa gcctcaagtg gagccgaaga aatgtcctgg aagggttgtg 1800
ggggggtgtg tggcccaccc acattcctgg ccctggcaag tcagtcttag aacaaggttt 1860
ggaatgcact tctgtggagg caccttgata tccccagagt gggtgttgac tgctgcccac 1920
tgcttggaga agtccccaag gccttcatcc tacaaggtca tcctgggtgc acaccaagaa 1980
gtgaatctcg aaccgcatgt tcaggaaata gaagtgtcta ggctgttctt ggagcccaca 2040
cgaaaagata ttgccttgct aaagctaagc agtcctgccg tcatcactga caaagtaatc 2100
ccagcttgtc tgccatcccc aaattatgtg gtcgctgacc ggaccgaatg tttcatcact 2160
ggctggggag aaacccaagg tacttttgga gctggccttc tcaaggaagc ccagctccct 2220
gtgattgaga ataaagtgtg caatcgctat gagtttctga atggaagagt ccaatccacc 2280
gaactctgtg ctgggcattt ggccggaggc actgacagtt gccagggtga cagtggaggt 2340
cctctggttt gcttcgagaa ggacaaatac attttacaag gagtcacttc ttggggtctt 2400
ggctgtgcac gccccaataa gcctggtgtc tatgttcgtg tttcaaggtt tgttacttgg 2460
attgagggag tgatgagaaa taattaattg gacgggagac agagtgacgc actgactcac 2520
ctagaggctg ggacgtgggt agggatttag catgctggaa ataactggca gtaatcaaac 2580
gaagacactg tccccagcta ccagctacgc caaacctcgg cattttttgt gttattttct 2640
gactgctgga ttctgtagta aggtgacata gctatgacat ttgttaaaaa taaactctgt 2700
acttaacttt gatttgagta aattttggtt tt 2732

<210> 52
<211> 384
<212> DNA
<213> human

<400> 52
ggccctacgg gcaccggtga atccaagtgt cctctgatgg tcaaagttct agatgctgtc 60
cgaggcagtc ctgccatcaa tgtggccgtg catgtgttca gaaaggctgc tgatgacacc 120
tgggagccat ttgcctctgg gaaaaccagt gagtctggag agctgcatgg gctcacaact 180
gaggaggaat ttgtagaagg gatatacaaa gtggaaatag acaccaaatc ttactggaag 240
gcacttggca tctccccatt ccatgagcat gcagaggtgg tattcacagc caacgactcc 300
ggcccccgcc gctacaccat tgccgccctg ctgagcccct actcctattc caccacggct 360
gtcgtcacca atcccaagga atga 384

<210> 53
<211> 165
<212> PRT
<213> human

<400> 53

<210> 54
<211> 165
<212> PRT
<213> human

<400> 54

<210> 55
<211> 274
<212> PRT
<213> human

<400> 55
Met Ala Cys Asp Leu Pro Gln Thr His Ser Leu Gly Ser Arg Arg Thr
1               5                   10                  15
Leu Met Leu Leu Ala Gln Met Arg Arg Ile Ser Leu Phe Ser Cys Leu
            20                  25                  30
Lys Asp Arg His Asp Phe Gly Phe Pro Gln Glu Glu Phe Gly Asn Gln
        35                  40                  45
Phe Gln Lys Ala Glu Thr Ile Pro Val Leu His Glu Met Ile Gln Gln
    50                  55                  60
Ile Phe Asn Leu Phe Thr Thr Lys Asp Ser Ser Ala Ala Trp Asp Glu
65                  70                  75                  80
Asp Leu Leu Asp Lys Phe Cys Thr Glu Leu Tyr Gln Gln Leu Asn Asp
                85                  90                  95
Leu Glu Ala Cys Val Met Gln Glu Glu Arg Val Gly Glu Thr Pro Leu
            100                 105                 110
Met Asn Val Asp Ser Ile Leu Ala Val Lys Lys Tyr Phe Arg Arg Ile
        115                 120                 125
Thr Leu Tyr Leu Thr Glu Lys Lys Tyr Ser Pro Cys Ala Trp Glu Val
    130                 135                 140
Val Arg Ala Glu Ile Met Arg Ser Leu Ser Leu Ser Thr Asn Leu Gln
145                 150                 155                 160
Glu Arg Leu Arg Arg Lys Glu Tyr Val Glu Gly Gly Gly Gly Ser Pro
                165                 170                 175
Gly Gly Gly Ser Gly Gly Gly Ser Asn Met Ala Val Tyr Leu Ser Glu
            180                 185                 190
Cys Lys Thr Gly Asn Gly Lys Asn Tyr Arg Gly Thr Met Ser Lys Thr
        195                 200                 205
Lys Asn Gly Ile Thr Cys Gln Lys Trp Ser Ser Thr Ser Pro His Arg
    210                 215                 220
Pro Arg Phe Ser Pro Ala Thr His Pro Ser Glu Gly Leu Glu Glu Asn
225                 230                 235                 240
Tyr Cys Arg Asn Pro Asp Asn Asp Pro Gln Gly Pro Trp Cys Tyr Thr

                    245                     250                     255
Thr Asp Pro Glu Lys Arg Tyr Asp Tyr Cys Asp Ile Leu Glu Cys Glu
                260                     265                     270
Glu Glu

        <210> 56
        <211> 453
        <212> PRT
        <213> human

        <400> 56
Met Ala Cys Asp Leu Pro Gln Thr His Ser Leu Gly Ser Arg Arg Thr
 1               5                  10                  15
Leu Met Leu Leu Ala Gln Met Arg Arg Ile Ser Leu Phe Ser Cys Leu
                20                  25                  30
Lys Asp Arg His Asp Phe Gly Phe Pro Gln Glu Glu Phe Gly Asn Gln
            35                  40                  45
Phe Gln Lys Ala Glu Thr Ile Pro Val Leu His Glu Met Ile Gln Gln
        50                  55                  60
Ile Phe Asn Leu Phe Thr Thr Lys Asp Ser Ser Ala Ala Trp Asp Glu
65                  70                  75                  80
Asp Leu Leu Asp Lys Phe Cys Thr Glu Leu Tyr Gln Gln Leu Asn Asp
                85                  90                  95
Leu Glu Ala Cys Val Met Gln Glu Glu Arg Val Gly Glu Thr Pro Leu
            100                 105                 110
Met Asn Val Asp Ser Ile Leu Ala Val Lys Lys Tyr Phe Arg Arg Ile
        115                 120                 125
Thr Leu Tyr Leu Thr Glu Lys Lys Tyr Ser Pro Cys Ala Trp Glu Val
    130                 135                 140
Val Arg Ala Glu Ile Met Arg Ser Leu Ser Leu Ser Thr Asn Leu Gln
145                 150                 155                 160
Glu Arg Leu Arg Arg Lys Glu Tyr Val Glu Gly Gly Gly Gly Ser Pro
                165                 170                 175
Gly Gly Gly Ser Gly Gly Gly Ser Asn Met Ala Val Tyr Leu Ser Glu
            180                 185                 190
Cys Lys Thr Gly Asn Gly Lys Asn Tyr Arg Gly Thr Met Ser Lys Thr
        195                 200                 205
Lys Asn Gly Ile Thr Cys Gln Lys Trp Ser Ser Thr Ser Pro His Arg
    210                 215                 220
Pro Arg Phe Ser Pro Ala Thr His Pro Ser Glu Gly Leu Glu Glu Asn
225                 230                 235                 240
Tyr Cys Arg Asn Pro Asp Asn Asp Pro Gln Gly Pro Trp Cys Tyr Thr
                245                 250                 255
Thr Asp Pro Glu Lys Arg Tyr Asp Tyr Cys Asp Ile Leu Glu Cys Glu
            260                 265                 270
Glu Glu Cys Met His Cys Ser Gly Glu Asn Tyr Asp Gly Lys Ile Ser
        275                 280                 285
Lys Thr Met Ser Gly Leu Glu Cys Gln Ala Trp Asp Ser Gln Ser Pro
    290                 295                 300
His Ala His Gly Tyr Ile Pro Ser Lys Phe Pro Asn Lys Asn Leu Lys
305                 310                 315                 320
Lys Asn Tyr Cys Arg Asn Pro Asp Arg Glu Leu Arg Pro Trp Cys Phe
                325                 330                 335
Thr Thr Asp Pro Asn Lys Arg Trp Glu Leu Cys Asp Ile Pro Arg Cys
            340                 345                 350
Thr Thr Pro Pro Pro Ser Ser Gly Pro Thr Tyr Gln Cys Leu Lys Gly
        355                 360                 365
Thr Gly Glu Asn Tyr Arg Gly Asn Val Ala Val Thr Val Ser Gly His
    370                 375                 380
Thr Cys Gln His Trp Ser Ala Gln Thr Pro His Thr His Asn Arg Thr
385                 390                 395                 400
Pro Glu Asn Phe Pro Cys Lys Asn Leu Asp Glu Asn Tyr Cys Arg Asn
                405                 410                 415
Pro Asp Gly Lys Arg Ala Pro Trp Cys His Thr Thr Asn Ser Gln Val
            420                 425                 430
Arg Trp Glu Tyr Cys Lys Ile Pro Ser Cys Asp Ser Ser Pro Val Ser
        435                 440                 445

Thr Glu Gln Leu Ala
    450

        <210> 57
        <211> 274
        <212> PRT
        <213> human

        <400> 57
Met Ala Cys Asp Leu Pro Gln Thr His Ser Leu Gly Ser Arg Arg Thr
 1               5                  10                  15
Leu Met Leu Leu Ala Gln Met Arg Arg Ile Ser Leu Phe Ser Cys Leu
            20                  25                  30
Lys Asp Arg His Asp Phe Gly Phe Pro Gln Glu Glu Phe Gly Asn Gln
        35                  40                  45
Phe Gln Lys Ala Glu Thr Ile Pro Val Leu His Glu Met Ile Gln Gln
    50                  55                  60
Ile Phe Asn Leu Phe Ser Thr Lys Asp Ser Ser Ala Ala Trp Asp Glu
65                  70                  75                  80
Thr Leu Leu Asp Lys Phe Tyr Thr Glu Leu Tyr Gln Gln Leu Asn Asp
                85                  90                  95
Leu Glu Ala Cys Val Ile Gln Gly Val Gly Val Thr Glu Thr Pro Leu
            100                 105                 110
Met Lys Glu Asp Ser Ile Leu Ala Val Arg Lys Tyr Phe Gln Arg Ile
        115                 120                 125
Thr Leu Tyr Leu Lys Glu Lys Lys Tyr Ser Pro Cys Ala Trp Glu Val
    130                 135                 140
Val Arg Ala Glu Ile Met Arg Ser Phe Ser Leu Ser Thr Asn Leu Gln
145                 150                 155                 160
Glu Ser Leu Arg Ser Lys Glu Tyr Val Glu Gly Gly Gly Gly Ser Pro
                165                 170                 175
Gly Gly Gly Ser Gly Gly Gly Ser Asn Met Ala Val Tyr Leu Ser Glu
            180                 185                 190
Cys Lys Thr Gly Asn Gly Lys Asn Tyr Arg Gly Thr Met Ser Lys Thr
        195                 200                 205
Lys Asn Gly Ile Thr Cys Gln Lys Trp Ser Ser Thr Ser Pro His Arg
    210                 215                 220
Pro Arg Phe Ser Pro Ala Thr His Pro Ser Glu Gly Leu Glu Glu Asn
225                 230                 235                 240
Tyr Cys Arg Asn Pro Asp Asn Asp Pro Gln Gly Pro Trp Cys Tyr Thr
                245                 250                 255
Thr Asp Pro Glu Lys Arg Tyr Asp Tyr Cys Asp Ile Leu Glu Cys Glu
            260                 265                 270
Glu Glu

        <210> 58
        <211> 453
        <212> PRT
        <213> human

        <400> 58
Met Ala Cys Asp Leu Pro Gln Thr His Ser Leu Gly Ser Arg Arg Thr
 1               5                  10                  15
Leu Met Leu Leu Ala Gln Met Arg Arg Ile Ser Leu Phe Ser Cys Leu
            20                  25                  30
Lys Asp Arg His Asp Phe Gly Phe Pro Gln Glu Glu Phe Gly Asn Gln
        35                  40                  45
Phe Gln Lys Ala Glu Thr Ile Pro Val Leu His Glu Met Ile Gln Gln
    50                  55                  60
Ile Phe Asn Leu Phe Ser Thr Lys Asp Ser Ser Ala Ala Trp Asp Glu
65                  70                  75                  80
Thr Leu Leu Asp Lys Phe Tyr Thr Glu Leu Tyr Gln Gln Leu Asn Asp
                85                  90                  95
Leu Glu Ala Cys Val Ile Gln Gly Val Gly Val Thr Glu Thr Pro Leu
            100                 105                 110
Met Lys Glu Asp Ser Ile Leu Ala Val Arg Lys Tyr Phe Gln Arg Ile
        115                 120                 125

Thr Leu Tyr Leu Lys Glu Lys Lys Tyr Ser Pro Cys Ala Trp Glu Val
    130                 135                 140
Val Arg Ala Glu Ile Met Arg Ser Phe Ser Leu Ser Thr Asn Leu Gln
145                 150                 155                 160
Glu Ser Leu Arg Ser Lys Glu Tyr Val Glu Gly Gly Gly Gly Ser Pro
                165                 170                 175
Gly Gly Gly Ser Gly Gly Gly Ser Asn Met Ala Val Tyr Leu Ser Glu
            180                 185                 190
Cys Lys Thr Gly Asn Gly Lys Asn Tyr Arg Gly Thr Met Ser Lys Thr
        195                 200                 205
Lys Asn Gly Ile Thr Cys Gln Lys Trp Ser Ser Thr Ser Pro His Arg
    210                 215                 220
Pro Arg Phe Ser Pro Ala Thr His Pro Ser Glu Gly Leu Glu Glu Asn
225                 230                 235                 240
Tyr Cys Arg Asn Pro Asp Asn Asp Pro Gln Gly Pro Trp Cys Tyr Thr
                245                 250                 255
Thr Asp Pro Glu Lys Arg Tyr Asp Tyr Cys Asp Ile Leu Glu Cys Glu
            260                 265                 270
Glu Glu Cys Met His Cys Ser Gly Glu Asn Tyr Asp Gly Lys Ile Ser
        275                 280                 285
Lys Thr Met Ser Gly Leu Glu Cys Gln Ala Trp Asp Ser Gln Ser Pro
    290                 295                 300
His Ala His Gly Tyr Ile Pro Ser Lys Phe Pro Asn Lys Asn Leu Lys
305                 310                 315                 320
Lys Asn Tyr Cys Arg Asn Pro Asp Arg Glu Leu Arg Pro Trp Cys Phe
                325                 330                 335
Thr Thr Asp Pro Asn Lys Arg Trp Glu Leu Cys Asp Ile Pro Arg Cys
            340                 345                 350
Thr Thr Pro Pro Pro Ser Ser Gly Pro Thr Tyr Gln Cys Leu Lys Gly
        355                 360                 365
Thr Gly Glu Asn Tyr Arg Gly Asn Val Ala Val Thr Val Ser Gly His
    370                 375                 380
Thr Cys Gln His Trp Ser Ala Gln Thr Pro His Thr His Asn Arg Thr
385                 390                 395                 400
Pro Glu Asn Phe Pro Cys Lys Asn Leu Asp Glu Asn Tyr Cys Arg Asn
                405                 410                 415
Pro Asp Gly Lys Arg Ala Pro Trp Cys His Thr Thr Asn Ser Gln Val
            420                 425                 430
Arg Trp Glu Tyr Cys Lys Ile Pro Ser Cys Asp Ser Ser Pro Val Ser
        435                 440                 445
Thr Glu Gln Leu Ala
    450

<210> 59
<211> 453
<212> PRT
<213> human

<400> 59
Met Ala Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Cys Met His Cys Ser Gly Glu
                85                  90                  95
Asn Tyr Asp Gly Lys Ile Ser Lys Thr Met Ser Gly Leu Glu Cys Gln
            100                 105                 110
Ala Trp Asp Ser Gln Ser Pro His Ala His Gly Tyr Ile Pro Ser Lys
        115                 120                 125
Phe Pro Asn Lys Asn Leu Lys Lys Asn Tyr Cys Arg Asn Pro Asp Arg
    130                 135                 140
Glu Leu Arg Pro Trp Cys Phe Thr Thr Asp Pro Asn Lys Arg Trp Glu

145                 150                 155                 160
Leu Cys Asp Ile Pro Arg Cys Thr Thr Pro Pro Pro Ser Ser Gly Pro
                165                 170                 175
Thr Tyr Gln Cys Leu Lys Gly Thr Gly Glu Asn Tyr Arg Gly Asn Val
            180                 185                 190
Ala Val Thr Val Ser Gly His Thr Cys Gln His Trp Ser Ala Gln Thr
        195                 200                 205
Pro His Thr His Asn Arg Thr Pro Glu Asn Phe Pro Cys Lys Asn Leu
    210                 215                 220
Asp Glu Asn Tyr Cys Arg Asn Pro Asp Gly Lys Arg Ala Pro Trp Cys
225                 230                 235                 240
His Thr Thr Asn Ser Gln Val Arg Trp Glu Tyr Cys Lys Ile Pro Ser
                245                 250                 255
Cys Asp Ser Ser Pro Val Ser Thr Glu Gln Leu Ala Tyr Val Glu Gly
            260                 265                 270
Gly Gly Gly Ser Pro Gly Gly Gly Ser Gly Gly Gly Ser Asn Met Ala
        275                 280                 285
Cys Asp Leu Pro Gln Thr His Ser Leu Gly Ser Arg Arg Thr Leu Met
    290                 295                 300
Leu Leu Ala Gln Met Arg Arg Ile Ser Leu Phe Ser Cys Leu Lys Asp
305                 310                 315                 320
Arg His Asp Phe Gly Phe Pro Gln Glu Glu Phe Gly Asn Gln Phe Gln
                325                 330                 335
Lys Ala Glu Thr Ile Pro Val Leu His Glu Met Ile Gln Gln Ile Phe
            340                 345                 350
Asn Leu Phe Ser Thr Lys Asp Ser Ser Ala Ala Trp Asp Glu Thr Leu
        355                 360                 365
Leu Asp Lys Phe Tyr Thr Glu Leu Tyr Gln Gln Leu Asn Asp Leu Glu
    370                 375                 380
Ala Cys Val Ile Gln Gly Val Gly Val Thr Glu Thr Pro Leu Met Lys
385                 390                 395                 400
Glu Asp Ser Ile Leu Ala Val Arg Lys Tyr Phe Gln Arg Ile Thr Leu
                405                 410                 415
Tyr Leu Lys Glu Lys Lys Tyr Ser Pro Cys Ala Trp Glu Val Val Arg
            420                 425                 430
Ala Glu Ile Met Arg Ser Phe Ser Leu Ser Thr Asn Leu Gln Glu Ser
        435                 440                 445
Leu Arg Ser Lys Glu
    450

<210> 60
<211> 274
<212> PRT
<213> human

<400> 60
Met Ala Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Tyr Val Glu Gly Gly Gly Gly
                85                  90                  95
Ser Pro Gly Gly Gly Ser Gly Gly Gly Ser Asn Met Ala Cys Asp Leu
            100                 105                 110
Pro Gln Thr His Ser Leu Gly Ser Arg Arg Thr Leu Met Leu Leu Ala
        115                 120                 125
Gln Met Arg Arg Ile Ser Leu Phe Ser Cys Leu Lys Asp Arg His Asp
    130                 135                 140
Phe Gly Phe Pro Gln Glu Glu Phe Gly Asn Gln Phe Gln Lys Ala Glu
145                 150                 155                 160
Thr Ile Pro Val Leu His Glu Met Ile Gln Gln Ile Phe Asn Leu Phe
                165                 170                 175

Thr Thr Lys Asp Ser Ser Ala Ala Trp Asp Glu Asp Leu Leu Asp Lys
            180                 185                 190
Phe Cys Thr Glu Leu Tyr Gln Gln Leu Asn Asp Leu Glu Ala Cys Val
        195                 200                 205
Met Gln Glu Glu Arg Val Gly Glu Thr Pro Leu Met Asn Val Asp Ser
    210                 215                 220
Ile Leu Ala Val Lys Lys Tyr Phe Arg Arg Ile Thr Leu Tyr Leu Thr
225                 230                 235                 240
Glu Lys Lys Tyr Ser Pro Cys Ala Trp Glu Val Val Arg Ala Glu Ile
                245                 250                 255
Met Arg Ser Leu Ser Leu Ser Thr Asn Leu Gln Glu Arg Leu Arg Arg
            260                 265                 270
Lys Glu

<210> 61
<211> 274
<212> PRT
<213> human

<400> 61
Met Ala Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Tyr Val Glu Gly Gly Gly Gly
                85                  90                  95
Ser Pro Gly Gly Gly Ser Gly Gly Gly Ser Asn Met Ala Cys Asp Leu
            100                 105                 110
Pro Gln Thr His Ser Leu Gly Ser Arg Arg Thr Leu Met Leu Leu Ala
        115                 120                 125
Gln Met Arg Arg Ile Ser Leu Phe Ser Cys Leu Lys Asp Arg His Asp
    130                 135                 140
Phe Gly Phe Pro Gln Glu Glu Phe Gly Asn Gln Phe Gln Lys Ala Glu
145                 150                 155                 160
Thr Ile Pro Val Leu His Glu Met Ile Gln Gln Ile Phe Asn Leu Phe
                165                 170                 175
Ser Thr Lys Asp Ser Ser Ala Ala Trp Asp Glu Thr Leu Leu Asp Lys
            180                 185                 190
Phe Tyr Thr Glu Leu Tyr Gln Gln Leu Asn Asp Leu Glu Ala Cys Val
        195                 200                 205
Ile Gln Gly Val Gly Val Thr Glu Thr Pro Leu Met Lys Glu Asp Ser
    210                 215                 220
Ile Leu Ala Val Arg Lys Tyr Phe Gln Arg Ile Thr Leu Tyr Leu Lys
225                 230                 235                 240
Glu Lys Lys Tyr Ser Pro Cys Ala Trp Glu Val Val Arg Ala Glu Ile
                245                 250                 255
Met Arg Ser Phe Ser Leu Ser Thr Asn Leu Gln Glu Ser Leu Arg Ser
            260                 265                 270
Lys Glu

<210> 62
<211> 453
<212> PRT
<213> human

<400> 62
Met Ala Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30

Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Cys Met His Cys Ser Gly Glu
                85                  90                  95
Asn Tyr Asp Gly Lys Ile Ser Lys Thr Met Ser Gly Leu Glu Cys Gln
            100                 105                 110
Ala Trp Asp Ser Gln Ser Pro His Ala His Gly Tyr Ile Pro Ser Lys
        115                 120                 125
Phe Pro Asn Lys Asn Leu Lys Lys Asn Tyr Cys Arg Asn Pro Asp Arg
    130                 135                 140
Glu Leu Arg Pro Trp Cys Phe Thr Thr Asp Pro Asn Lys Arg Trp Glu
145                 150                 155                 160
Leu Cys Asp Ile Pro Arg Cys Thr Thr Pro Pro Pro Ser Ser Gly Pro
                165                 170                 175
Thr Tyr Gln Cys Leu Lys Gly Thr Gly Glu Asn Tyr Arg Gly Asn Val
            180                 185                 190
Ala Val Thr Val Ser Gly His Thr Cys Gln His Trp Ser Ala Gln Thr
        195                 200                 205
Pro His Thr His Asn Arg Thr Pro Glu Asn Phe Pro Cys Lys Asn Leu
    210                 215                 220
Asp Glu Asn Tyr Cys Arg Asn Pro Asp Gly Lys Arg Ala Pro Trp Cys
225                 230                 235                 240
His Thr Thr Asn Ser Gln Val Arg Trp Glu Tyr Cys Lys Ile Pro Ser
                245                 250                 255
Cys Asp Ser Ser Pro Val Ser Thr Glu Gln Leu Ala Tyr Val Glu Gly
            260                 265                 270
Gly Gly Gly Ser Pro Gly Gly Gly Ser Gly Gly Gly Ser Asn Met Ala
        275                 280                 285
Cys Asp Leu Pro Gln Thr His Ser Leu Gly Ser Arg Arg Thr Leu Met
    290                 295                 300
Leu Leu Ala Gln Met Arg Arg Ile Ser Leu Phe Ser Cys Leu Lys Asp
305                 310                 315                 320
Arg His Asp Phe Gly Phe Pro Gln Glu Glu Phe Gly Asn Gln Phe Gln
                325                 330                 335
Lys Ala Glu Thr Ile Pro Val Leu His Glu Met Ile Gln Gln Ile Phe
            340                 345                 350
Asn Leu Phe Thr Thr Lys Asp Ser Ser Ala Ala Trp Asp Glu Asp Leu
        355                 360                 365
Leu Asp Lys Phe Cys Thr Glu Leu Tyr Gln Gln Leu Asn Asp Leu Glu
    370                 375                 380
Ala Cys Val Met Gln Glu Glu Arg Val Gly Glu Thr Pro Leu Met Asn
385                 390                 395                 400
Val Asp Ser Ile Leu Ala Val Lys Lys Tyr Phe Arg Arg Ile Thr Leu
                405                 410                 415
Tyr Leu Thr Glu Lys Lys Tyr Ser Pro Cys Ala Trp Glu Val Val Arg
            420                 425                 430
Ala Glu Ile Met Arg Ser Leu Ser Leu Ser Thr Asn Leu Gln Glu Arg
        435                 440                 445
Leu Arg Arg Lys Glu
    450

<210> 63
<211> 566
<212> PRT
<213> human

<400> 63
Met Glu His Lys Glu Val Val Leu Leu Leu Leu Leu Phe Leu Lys Ser
1               5                   10                  15
Gly Gln Gly Glu Pro Leu Asp Asp Tyr Val Asn Thr Gln Gly Ala Ser
            20                  25                  30
Leu Phe Ser Val Thr Lys Lys Gln Leu Gly Ala Gly Ser Ile Glu Glu
        35                  40                  45
Cys Ala Ala Lys Cys Glu Glu Asp Glu Glu Phe Thr Cys Arg Ala Phe

50                  55                  60
Gln Tyr His Ser Lys Glu Gln Gln Cys Val Ile Met Ala Glu Asn Arg
65                  70                  75                  80
Lys Ser Ser Ile Ile Ile Arg Met Arg Asp Val Val Leu Phe Glu Lys
                85                  90                  95
Lys Val Tyr Leu Ser Glu Cys Lys Thr Gly Asn Gly Lys Asn Tyr Arg
            100                 105                 110
Gly Thr Met Ser Lys Thr Lys Asn Gly Ile Thr Cys Gln Lys Trp Ser
        115                 120                 125
Ser Thr Ser Pro His Arg Pro Arg Phe Ser Pro Ala Thr His Pro Ser
    130                 135                 140
Glu Gly Leu Glu Glu Asn Tyr Cys Arg Asn Pro Asp Asn Asp Pro Gln
145                 150                 155                 160
Gly Pro Trp Cys Tyr Thr Thr Asp Pro Glu Lys Arg Tyr Asp Tyr Cys
                165                 170                 175
Asp Ile Leu Glu Cys Glu Glu Glu Cys Met His Cys Ser Gly Glu Asn
            180                 185                 190
Tyr Asp Gly Lys Ile Ser Lys Thr Met Ser Gly Leu Glu Cys Gln Ala
        195                 200                 205
Trp Asp Ser Gln Ser Pro His Ala His Gly Tyr Ile Pro Ser Lys Phe
    210                 215                 220
Pro Asn Lys Asn Leu Lys Lys Asn Tyr Cys Arg Asn Pro Asp Arg Glu
225                 230                 235                 240
Leu Arg Pro Trp Cys Phe Thr Thr Asp Pro Asn Lys Arg Trp Glu Leu
                245                 250                 255
Cys Asp Ile Pro Arg Cys Thr Thr Pro Pro Pro Ser Ser Gly Pro Thr
            260                 265                 270
Tyr Gln Cys Leu Lys Gly Thr Gly Glu Asn Tyr Arg Gly Asn Val Ala
        275                 280                 285
Val Thr Val Ser Gly His Thr Cys Gln His Trp Ser Ala Gln Thr Pro
    290                 295                 300
His Thr His Asn Arg Thr Pro Glu Asn Phe Pro Cys Lys Asn Leu Asp
305                 310                 315                 320
Glu Asn Tyr Cys Arg Asn Pro Asp Gly Lys Arg Ala Pro Trp Cys His
                325                 330                 335
Thr Thr Asn Ser Gln Val Arg Trp Glu Tyr Cys Lys Ile Pro Ser Cys
            340                 345                 350
Asp Ser Ser Pro Val Ser Thr Glu Gln Leu Ala Pro Thr Ala Pro Pro
        355                 360                 365
Glu Leu Thr Pro Val Val Gln Asp Cys Tyr His Gly Asp Gly Gln Ser
    370                 375                 380
Tyr Arg Gly Thr Ser Ser Thr Thr Thr Thr Gly Lys Lys Cys Gln Ser
385                 390                 395                 400
Trp Ser Ser Met Thr Pro His Arg His Gln Lys Thr Pro Glu Asn Tyr
                405                 410                 415
Pro Asn Ala Gly Leu Thr Met Asn Tyr Cys Arg Asn Pro Asp Ala Asp
            420                 425                 430
Lys Gly Pro Trp Cys Phe Thr Thr Asp Pro Ser Val Arg Trp Glu Tyr
        435                 440                 445
Cys Asn Leu Lys Lys Cys Ser Gly Thr Glu Ala Ser Val Val Ala Pro
    450                 455                 460
Pro Pro Val Val Leu Leu Pro Asp Val Glu Thr Pro Ser Glu Glu Asp
465                 470                 475                 480
Cys Met Phe Gly Asn Gly Lys Gly Tyr Arg Gly Lys Arg Ala Thr Thr
                485                 490                 495
Val Thr Gly Thr Pro Cys Gln Asp Trp Ala Ala Gln Glu Pro His Arg
            500                 505                 510
His Ser Ile Phe Thr Pro Glu Thr Asn Pro Arg Ala Gly Leu Glu Lys
        515                 520                 525
Asn Tyr Cys Arg Asn Pro Asp Gly Asp Val Gly Gly Pro Trp Cys Tyr
    530                 535                 540
Thr Thr Asn Pro Arg Lys Leu Tyr Asp Tyr Cys Asp Val Pro Gln Cys
545                 550                 555                 560
Ala Ala Pro Ser Phe Asp
                565

<210> 64
<211> 381

<212> PRT
<213> human

<400> 64
Met Ser Arg Leu Pro Val Leu Leu Leu Leu Gln Leu Leu Val Arg Pro
1               5                   10                  15
Ala Met Ser Val Tyr Leu Ser Glu Cys Lys Thr Gly Asn Gly Lys Asn
            20                  25                  30
Tyr Arg Gly Thr Met Ser Lys Thr Lys Asn Gly Ile Thr Cys Gln Lys
        35                  40                  45
Trp Ser Ser Thr Ser Pro His Arg Pro Arg Phe Ser Pro Ala Thr His
    50                  55                  60
Pro Ser Glu Gly Leu Glu Glu Asn Tyr Cys Arg Asn Pro Asp Asn Asp
65                  70                  75                  80
Pro Gln Gly Pro Trp Cys Tyr Thr Thr Asp Pro Glu Lys Arg Tyr Asp
                85                  90                  95
Tyr Cys Asp Ile Leu Glu Cys Glu Glu Glu Cys Met His Cys Ser Gly
            100                 105                 110
Glu Asn Tyr Asp Gly Lys Ile Ser Lys Thr Met Ser Gly Leu Glu Cys
        115                 120                 125
Gln Ala Trp Asp Ser Gln Ser Pro His Ala His Gly Tyr Ile Pro Ser
    130                 135                 140
Lys Phe Pro Asn Lys Asn Leu Lys Lys Asn Tyr Cys Arg Asn Pro Asp
145                 150                 155                 160
Arg Glu Leu Arg Pro Trp Cys Phe Thr Thr Asp Pro Asn Lys Arg Trp
                165                 170                 175
Glu Leu Cys Asp Ile Pro Arg Cys Thr Thr Pro Pro Pro Ser Ser Gly
            180                 185                 190
Pro Thr Tyr Gln Cys Leu Lys Gly Thr Gly Glu Asn Tyr Arg Gly Asn
        195                 200                 205
Val Ala Val Thr Val Ser Gly His Thr Cys Gln His Trp Ser Ala Gln
    210                 215                 220
Thr Pro His Thr His Asn Arg Thr Pro Glu Asn Phe Pro Cys Lys Asn
225                 230                 235                 240
Leu Asp Glu Asn Tyr Cys Arg Asn Pro Asp Gly Lys Arg Ala Pro Trp
                245                 250                 255
Cys His Thr Thr Asn Ser Gln Val Arg Trp Glu Tyr Cys Lys Ile Pro
            260                 265                 270
Ser Cys Asp Ser Ser Pro Val Ser Thr Glu Gln Leu Ala Pro Thr Ala
        275                 280                 285
Pro Pro Glu Leu Thr Pro Val Val Gln Asp Cys Tyr His Gly Asp Gly
    290                 295                 300
Gln Ser Tyr Arg Gly Thr Ser Ser Thr Thr Thr Thr Gly Lys Lys Cys
305                 310                 315                 320
Gln Ser Trp Ser Ser Met Thr Pro His Arg His Gln Lys Thr Pro Glu
                325                 330                 335
Asn Tyr Pro Asn Ala Gly Leu Thr Met Asn Tyr Cys Arg Asn Pro Asp
            340                 345                 350
Ala Asp Lys Gly Pro Trp Cys Phe Thr Thr Asp Pro Ser Val Arg Trp
        355                 360                 365
Glu Tyr Cys Asn Leu Lys Lys Cys Ser Gly Thr Glu Ala
    370                 375                 380

<210> 65
<211> 285
<212> PRT
<213> human

<400> 65
Met Ser Arg Leu Pro Val Leu Leu Leu Leu Gln Leu Leu Val Arg Pro
1               5                   10                  15
Ala Met Ser Val Tyr Leu Ser Glu Cys Lys Thr Gly Asn Gly Lys Asn
            20                  25                  30
Tyr Arg Gly Thr Met Ser Lys Thr Lys Asn Gly Ile Thr Cys Gln Lys
        35                  40                  45
Trp Ser Ser Thr Ser Pro His Arg Pro Arg Phe Ser Pro Ala Thr His
    50                  55                  60
Pro Ser Glu Gly Leu Glu Glu Asn Tyr Cys Arg Asn Pro Asp Asn Asp

65                  70                  75                  80
Pro Gln Gly Pro Trp Cys Tyr Thr Thr Asp Pro Glu Lys Arg Tyr Asp
                85                  90                  95
Tyr Cys Asp Ile Leu Glu Cys Glu Glu Glu Cys Met His Cys Ser Gly
            100                 105                 110
Glu Asn Tyr Asp Gly Lys Ile Ser Lys Thr Met Ser Gly Leu Glu Cys
        115                 120                 125
Gln Ala Trp Asp Ser Gln Ser Pro His Ala His Gly Tyr Ile Pro Ser
    130                 135                 140
Lys Phe Pro Asn Lys Asn Leu Lys Lys Asn Tyr Cys Arg Asn Pro Asp
145                 150                 155                 160
Arg Glu Leu Arg Pro Trp Cys Phe Thr Thr Asp Pro Asn Lys Arg Trp
                165                 170                 175
Glu Leu Cys Asp Ile Pro Arg Cys Thr Thr Pro Pro Pro Ser Ser Gly
            180                 185                 190
Pro Thr Tyr Gln Cys Leu Lys Gly Thr Gly Glu Asn Tyr Arg Gly Asn
        195                 200                 205
Val Ala Val Thr Val Ser Gly His Thr Cys Gln His Trp Ser Ala Gln
    210                 215                 220
Thr Pro His Thr His Asn Arg Thr Pro Glu Asn Phe Pro Cys Lys Asn
225                 230                 235                 240
Leu Asp Glu Asn Tyr Cys Arg Asn Pro Asp Gly Lys Arg Ala Pro Trp
                245                 250                 255
Cys His Thr Thr Asn Ser Gln Val Arg Trp Glu Tyr Cys Lys Ile Pro
            260                 265                 270
Ser Cys Asp Ser Ser Pro Val Ser Thr Glu Gln Leu Ala
        275                 280                 285

<210> 66
<211> 297
<212> PRT
<213> human

<400> 66
Met Ser Arg Leu Pro Val Leu Leu Leu Leu Gln Leu Leu Val Arg Pro
 1               5                  10                  15
Ala Met Ser Glu Glu Glu Cys Met His Cys Ser Gly Glu Asn Tyr Asp
            20                  25                  30
Gly Lys Ile Ser Lys Thr Met Ser Gly Leu Glu Cys Gln Ala Trp Asp
        35                  40                  45
Ser Gln Ser Pro His Ala His Gly Tyr Ile Pro Ser Lys Phe Pro Asn
    50                  55                  60
Lys Asn Leu Lys Lys Asn Tyr Cys Arg Asn Pro Asp Arg Glu Leu Arg
65                  70                  75                  80
Pro Trp Cys Phe Thr Thr Asp Pro Asn Lys Arg Trp Glu Leu Cys Asp
                85                  90                  95
Ile Pro Arg Cys Thr Thr Pro Pro Pro Ser Ser Gly Pro Thr Tyr Gln
            100                 105                 110
Cys Leu Lys Gly Thr Gly Glu Asn Tyr Arg Gly Asn Val Ala Val Thr
        115                 120                 125
Val Ser Gly His Thr Cys Gln His Trp Ser Ala Gln Thr Pro His Thr
    130                 135                 140
His Asn Arg Thr Pro Glu Asn Phe Pro Cys Lys Asn Leu Asp Glu Asn
145                 150                 155                 160
Tyr Cys Arg Asn Pro Asp Gly Lys Arg Ala Pro Trp Cys His Thr Thr
                165                 170                 175
Asn Ser Gln Val Arg Trp Glu Tyr Cys Lys Ile Pro Ser Cys Asp Ser
            180                 185                 190
Ser Pro Val Ser Thr Glu Gln Leu Ala Pro Thr Ala Pro Pro Glu Leu
        195                 200                 205
Thr Pro Val Val Gln Asp Cys Tyr His Gly Asp Gly Gln Ser Tyr Arg
    210                 215                 220
Gly Thr Ser Ser Thr Thr Thr Thr Gly Lys Lys Cys Gln Ser Trp Ser
225                 230                 235                 240
Ser Met Thr Pro His Arg His Gln Lys Thr Pro Glu Asn Tyr Pro Asn
                245                 250                 255
Ala Gly Leu Thr Met Asn Tyr Cys Arg Asn Pro Asp Ala Asp Lys Gly
            260                 265                 270

Pro Trp Cys Phe Thr Thr Asp Pro Ser Val Arg Trp Glu Tyr Cys Asn
        275                 280                 285
Leu Lys Lys Cys Ser Gly Thr Glu Ala
    290                 295

<210> 67
<211> 201
<212> PRT
<213> human

<400> 67
Met Ser Arg Leu Pro Val Leu Leu Leu Leu Gln Leu Leu Val Arg Pro
1               5                   10                  15
Ala Met Ser Glu Glu Glu Cys Met His Cys Ser Gly Glu Asn Tyr Asp
            20                  25                  30
Gly Lys Ile Ser Lys Thr Met Ser Gly Leu Glu Cys Gln Ala Trp Asp
        35                  40                  45
Ser Gln Ser Pro His Ala His Gly Tyr Ile Pro Ser Lys Phe Pro Asn
    50                  55                  60
Lys Asn Leu Lys Lys Asn Tyr Cys Arg Asn Pro Asp Arg Glu Leu Arg
65                  70                  75                  80
Pro Trp Cys Phe Thr Thr Asp Pro Asn Lys Arg Trp Glu Leu Cys Asp
                85                  90                  95
Ile Pro Arg Cys Thr Thr Pro Pro Pro Ser Ser Gly Pro Thr Tyr Gln
            100                 105                 110
Cys Leu Lys Gly Thr Gly Glu Asn Tyr Arg Gly Asn Val Ala Val Thr
        115                 120                 125
Val Ser Gly His Thr Cys Gln His Trp Ser Ala Gln Thr Pro His Thr
    130                 135                 140
His Asn Arg Thr Pro Glu Asn Phe Pro Cys Lys Asn Leu Asp Glu Asn
145                 150                 155                 160
Tyr Cys Arg Asn Pro Asp Gly Lys Arg Ala Pro Trp Cys His Thr Thr
                165                 170                 175
Asn Ser Gln Val Arg Trp Glu Tyr Cys Lys Ile Pro Ser Cys Asp Ser
            180                 185                 190
Ser Pro Val Ser Thr Glu Gln Leu Ala
        195                 200

<210> 68
<211> 380
<212> PRT
<213> human

<400> 68
Met Ser Leu Phe Glu Lys Lys Val Tyr Leu Ser Glu Cys Lys Thr Gly
1               5                   10                  15
Asn Gly Lys Asn Tyr Arg Gly Thr Met Ser Lys Thr Lys Asn Gly Ile
            20                  25                  30
Thr Cys Gln Lys Trp Ser Ser Thr Ser Pro His Arg Pro Arg Phe Ser
        35                  40                  45
Pro Ala Thr His Pro Ser Glu Gly Leu Glu Glu Asn Tyr Cys Arg Asn
    50                  55                  60
Pro Asp Asn Asp Pro Gln Gly Pro Trp Cys Tyr Thr Thr Asp Pro Glu
65                  70                  75                  80
Lys Arg Tyr Asp Tyr Cys Asp Ile Leu Glu Cys Glu Glu Glu Cys Met
                85                  90                  95
His Cys Ser Gly Glu Asn Tyr Asp Gly Lys Ile Ser Lys Thr Met Ser
            100                 105                 110
Gly Leu Glu Cys Gln Ala Trp Asp Ser Gln Ser Pro His Ala His Gly
        115                 120                 125
Tyr Ile Pro Ser Lys Phe Pro Asn Lys Asn Leu Lys Lys Asn Tyr Cys
    130                 135                 140
Arg Asn Pro Asp Arg Glu Leu Arg Pro Trp Cys Phe Thr Thr Asp Pro
145                 150                 155                 160
Asn Lys Arg Trp Glu Leu Cys Asp Ile Pro Arg Cys Thr Thr Pro Pro
                165                 170                 175
Pro Ser Ser Gly Pro Thr Tyr Gln Cys Leu Lys Gly Thr Gly Glu Asn
            180                 185                 190

Tyr Arg Gly Asn Val Ala Val Thr Val Ser Gly His Thr Cys Gln His
        195                 200                 205
Trp Ser Ala Gln Thr Pro His Thr His Asn Arg Thr Pro Glu Asn Phe
    210                 215                 220
Pro Cys Lys Asn Leu Asp Glu Asn Tyr Cys Arg Asn Pro Asp Gly Lys
225                 230                 235                 240
Arg Ala Pro Trp Cys His Thr Thr Asn Ser Gln Val Arg Trp Glu Tyr
                245                 250                 255
Cys Lys Ile Pro Ser Cys Asp Ser Ser Pro Val Ser Thr Glu Gln Leu
            260                 265                 270
Ala Pro Thr Ala Pro Pro Glu Leu Thr Pro Val Val Gln Asp Cys Tyr
        275                 280                 285
His Gly Asp Gly Gln Ser Tyr Arg Gly Thr Ser Ser Thr Thr Thr Thr
    290                 295                 300
Gly Lys Lys Cys Gln Ser Trp Ser Ser Met Thr Pro His Arg His Gln
305                 310                 315                 320
Lys Thr Pro Glu Asn Tyr Pro Asn Ala Gly Leu Thr Met Asn Tyr Cys
                325                 330                 335
Arg Asn Pro Asp Ala Asp Lys Gly Pro Trp Cys Phe Thr Thr Asp Pro
            340                 345                 350
Ser Val Arg Trp Glu Tyr Cys Asn Leu Lys Lys Cys Ser Gly Thr Glu
        355                 360                 365
Ala Ser Val Val Ala Pro Pro Pro Val Val Leu Leu
    370                 375                 380

<210> 69
<211> 364
<212> PRT
<213> human

<400> 69
Met Ser Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Cys Met His Cys Ser Gly Glu
                85                  90                  95
Asn Tyr Asp Gly Lys Ile Ser Lys Thr Met Ser Gly Leu Glu Cys Gln
            100                 105                 110
Ala Trp Asp Ser Gln Ser Pro His Ala His Gly Tyr Ile Pro Ser Lys
        115                 120                 125
Phe Pro Asn Lys Asn Leu Lys Lys Asn Tyr Cys Arg Asn Pro Asp Arg
    130                 135                 140
Glu Leu Arg Pro Trp Cys Phe Thr Thr Asp Pro Asn Lys Arg Trp Glu
145                 150                 155                 160
Leu Cys Asp Ile Pro Arg Cys Thr Thr Pro Pro Pro Ser Ser Gly Pro
                165                 170                 175
Thr Tyr Gln Cys Leu Lys Gly Thr Gly Glu Asn Tyr Arg Gly Asn Val
            180                 185                 190
Ala Val Thr Val Ser Gly His Thr Cys Gln His Trp Ser Ala Gln Thr
        195                 200                 205
Pro His Thr His Asn Arg Thr Pro Glu Asn Phe Pro Cys Lys Asn Leu
    210                 215                 220
Asp Glu Asn Tyr Cys Arg Asn Pro Asp Gly Lys Arg Ala Pro Trp Cys
225                 230                 235                 240
His Thr Thr Asn Ser Gln Val Arg Trp Glu Tyr Cys Lys Ile Pro Ser
                245                 250                 255
Cys Asp Ser Ser Pro Val Ser Thr Glu Gln Leu Ala Pro Thr Ala Pro
            260                 265                 270
Pro Glu Leu Thr Pro Val Val Gln Asp Cys Tyr His Gly Asp Gly Gln
        275                 280                 285
Ser Tyr Arg Gly Thr Ser Ser Thr Thr Thr Thr Gly Lys Lys Cys Gln

290                 295                 300
Ser Trp Ser Ser Met Thr Pro His Arg His Gln Lys Thr Pro Glu Asn
305                 310                 315                 320
Tyr Pro Asn Ala Gly Leu Thr Met Asn Tyr Cys Arg Asn Pro Asp Ala
                325                 330                 335
Asp Lys Gly Pro Trp Cys Phe Thr Thr Asp Pro Ser Val Arg Trp Glu
            340                 345                 350
Tyr Cys Asn Leu Lys Lys Cys Ser Gly Thr Glu Ala
        355                 360

<210> 70
<211> 184
<212> PRT
<213> human

<400> 70
Met Ser Glu Glu Glu Cys Met His Cys Ser Gly Glu Asn Tyr Asp Gly
 1               5                  10                  15
Lys Ile Ser Lys Thr Met Ser Gly Leu Glu Cys Gln Ala Trp Asp Ser
            20                  25                  30
Gln Ser Pro His Ala His Gly Tyr Ile Pro Ser Lys Phe Pro Asn Lys
        35                  40                  45
Asn Leu Lys Lys Asn Tyr Cys Arg Asn Pro Asp Arg Glu Leu Arg Pro
    50                  55                  60
Trp Cys Phe Thr Thr Asp Pro Asn Lys Arg Trp Glu Leu Cys Asp Ile
65                  70                  75                  80
Pro Arg Cys Thr Thr Pro Pro Pro Ser Ser Gly Pro Thr Tyr Gln Cys
                85                  90                  95
Leu Lys Gly Thr Gly Glu Asn Tyr Arg Gly Asn Val Ala Val Thr Val
            100                 105                 110
Ser Gly His Thr Cys Gln His Trp Ser Ala Gln Thr Pro His Thr His
        115                 120                 125
Asn Arg Thr Pro Glu Asn Phe Pro Cys Lys Asn Leu Asp Glu Asn Tyr
    130                 135                 140
Cys Arg Asn Pro Asp Gly Lys Arg Ala Pro Trp Cys His Thr Thr Asn
145                 150                 155                 160
Ser Gln Val Arg Trp Glu Tyr Cys Lys Ile Pro Ser Cys Asp Ser Ser
                165                 170                 175
Pro Val Ser Thr Glu Gln Leu Ala
            180

<210> 71
<211> 280
<212> PRT
<213> human

<400> 71
Met Ser Glu Glu Glu Cys Met His Cys Ser Gly Glu Asn Tyr Asp Gly
 1               5                  10                  15
Lys Ile Ser Lys Thr Met Ser Gly Leu Glu Cys Gln Ala Trp Asp Ser
            20                  25                  30
Gln Ser Pro His Ala His Gly Tyr Ile Pro Ser Lys Phe Pro Asn Lys
        35                  40                  45
Asn Leu Lys Lys Asn Tyr Cys Arg Asn Pro Asp Arg Glu Leu Arg Pro
    50                  55                  60
Trp Cys Phe Thr Thr Asp Pro Asn Lys Arg Trp Glu Leu Cys Asp Ile
65                  70                  75                  80
Pro Arg Cys Thr Thr Pro Pro Pro Ser Ser Gly Pro Thr Tyr Gln Cys
                85                  90                  95
Leu Lys Gly Thr Gly Glu Asn Tyr Arg Gly Asn Val Ala Val Thr Val
            100                 105                 110
Ser Gly His Thr Cys Gln His Trp Ser Ala Gln Thr Pro His Thr His
        115                 120                 125
Asn Arg Thr Pro Glu Asn Phe Pro Cys Lys Asn Leu Asp Glu Asn Tyr
    130                 135                 140
Cys Arg Asn Pro Asp Gly Lys Arg Ala Pro Trp Cys His Thr Thr Asn
145                 150                 155                 160
Ser Gln Val Arg Trp Glu Tyr Cys Lys Ile Pro Ser Cys Asp Ser Ser

                    165                     170                     175
Pro Val Ser Thr Glu Gln Leu Ala Pro Thr Ala Pro Pro Glu Leu Thr
                180                     185                     190
Pro Val Val Gln Asp Cys Tyr His Gly Asp Gly Gln Ser Tyr Arg Gly
            195                     200                     205
Thr Ser Ser Thr Thr Thr Thr Gly Lys Lys Cys Gln Ser Trp Ser Ser
        210                     215                     220
Met Thr Pro His Arg His Gln Lys Thr Pro Glu Asn Tyr Pro Asn Ala
225                     230                     235                     240
Gly Leu Thr Met Asn Tyr Cys Arg Asn Pro Asp Ala Asp Lys Gly Pro
                245                     250                     255
Trp Cys Phe Thr Thr Asp Pro Ser Val Arg Trp Glu Tyr Cys Asn Leu
                260                     265                     270
Lys Lys Cys Ser Gly Thr Glu Ala
        275                     280

        <210> 72
        <211> 268
        <212> PRT
        <213> human

        <400> 72
Met Ser Val Tyr Leu Ser Glu Cys Lys Thr Gly Asn Gly Lys Asn Tyr
 1               5                  10                  15
Arg Gly Thr Met Ser Lys Thr Lys Asn Gly Ile Thr Cys Gln Lys Trp
                20                  25                  30
Ser Ser Thr Ser Pro His Arg Pro Arg Phe Ser Pro Ala Thr His Pro
            35                  40                  45
Ser Glu Gly Leu Glu Glu Asn Tyr Cys Arg Asn Pro Asp Asn Asp Pro
        50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Cys Met His Cys Ser Gly Glu
                85                  90                  95
Asn Tyr Asp Gly Lys Ile Ser Lys Thr Met Ser Gly Leu Glu Cys Gln
                100                 105                 110
Ala Trp Asp Ser Gln Ser Pro His Ala His Gly Tyr Ile Pro Ser Lys
            115                 120                 125
Phe Pro Asn Lys Asn Leu Lys Lys Asn Tyr Cys Arg Asn Pro Asp Arg
        130                 135                 140
Glu Leu Arg Pro Trp Cys Phe Thr Thr Asp Pro Asn Lys Arg Trp Glu
145                 150                 155                 160
Leu Cys Asp Ile Pro Arg Cys Thr Thr Pro Pro Pro Ser Ser Gly Pro
                165                 170                 175
Thr Tyr Gln Cys Leu Lys Gly Thr Gly Glu Asn Tyr Arg Gly Asn Val
                180                 185                 190
Ala Val Thr Val Ser Gly His Thr Cys Gln His Trp Ser Ala Gln Thr
            195                 200                 205
Pro His Thr His Asn Arg Thr Pro Glu Asn Phe Pro Cys Lys Asn Leu
        210                 215                 220
Asp Glu Asn Tyr Cys Arg Asn Pro Asp Gly Lys Arg Ala Pro Trp Cys
225                 230                 235                 240
His Thr Thr Asn Ser Gln Val Arg Trp Glu Tyr Cys Lys Ile Pro Ser
                245                 250                 255
Cys Asp Ser Ser Pro Val Ser Thr Glu Gln Leu Ala
                260                 265

        <210> 73
        <211> 89
        <212> PRT
        <213> human

        <400> 73
Met Ser Val Tyr Leu Ser Glu Cys Lys Thr Gly Asn Gly Lys Asn Tyr
 1               5                  10                  15
Arg Gly Thr Met Ser Lys Thr Lys Asn Gly Ile Thr Cys Gln Lys Trp
                20                  25                  30
Ser Ser Thr Ser Pro His Arg Pro Arg Phe Ser Pro Ala Thr His Pro

35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu
                85

<210> 74
<211> 106
<212> PRT
<213> human

<400> 74
Met Ser Arg Leu Pro Val Leu Leu Leu Leu Gln Leu Leu Val Arg Pro
1               5                   10                  15
Ala Met Ala Val Tyr Leu Ser Glu Cys Lys Thr Gly Asn Gly Lys Asn
            20                  25                  30
Tyr Arg Gly Thr Met Ser Lys Thr Lys Asn Gly Ile Thr Cys Gln Lys
        35                  40                  45
Trp Ser Ser Thr Ser Pro His Arg Pro Arg Phe Ser Pro Ala Thr His
    50                  55                  60
Pro Ser Glu Gly Leu Glu Glu Asn Tyr Cys Arg Asn Pro Asp Asn Asp
65                  70                  75                  80
Pro Gln Gly Pro Trp Cys Tyr Thr Thr Asp Pro Glu Lys Arg Tyr Asp
                85                  90                  95
Tyr Cys Asp Ile Leu Glu Cys Glu Glu Glu
            100                 105

<210> 75
<211> 470
<212> PRT
<213> human

<400> 75
Met Ser Arg Leu Pro Val Leu Leu Leu Leu Gln Leu Leu Val Arg Pro
1               5                   10                  15
Ala Met Ala Val Tyr Leu Ser Glu Cys Lys Thr Gly Asn Gly Lys Asn
            20                  25                  30
Tyr Arg Gly Thr Met Ser Lys Thr Lys Asn Gly Ile Thr Cys Gln Lys
        35                  40                  45
Trp Ser Ser Thr Ser Pro His Arg Pro Arg Phe Ser Pro Ala Thr His
    50                  55                  60
Pro Ser Glu Gly Leu Glu Glu Asn Tyr Cys Arg Asn Pro Asp Asn Asp
65                  70                  75                  80
Pro Gln Gly Pro Trp Cys Tyr Thr Thr Asp Pro Glu Lys Arg Tyr Asp
                85                  90                  95
Tyr Cys Asp Ile Leu Glu Cys Glu Glu Glu Cys Met His Cys Ser Gly
            100                 105                 110
Glu Asn Tyr Asp Gly Lys Ile Ser Lys Thr Met Ser Gly Leu Glu Cys
        115                 120                 125
Gln Ala Trp Asp Ser Gln Ser Pro His Ala His Gly Tyr Ile Pro Ser
    130                 135                 140
Lys Phe Pro Asn Lys Asn Leu Lys Lys Asn Tyr Cys Arg Asn Pro Asp
145                 150                 155                 160
Arg Glu Leu Arg Pro Trp Cys Phe Thr Thr Asp Pro Asn Lys Arg Trp
                165                 170                 175
Glu Leu Cys Asp Ile Pro Arg Cys Thr Thr Pro Pro Pro Ser Ser Gly
            180                 185                 190
Pro Thr Tyr Gln Cys Leu Lys Gly Thr Gly Glu Asn Tyr Arg Gly Asn
        195                 200                 205
Val Ala Val Thr Val Ser Gly His Thr Cys Gln His Trp Ser Ala Gln
    210                 215                 220
Thr Pro His Thr His Asn Arg Thr Pro Glu Asn Phe Pro Cys Lys Asn
225                 230                 235                 240
Leu Asp Glu Asn Tyr Cys Arg Asn Pro Asp Gly Lys Arg Ala Pro Trp
                245                 250                 255
Cys His Thr Thr Asn Ser Gln Val Arg Trp Glu Tyr Cys Lys Ile Pro

260                 265                 270
Ser Cys Asp Ser Ser Pro Val Ser Thr Glu Gln Leu Ala Tyr Val Glu
        275                 280                 285
Gly Gly Gly Gly Ser Pro Gly Gly Gly Ser Gly Gly Gly Ser Asn Met
    290                 295                 300
Ala Cys Asp Leu Pro Gln Thr His Ser Leu Gly Ser Arg Arg Thr Leu
305                 310                 315                 320
Met Leu Leu Ala Gln Met Arg Arg Ile Ser Leu Phe Ser Cys Leu Lys
                325                 330                 335
Asp Arg His Asp Phe Gly Phe Pro Gln Glu Glu Phe Gly Asn Gln Phe
            340                 345                 350
Gln Lys Ala Glu Thr Ile Pro Val Leu His Glu Met Ile Gln Gln Ile
        355                 360                 365
Phe Asn Leu Phe Thr Thr Lys Asp Ser Ser Ala Ala Trp Asp Glu Asp
    370                 375                 380
Leu Leu Asp Lys Phe Cys Thr Glu Leu Tyr Gln Gln Leu Asn Asp Leu
385                 390                 395                 400
Glu Ala Cys Val Met Gln Glu Glu Arg Val Gly Glu Thr Pro Leu Met
                405                 410                 415
Asn Val Asp Ser Ile Leu Ala Val Lys Lys Tyr Phe Arg Arg Ile Thr
            420                 425                 430
Leu Tyr Leu Thr Glu Lys Lys Tyr Ser Pro Cys Ala Trp Glu Val Val
        435                 440                 445
Arg Ala Glu Ile Met Arg Ser Leu Ser Leu Ser Thr Asn Leu Gln Glu
    450                 455                 460
Arg Leu Arg Arg Lys Glu
465                 470

<210> 76
<211> 183
<212> PRT
<213> human

<400> 76
His Ser His Arg Asp Phe Gln Pro Val Leu His Leu Val Ala Leu Asn
1               5                   10                  15
Ser Pro Leu Ser Gly Gly Met Arg Gly Ile Arg Gly Ala Asp Phe Gln
            20                  25                  30
Cys Phe Gln Gln Ala Arg Ala Val Gly Leu Ala Gly Thr Phe Arg Ala
        35                  40                  45
Phe Leu Ser Ser Arg Leu Gln Asp Leu Tyr Ser Ile Val Arg Arg Ala
    50                  55                  60
Asp Arg Ala Ala Val Pro Ile Val Asn Leu Lys Asp Glu Leu Leu Phe
65                  70                  75                  80
Pro Ser Trp Glu Ala Leu Phe Ser Gly Ser Glu Gly Pro Leu Lys Pro
                85                  90                  95
Gly Ala Arg Ile Phe Ser Phe Asp Gly Lys Asp Val Leu Arg His Pro
            100                 105                 110
Thr Trp Pro Gln Lys Ser Val Trp His Gly Ser Asp Pro Asn Gly Arg
        115                 120                 125
Arg Leu Thr Glu Ser Tyr Cys Glu Thr Trp Arg Thr Glu Ala Pro Ser
    130                 135                 140
Ala Thr Gly Gln Ala Ser Ser Leu Leu Gly Gly Arg Leu Leu Gly Gln
145                 150                 155                 160
Ser Ala Ala Ser Cys His His Ala Tyr Ile Val Leu Cys Ile Glu Asn
                165                 170                 175
Ser Phe Met Thr Ala Ser Lys
            180

<210> 77
<211> 810
<212> PRT
<213> human

<400> 77

<210> 78
<211> 127
<212> PRT
<213> human

<400> 78
Gly Pro Thr Gly Thr Gly Glu Ser Lys Cys Pro Leu Met Val Lys Val
1               5                   10                  15
Leu Asp Ala Val Arg Gly Ser Pro Ala Ile Asn Val Ala Val His Val
            20                  25                  30
Phe Arg Lys Ala Ala Asp Asp Thr Trp Glu Pro Phe Ala Ser Gly Lys
        35                  40                  45
Thr Ser Glu Ser Gly Glu Leu His Gly Leu Thr Thr Glu Glu Glu Phe
    50                  55                  60
Val Glu Gly Ile Tyr Lys Val Glu Ile Asp Thr Lys Ser Tyr Trp Lys
65                  70                  75                  80
Ala Leu Gly Ile Ser Pro Phe His Glu His Ala Glu Val Val Phe Thr
                85                  90                  95
Ala Asn Asp Ser Gly Pro Arg Arg Tyr Thr Ile Ala Ala Leu Leu Ser
            100                 105                 110
Pro Tyr Ser Tyr Ser Thr Thr Ala Val Val Thr Asn Pro Lys Glu
        115                 120                 125

<210> 79
<211> 18
<212> PRT
<213> human

<400> 79
Tyr Val Glu Gly Gly Gly Gly Ser Pro Gly Gly Gly Ser Gly Gly Gly
1               5                   10                  15
Ser Asn

<210> 80
<211> 4

<212> PRT
<213> human

<400> 80
Gly Gly Gly Ser
1

<210> 81
<211> 8
<212> PRT
<213> human

<400> 81
Gly Gly Gly Ser Gly Gly Gly Ser
1               5

<210> 82
<211> 12
<212> PRT
<213> human

<400> 82
Gly Gly Gly Ser Gly Gly Gly Ser Gly Gly Gly Ser
1               5                   10

<210> 83
<211> 7
<212> PRT
<213> human

<400> 83
Ser Gly Gly Ser Gly Gly Ser
1               5

<210> 84
<211> 6
<212> PRT
<213> human

<400> 84
Glu Phe Gly Asn Met Ala
1               5

<210> 85
<211> 7
<212> PRT
<213> human

<400> 85
Glu Phe Gly Gly Asn Met Ala
1               5

<210> 86
<211> 10
<212> PRT
<213> human

<400> 86
Glu Phe Gly Gly Asn Gly Gly Asn Met Ala
1               5                   10

<210> 87
<211> 7
<212> PRT
<213> human

<400> 87
Gly Gly Ser Asp Met Ala Gly

1                   5

        <210> 88
        <211> 4
        <212> PRT
        <213> human

        <400> 88
Gly Gly Gly Ser
1

        <210> 89
        <211> 5
        <212> PRT
        <213> human

        <400> 89
Gly Gly Gly Gly Ser
1               5

        <210> 90
        <211> 6
        <212> PRT
        <213> human

        <400> 90
Gly Gly Gly Gly Gly Ser
1               5

        <210> 91
        <211> 3
        <212> PRT
        <213> human

        <400> 91
Ala Gly Ser
1

        <210> 92
        <211> 7
        <212> PRT
        <213> human

        <400> 92
Gly Gly Gly Gly Gly Gly Ser
1               5

        <210> 93
        <211> 36
        <212> PRT
        <213> human

        <400> 93
Gly Gly Gly Ser Gly Gly Gly Ser Gly Gly Gly Ser Glu Gly Gly Gly
1               5                   10                  15
Ser Glu Gly Gly Gly Ser Glu Gly Gly Gly Ser Glu Gly Gly Gly Ser
            20                  25                  30
Gly Gly Gly Ser
        35

        <210> 94
        <211> 24
        <212> PRT
        <213> human

        <400> 94
Ile Ser Glu Pro Ser Gly Pro Ile Ser Thr Ile Asn Pro Ser Pro Pro
1               5                   10                  15

Ser Lys Glu Ser His Lys Ser Pro
            20

      <210> 95
      <211> 28
      <212> PRT
      <213> human

      <400> 95
Ile Glu Gly Arg Ile Ser Glu Pro Ser Gly Pro Ile Ser Thr Ile Asn
 1               5                   10                  15
Pro Ser Pro Pro Ser Lys Glu Ser His Lys Ser Pro
            20                  25

      <210> 96
      <211> 28
      <212> DNA
      <213> human

      <400> 96
catgccatgg cagtgtatct ctcagtgc                                          28

      <210> 97
      <211> 30
      <212> DNA
      <213> human

      <400> 97
catgccatgg cagtttatct ttcagagtgt                                        30

      <210> 98
      <211> 34
      <212> DNA
      <213> human

      <400> 98
gatcaagctt ttattaagcc aattgttccg tgga                                   34

      <210> 99
      <211> 34
      <212> DNA
      <213> human

      <400> 99
gatcaagctt ttattacgct tctgttcctg agca                                   34

      <210> 100
      <211> 34
      <212> DNA
      <213> human

      <400> 100
gatcaagctt ttattaaagc aggacaacag gcgg                                   34

      <210> 101
      <211> 48
      <212> DNA
      <213> human

      <400> 101
gatcacatgt chgargarga rtgyatgcay tgcagtggag aaaactat                    48

      <210> 102
      <211> 48
      <212> DNA
      <213> human

      <400> 102

gatcacatgt chgtbtayct btchgartgy aagactggga atggaaag 48

<210> 103
<211> 48
<212> DNA
<213> human

<400> 103
gatcacatgt chctbttyga raaraargtb tatctctcag agtgcaag 48

<210> 104
<211> 34
<212> DNA
<213> human

<400> 104
gatcggatcc gccaccatgg aacataagga agtg 34

<210> 105
<211> 31
<212> DNA
<213> human

<400> 105
gatcggatcc ttaatcaaat gaaggggccg c 31

<210> 106
<211> 25
<212> DNA
<213> human

<400> 106
gatcccatgg cagtgtatct gtccg 25

<210> 107
<211> 40
<212> DNA
<213> human

<400> 107
gatcaagctt tccagactcg agcgcttctg ttcctgagca 40

<210> 108
<211> 27
<212> DNA
<213> human

<400> 108
tgtccatcac cgtggtagca gtcctgg 27

<210> 109
<211> 33
<212> DNA
<213> human

<400> 109
tgccatacaa cgaattccca agtgcggtgg gag 33

<210> 110
<211> 30
<212> DNA
<213> human

<400> 110
catgccatgg cagtttatct ttcagagtgt 30

<210> 111
<211> 34

<212> DNA
<213> human

<400> 111
gatcaagctt ttattattcc tcttcacact caag                                    34

<210> 112
<211> 281
<212> DNA
<213> human

<400> 112
ccatggcagt ttatctttca gagtgtaaga ctgggaatgg aaagaattac agagggacga        60
tgtccaaaac aaaaaatggc atcacctgtc aaaaatggag ttccacttct ccccacagac       120
ctagattctc acctgctaca cacccctcag agggactgga ggagaactac tgcaggaatc      180
cagacaacga tccgcagggg ccctggtgct atactactga tccagaaaag agatatgact       240
actgcgacat tcttgagtgt gaagaggaat aataaaagct t                           281

<210> 113
<211> 89
<212> PRT
<213> human

<400> 113
Met Ala Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu
                85

SEQUENCE LISTING

<110> Bolanowski, Mark A.
      Caparon, Maire H.
      Casperson, Gerald F.
      Gregory, Susan A.
      Klein, Barbara K.
      McKearn, John P.

<120> Multi-functional Proteins

<130> C-3026

<150> 60/060,609
<151> 1997-10-02

<160> 113

<170> FastSEQ for Windows Version 3.0

<210> 1
<211> 32
<212> DNA
<213> human

<400> 1
gatcgaccat ggcttgtgat ctgcctcaaa cc                                32

<210> 2
<211> 36
<212> DNA
<213> human

<400> 2
cgatcgaagc ttattattcc ttacttctta aacttt                            36

<210> 3
<211> 115
<212> DNA
<213> human

<400> 3
gatcgaccat ggcttgtgat ctgccgcaaa ctcatagcct gggtagccgt cgcaccctga    60
tgctgctggc tcagatgcgc cgtatctctc ttttctcctg cttgaaggac agaca        115

<210> 4
<211> 61
<212> DNA
<213> human

<400> 4
ccggggagcc tccaccgccc tctacgtatt ccttacgacg cagacgttct tgcaggttgg 60
t 61

<210> 5
<211> 79
<212> DNA
<213> human

<400> 5
ccggggagcc tccaccgccc tctacgtatt ccttacttct taaactttct tgcaagtttg 60
ttgacaaaga aaaagatct 79

<210> 6
<211> 79
<212> DNA
<213> human

<400> 6
catgagatct ttttctttgt caacaaactt gcaagaaagt ttaagaagta aggaatacgt 60
agagggcggt ggaggctcc 79

<210> 7
<211> 38
<212> DNA
<213> human

<400> 7
ccggggagcc tccaccgccc tctacgtatt cctcttca 38

<210> 8
<211> 37
<212> DNA
<213> human

<400> 8
gtgtgaagag gaatacgtag agggcggtgg aggctcc 37

<210> 9
<211> 32
<212> DNA
<213> human

<400> 9
ccggggagcc tccaccgccc tctacgtaag cc 32

<210> 10
<211> 32
<212> DNA
<213> human

<400> 10
aattggctta cgtagagggc ggtggaggct cc 32

<210> 11
<211> 80
<212> DNA
<213> human

<400> 11
gatcttcaac ctgtttacca ccaaagattc ttctgctgct tgggatgagg acctgctgga 60
caaattctgc accgaactgt 80

<210> 12
<211> 84
<212> DNA
<213> human

<400> 12
ctgctggtac agttcggtgc agaatttgtc cagcaggtcc tcatcccaag cagcagaaga 60
atctttggtg gtaaacaggt tgaa 84

<210> 13
<211> 80
<212> DNA
<213> human

<400> 13
accagcagct gaacgacctc gaagcctgtg tgatgcagga ggagcgtgtg ggtgaaactc 60
cgctgatgaa cgtggactcc 80

<210> 14
<211> 80
<212> DNA
<213> human

<400> 14
gccaggatgg agtccacgtt catcagcgga gtttcaccca cacgctcctc ctgcatcaca 60
caggcttcga ggtcgttcag 80

<210> 15
<211> 80
<212> DNA
<213> human

<400> 15
atcctggctg tgaagaaata cttccgtcgt atcactctgt atctgactga gaagaaatac 60
agcccgtgtg cttgggaggt 80

<210> 16
<211> 80
<212> DNA
<213> human

<400> 16
cacgaacaac ctcccaagca cacgggctgt atttcttctc agtcagatac agagtgatac 60
gacggaagta tttcttcaca 80

<210> 17
<211> 75
<212> DNA
<213> human

<400> 17
tgttcgtgct gaaatcatgc gttccctgtc tctgtctacc aacctgcaag aacgtctgcg 60
tcgtaaggaa taata 75

<210> 18
<211> 71
<212> DNA
<213> human

<400> 18
agcttattat tccttacgac gcagacgttc ttgcaggttg gtagacagag acagggaacg 60
catgatttca g 71

<210> 19
<211> 29
<212> DNA
<213> human

<400> 19
   ggaaaaaggg ccccttggtg ccatacaac 29

<210> 20
<211> 29
<212> DNA
<213> human

<400> 20
   gttgtatggc accaaggggc cctttttcc 29

<210> 21
<211> 30
<212> DNA
<213> human

<400> 21
catgccatgg cagtttatct ttcagagtgt 30

<210> 22
<211> 34
<212> DNA
<213> human

<400> 22
gatcaagctt ttattaagcc aattgttccg tgga 34

<210> 23
<211> 32
<212> DNA
<213> human

<400> 23
aattggctta cgtagagggc ggtggaggct cc                                32

<210> 24
<211> 32
<212> DNA
<213> human

<400> 24
ccggggagcc tccaccgccc tctacgtaag cc                                32

<210> 25
<211> 59
<212> DNA
<213> human

<400> 25
ctaccaacct gcaagaacgt ctgcgtcgta aggaatacgt agagggcggt ggaggctcc      59

<210> 26
<211> 61
<212> DNA
<213> human

<400> 26
ccggggagcc tccaccgccc tctacgtatt ccttacgacg cagacgttct tgcaggttgg      60
t                                                                  61

<210> 27
<211> 507
<212> DNA
<213> human

<400> 27
atggcttgtg atctgcctca aacccacagc ctgggtagca ggaggacctt gatgctcctg      60
gcacagatga ggagaatctc tcttttctcc tgcttgaagg acagacatga ctttggattt     120
ccccaggagg agtttggcaa ccagttccaa aaggctgaaa ccatccctgt cctccatgag     180
atgatccagc agatcttcaa tctcttcagc acaaaggact catctgctgc ttgggatgag     240
accctcctag acaaattcta cactgaactc taccagcagc tgaatgacct ggaagcctgt     300
gtgatacagg gggtgggggt gacagagact cccctgatga aggaggactc cattctggct     360
gtgaggaaat acttccaaag aatcactctc tatctgaaag agaagaaata cagcccttgt     420
gcctgggagg ttgtcagagc agaaatcatg agatcttttt ctttgtcaac aaacttgcaa     480
gaaagtttaa gaagtaagga ataataa                                     507

<210> 28
<211> 507
<212> DNA
<213> human

<400> 28
atggcttgtg atctgccgca aactcatagc ctgggtagcc gtcgcaccct gatgctgctg      60
gctcagatgc gccgtatctc tcttttctcc tgcttgaagg acagacatga ctttggattt     120
ccccaggagg agtttggcaa ccagttccaa aaggctgaaa ccatccctgt cctccatgag     180

atgatccagc agatcttcaa tctcttcagc acaaaggact catctgctgc ttgggatgag 240
accctcctag acaaattcta cactgaactc taccagcagc tgaatgacct ggaagcctgt 300
gtgatacagg gggtgggggt gacagagact cccctgatga aggaggactc cattctggct 360
gtgaggaaat acttccaaag aatcactctc tatctgaaag agaagaaata cagcccttgt 420
gcctgggagg ttgtcagagc agaaatcatg agatcttttt ctttgtcaac aaacttgcaa 480
gaaagtttaa gaagtaagga ataataa 507

<210> 29
<211> 822
<212> DNA
<213> human

<400> 29
atggcttgtg atctgccgca aactcatagc ctgggtagcc gtcgcaccct gatgctgctg 60
gctcagatgc gccgtatctc tcttttctcc tgcttgaagg acagacatga ctttggattt 120
ccccaggagg agtttggcaa ccagttccaa aaggctgaaa ccatccctgt cctccatgag 180
atgatccagc agatcttcaa cctgtttacc accaaagatt cttctgctgc ttgggatgag 240
gacctgctgg acaaattctg caccgaactg taccagcagc tgaacgacct cgaagcctgt 300
gtgatgcagg aggagcgtgt gggtgaaact ccgctgatga acgtggactc catcctggct 360
gtgaagaaat acttccgtcg tatcactctg tatctgactg agaagaaata cagcccgtgt 420
gcttgggagg ttgttcgtgc tgaaatcatg cgttccctgt ctctgtctac caacctgcaa 480
gaacgtctgc gtcgtaagga atacgtagag ggcggtggag gctccccggg tggtggttct 540
ggcggcggct ccaacatggc agtttatctt tcagagtgta agactgggaa tggaaagaat 600
tacagaggga cgatgtccaa aacaaaaaat ggcatcacct gtcaaaaatg gagttccact 660
tctccccaca gacctagatt ctcacctgct acacacccct cagagggact ggaggagaac 720
tactgcagga atccagacaa cgatccgcag gggccctggt gctatactac tgatccagaa 780
aagagatatg actactgcga cattcttgag tgtgaagagg aa 822

<210> 30
<211> 1359
<212> DNA
<213> human

<400> 30
atggcttgtg atctgccgca aactcatagc ctgggtagcc gtcgcaccct gatgctgctg 60
gctcagatgc gccgtatctc tcttttctcc tgcttgaagg acagacatga ctttggattt 120
ccccaggagg agtttggcaa ccagttccaa aaggctgaaa ccatccctgt cctccatgag 180
atgatccagc agatcttcaa cctgtttacc accaaagatt cttctgctgc ttgggatgag 240
gacctgctgg acaaattctg caccgaactg taccagcagc tgaacgacct cgaagcctgt 300
gtgatgcagg aggagcgtgt gggtgaaact ccgctgatga acgtggactc catcctggct 360
gtgaagaaat acttccgtcg tatcactctg tatctgactg agaagaaata cagcccgtgt 420
gcttgggagg ttgttcgtgc tgaaatcatg cgttccctgt ctctgtctac caacctgcaa 480
gaacgtctgc gtcgtaagga atacgtagag ggcggtggag gctccccggg tggtggttct 540
ggcggcggct ccaacatggc agtttatctt tcagagtgta agactgggaa tggaaagaat 600
tacagaggga cgatgtccaa aacaaaaaat ggcatcacct gtcaaaaatg gagttccact 660
tctccccaca gacctagatt ctcacctgct acacacccct cagagggact ggaggagaac 720
tactgcagga atccagacaa cgatccgcag gggccctggt gctatactac tgatccagaa 780
aagagatatg actactgcga cattcttgag tgtgaagagg aatgtatgca ttgcagtgga 840
gaaaactatg acggcaaaat ttccaagacc atgtctggac tggaatgcca ggcctgggac 900
tctcagagcc cacacgctca tggatacatt ccttccaaat ttccaaacaa gaacctgaag 960
aagaattact gtcgtaaccc cgatagggag ctgcggcctt ggtgtttcac caccgacccc 1020
aacaagcgct gggaactttg tgacatcccc cgctgcacaa cacctccacc atcttctggt 1080
cccacctacc agtgtctgaa gggaacaggt gaaaactatc gcgggaatgt ggctgttacc 1140
gtgtccgggc acacctgtca gcactggagt gcacagaccc ctcacacaca taacaggaca 1200

ccagaaaact tcccctgcaa aaatttggat gaaaactact gccgcaatcc tgacggaaaa 1260
agggcccctt ggtgccatac aaccaacagc caagtgcggt gggagtactg taagataccg 1320
tcctgtgact cctccccagt atccacggaa caattggct 1359

<210> 31
<211> 822
<212> DNA
<213> human

<400> 31
atggcttgtg atctgccgca aactcatagc ctgggtagcc gtcgcaccct gatgctgctg 60
gctcagatgc gccgtatctc tcttttctcc tgcttgaagg acagacatga ctttggattt 120
ccccaggagg agtttggcaa ccagttccaa aaggctgaaa ccatccctgt cctccatgag 180
atgatccagc agatcttcaa tctcttcagc acaaaggact catctgctgc ttgggatgag 240
accctcctag acaaattcta cactgaactc taccagcagc tgaatgacct ggaagcctgt 300
gtgatacagg gggtgggggt gacagagact cccctgatga aggaggactc cattctggct 360
gtgaggaaat acttccaaag aatcactctc tatctgaaag agaagaaata cagcccttgt 420
gcctgggagg ttgtcagagc agaaatcatg agatcttttt ctttgtcaac aaacttgcaa 480
gaaagtttaa gaagtaagga atacgtagag ggcggtggag gctccccggg tggtggttct 540
ggcggcggct ccaacatggc agtttatctt tcagagtgta agactgggaa tggaaagaat 600
tacagaggga cgatgtccaa aacaaaaaat ggcatcacct gtcaaaaatg gagttccact 660
tctccccaca gacctagatt ctcacctgct acacacccct cagagggact ggaggagaac 720
tactgcagga atccagacaa cgatccgcag gggccctggt gctatactac tgatccagaa 780
aagagatatg actactgcga cattcttgag tgtgaagagg aa 822

<210> 32
<211> 1359
<212> DNA
<213> human

<400> 32
atggcttgtg atctgccgca aactcatagc ctgggtagcc gtcgcaccct gatgctgctg 60
gctcagatgc gccgtatctc tcttttctcc tgcttgaagg acagacatga ctttggattt 120
ccccaggagg agtttggcaa ccagttccaa aaggctgaaa ccatccctgt cctccatgag 180
atgatccagc agatcttcaa tctcttcagc acaaaggact catctgctgc ttgggatgag 240
accctcctag acaaattcta cactgaactc taccagcagc tgaatgacct ggaagcctgt 300
gtgatacagg gggtgggggt gacagagact cccctgatga aggaggactc cattctggct 360
gtgaggaaat acttccaaag aatcactctc tatctgaaag agaagaaata cagcccttgt 420
gcctgggagg ttgtcagagc agaaatcatg agatcttttt ctttgtcaac aaacttgcaa 480
gaaagtttaa gaagtaagga atacgtagag ggcggtggag gctccccggg tggtggttct 540
ggcggcggct ccaacatggc agtttatctt tcagagtgta agactgggaa tggaaagaat 600
tacagaggga cgatgtccaa aacaaaaaat ggcatcacct gtcaaaaatg gagttccact 660
tctccccaca gacctagatt ctcacctgct acacacccct cagagggact ggaggagaac 720
tactgcagga atccagacaa cgatccgcag gggccctggt gctatactac tgatccagaa 780
aagagatatg actactgcga cattcttgag tgtgaagagg aatgtatgca ttgcagtgga 840
gaaaactatg acggcaaaat ttccaagacc atgtctggac tggaatgcca ggcctgggac 900
tctcagagcc cacacgctca tggatacatt ccttccaaat ttccaaacaa gaacctgaag 960
aagaattact gtcgtaaccc cgatagggag ctgcggcctt ggtgtttcac caccgacccc 1020
aacaagcgct gggaactttg tgacatcccc cgctgcacaa cacctccacc atcttctggt 1080
cccacctacc agtgtctgaa gggaacaggt gaaaactatc gcgggaatgt ggctgttacc 1140
gtgtccgggc acacctgtca gcactggagt gcacagaccc ctcacacaca taacaggaca 1200
ccagaaaact tcccctgcaa aaatttggat gaaaactact gccgcaatcc tgacggaaaa 1260
agggcccctt ggtgccatac aaccaacagc caagtgcggt gggagtactg taagataccg 1320
tcctgtgact cctccccagt atccacggaa caattggct 1359

<210> 33
<211> 1359
<212> DNA
<213> human

<400> 33
atggcagttt atctttcaga gtgtaagact gggaatggaa agaattacag agggacgatg 60
tccaaaacaa aaaatggcat cacctgtcaa aaatggagtt ccacttctcc ccacagacct 120
agattctcac ctgctacaca cccctcagag ggactggagg agaactactg caggaatcca 180
gacaacgatc cgcaggggcc ctggtgctat actactgatc cagaaaagag atatgactac 240
tgcgacattc ttgagtgtga agaggaatgt atgcattgca gtggagaaaa ctatgacggc 300
aaaatttcca agaccatgtc tggactggaa tgccaggcct gggactctca gagcccacac 360
gctcatggat acattccttc caaatttcca aacaagaacc tgaagaagaa ttactgtcgt 420
aaccccgata gggagctgcg gccttggtgt ttcaccaccg accccaacaa gcgctgggaa 480
ctttgtgaca tcccccgctg cacaacacct ccaccatctt ctggtcccac ctaccagtgt 540
ctgaagggaa caggtgaaaa ctatcgcggg aatgtggctg ttaccgtgtc cgggcacacc 600
tgtcagcact ggagtgcaca gacccctcac acacataaca ggacaccaga aaacttcccc 660
tgcaaaaatt tggatgaaaa ctactgccgc aatcctgacg gaaaaagggc cccttggtgc 720
catacaacca acagccaagt gcggtgggag tactgtaaga taccgtcctg tgactcctcc 780
ccagtatcca cggaacaatt ggcttacgta gagggcggtg gaggctcccc gggtggtggt 840
tctggcggcg gctccaacat ggcttgtgat ctgccgcaaa ctcatagcct gggtagccgt 900
cgcaccctga tgctgctggc tcagatgcgc cgtatctctc ttttctcctg cttgaaggac 960
agacatgact ttggatttcc ccaggaggag tttggcaacc agttccaaaa ggctgaaacc 1020
atccctgtcc tccatgagat gatccagcag atcttcaatc tcttcagcac aaaggactca 1080
tctgctgctt gggatgagac cctcctagac aaattctaca ctgaactcta ccagcagctg 1140
aatgacctgg aagcctgtgt gatacagggg gtgggggtga cagagactcc cctgatgaag 1200
gaggactcca ttctggctgt gaggaaatac ttccaaagaa tcactctcta tctgaaagag 1260
aagaaataca gcccttgtgc ctgggaggtt gtcagagcag aaatcatgag atctttttct 1320
ttgtcaacaa acttgcaaga aagtttaaga agtaaggaa 1359

<210> 34
<211> 822
<212> DNA
<213> human

<400> 34
atggcagttt atctttcaga gtgtaagact gggaatggaa agaattacag agggacgatg 60
tccaaaacaa aaaatggcat cacctgtcaa aaatggagtt ccacttctcc ccacagacct 120
agattctcac ctgctacaca cccctcagag ggactggagg agaactactg caggaatcca 180
gacaacgatc cgcaggggcc ctggtgctat actactgatc cagaaaagag atatgactac 240
tgcgacattc ttgagtgtga agaggaatac gtagagggcg gtggaggctc cccgggtggt 300
ggttctggcg gcggctccaa catggcttgt gatctgccgc aaactcatag cctgggtagc 360
cgtcgcaccc tgatgctgct ggctcagatg cgccgtatct ctcttttctc ctgcttgaag 420
gacagacatg actttggatt tccccaggag gagtttggca accagttcca aaaggctgaa 480
accatccctg tcctccatga gatgatccag cagatcttca acctgtttac caccaaagat 540
tcttctgctg cttgggatga ggacctgctg gacaaattct gcaccgaact gtaccagcag 600
ctgaacgacc tcgaagcctg tgtgatgcag gaggagcgtg tgggtgaaac tccgctgatg 660
aacgtggact ccatcctggc tgtgaagaaa tacttccgtc gtatcactct gtatctgact 720
gagaagaaat acagcccgtg tgcttgggag gttgttcgtg ctgaaatcat gcgttccctg 780
tctctgtcta ccaacctgca agaacgtctg cgtcgtaagg aa 822

<210> 35
<211> 822

<212> DNA
<213> human

<400> 35
atggcagttt atctttcaga gtgtaagact gggaatggaa agaattacag agggacgatg 60
tccaaaacaa aaaatggcat cacctgtcaa aaatggagtt ccacttctcc ccacagacct 120
agattctcac ctgctacaca cccctcagag ggactggagg agaactactg caggaatcca 180
gacaacgatc cgcaggggcc ctggtgctat actactgatc cagaaaagag atatgactac 240
tgcgacattc ttgagtgtga agaggaatac gtagagggcg gtggaggctc cccgggtggt 300
ggttctggcg gcggctccaa catggcttgt gatctgccgc aaactcatag cctgggtagc 360
cgtcgcaccc tgatgctgct ggctcagatg cgccgtatct ctcttttctc ctgcttgaag 420
gacagacatg actttggatt tccccaggag gagtttggca accagttcca aaaggctgaa 480
accatccctg tcctccatga gatgatccag cagatcttca atctcttcag cacaaaggac 540
tcatctgctg cttgggatga gaccctccta gacaaattct acactgaact ctaccagcag 600
ctgaatgacc tggaagcctg tgtgatacag ggggtggggg tgacagagac tcccctgatg 660
aaggaggact ccattctggc tgtgaggaaa tacttccaaa gaatcactct ctatctgaaa 720
gagaagaaat acagcccttg tgcctgggag gttgtcagag cagaaatcat gagatctttt 780
tctttgtcaa caaacttgca agaaagttta agaagtaagg aa 822

<210> 36
<211> 1359
<212> DNA
<213> human

<400> 36
atggcagttt atctttcaga gtgtaagact gggaatggaa agaattacag agggacgatg 60
tccaaaacaa aaaatggcat cacctgtcaa aaatggagtt ccacttctcc ccacagacct 120
agattctcac ctgctacaca cccctcagag ggactggagg agaactactg caggaatcca 180
gacaacgatc cgcaggggcc ctggtgctat actactgatc cagaaaagag atatgactac 240
tgcgacattc ttgagtgtga agaggaatgt atgcattgca gtggagaaaa ctatgacggc 300
aaaatttcca agaccatgtc tggactggaa tgccaggcct gggactctca gagcccacac 360
gctcatggat acattccttc caaatttcca aacaagaacc tgaagaagaa ttactgtcgt 420
aaccccgata gggagctgcg gccttggtgt ttcaccaccg accccaacaa gcgctgggaa 480
ctttgtgaca tcccccgctg cacaacacct ccaccatctt ctggtcccac ctaccagtgt 540
ctgaagggaa caggtgaaaa ctatcgcggg aatgtggctg ttaccgtgtc cgggcacacc 600
tgtcagcact ggagtgcaca gacccctcac acacataaca ggacaccaga aaacttcccc 660
tgcaaaaatt tggatgaaaa ctactgccgc aatcctgacg gaaaaagggc cccttggtgc 720
catacaacca acagccaagt gcggtgggag tactgtaaga taccgtcctg tgactcctcc 780
ccagtatcca cggaacaatt ggcttacgta gagggcggtg gaggctcccc gggtggtggt 840
tctggcggcg gctccaacat ggcttgtgat ctgccgcaaa ctcatagcct gggtagccgt 900
cgcaccctga tgctgctggc tcagatgcgc cgtatctctc ttttctcctg cttgaaggac 960
agacatgact ttggatttcc ccaggaggag tttggcaacc agttccaaaa ggctgaaacc 1020
atccctgtcc tccatgagat gatccagcag atcttcaacc tgtttaccac caaagattct 1080
tctgctgctt gggatgagga cctgctggac aaattctgca ccgaactgta ccagcagctg 1140
aacgacctcg aagcctgtgt gatgcaggag gagcgtgtgg gtgaaactcc gctgatgaac 1200
gtggactcca tcctggctgt gaagaaatac ttccgtcgta tcactctgta tctgactgag 1260
aagaaataca gcccgtgtgc ttgggaggtt gttcgtgctg aaatcatgcg ttccctgtct 1320
ctgtctacca acctgcaaga acgtctgcgt cgtaaggaa 1359

<210> 37
<211> 1719
<212> DNA
<213> human

<400> 37
ggatccgcca ccatggaaca taaggaagtg gttcttctac ttcttttatt tctgaaatca 60
ggtcaaggag agcctctgga tgactatgtg aatacccagg gggcttcact gttcagtgtc 120
actaagaagc agctgggagc aggaagtata gaagaatgtg cagcaaaatg tgaggaggac 180
gaagaattca cctgcagggc attccaatat cacagtaaag agcaacaatg tgtgataatg 240
gctgaaaaca ggaagtcctc cataatcatt aggatgagag atgtagtttt atttgaaaag 300
aaagtgtatc tctcagagtg caagactggg aatggaaaga attacagagg gacgatgtcc 360
aaaacaaaaa atggcatcac ctgtcaaaaa tggagttcca cttctcccca cagacctaga 420
ttctcacctg ctacacaccc ctcagaggga ctggaggaga actactgcag gaatccagac 480
aacgatccgc aggggccctg gtgctatact actgatccag aaaagagata tgactactgc 540
gacattcttg agtgtgaaga ggaatgtatg cattgcagtg gagaaaacta tgacggcaaa 600
atttccaaga ccatgtctgg actggaatgc caggcctggg actctcagag cccacacgct 660
catggataca ttccttccaa atttccaaac aagaacctga agaagaatta ctgtcgtaac 720
cccgataggg agctgcggcc ttggtgtttc accaccgacc ccaacaagcg ctgggaactt 780
tgtgacatcc cccgctgcac aacacctcca ccatcttctg gtcccaccta ccagtgtctg 840
aagggaacag gtgaaaacta tcgcgggaat gtggctgtta ccgtgtccgg gcacacctgt 900
cagcactgga gtgcacagac ccctcacaca cataacagga caccagaaaa cttcccctgc 960
aaaaatttgg atgaaaacta ctgccgcaat cctgacggaa aaagggcccc atggtgccat 1020
acaaccaaca gccaagtgcg gtgggagtac tgtaagatac cgtcctgtga ctcctcccca 1080
gtatccacgg aacagttggc tcccacagca ccacctgagc taacccctgt ggtccaggac 1140
tgctaccatg gtgatggaca gagctaccga ggcacatcct ccaccaccac cacaggaaag 1200
aagtgtcagt cttggtcatc tatgacacca caccggcacc agaagacccc agaaaactac 1260
ccaaatgctg gcctgacaat gaactactgc aggaatccag atgccgataa aggcccctgg 1320
tgttttacca cagaccccag cgtcaggtgg gagtactgca acctgaaaaa atgctcagga 1380
acagaagcga gtgttgtagc acctccgcct gttgtcctgc ttccagatgt agagactcct 1440
tccgaagaag actgtatgtt tgggaatggg aaaggatacc gaggcaagag ggcgaccact 1500
gttactggga cgccatgcca ggactgggct gcccaggagc cccatagaca cagcattttc 1560
actccagaga caaatccacg ggcgggtctg gaaaaaaatt actgccgtaa ccctgatggt 1620
gatgtaggtg gtccctggtg ctacacgaca aatccaagaa aactttacga ctactgtgat 1680
gtccctcagt gtgcggcccc ttcatttgat taaggatcc 1719

<210> 38
<211> 1167
<212> DNA
<213> human

<400> 38
ggatccacca tgagccgcct gcccgtcctg ctcctgctcc aactcctggt ccgccccgcc 60
atgtctgtgt atctctcaga gtgcaagact gggaatggaa agaattacag agggacgatg 120
tccaaaacaa aaaatggcat cacctgtcaa aaatggagtt ccacttctcc ccacagacct 180
agattctcac ctgctacaca cccctcagag ggactggagg agaactactg caggaatcca 240
gacaacgatc cgcaggggcc ctggtgctat actactgatc cagaaaagag atatgactac 300
tgcgacattc ttgagtgtga agaggaatgt atgcattgca gtggagaaaa ctatgacggc 360
aaaatttcca agaccatgtc tggactggaa tgccaggcct gggactctca gagcccacac 420
gctcatggat acattccttc caaatttcca aacaagaacc tgaagaagaa ttactgtcgt 480
aaccccgata gggagctgcg gccttggtgt ttcaccaccg accccaacaa gcgctgggaa 540
ctttgtgaca tcccccgctg cacaacacct ccaccatctt ctggtcccac ctaccagtgt 600
ctgaagggaa caggtgaaaa ctatcgcggg aatgtggctg ttaccgtgtc cgggcacacc 660
tgtcagcact ggagtgcaca gacccctcac acacataaca ggacaccaga aaacttcccc 720
tgcaaaaatt tggatgaaaa ctactgccgc aatcctgacg gaaaaagggc cccatggtgc 780
catacaacca acagccaagt gcggtgggag tactgtaaga taccgtcctg tgactcctcc 840
ccagtatcca cggaacagtt ggctcccaca gcaccacctg agctaacccc tgtggtccag 900
gactgctacc atggtgatgg acagagctac cgaggcacat cctccaccac caccacagga 960
aagaagtgtc agtcttggtc atctatgaca ccacaccggc accagaagac cccagaaaac 1020

tacccaaatg ctggcctgac aatgaactac tgcaggaatc cagatgccga taaaggcccc 1080
tggtgtttta ccacagaccc cagcgtcagg tgggagtact gcaacctgaa aaaatgctca 1140
ggaacagaag cgtgaaagct tggatcc 1167

<210> 39
<211> 879
<212> DNA
<213> human

<400> 39
ggatccacca tgagccgcct gcccgtcctg ctcctgctcc aactcctggt ccgccccgcc 60
atgtctgtgt atctctcaga gtgcaagact gggaatggaa agaattacag agggacgatg 120
tccaaaacaa aaaatggcat cacctgtcaa aaatggagtt ccacttctcc ccacagacct 180
agattctcac ctgctacaca cccctcagag ggactggagg agaactactg caggaatcca 240
gacaacgatc cgcaggggcc ctggtgctat actactgatc cagaaaagag atatgactac 300
tgcgacattc ttgagtgtga agaggaatgt atgcattgca gtggagaaaa ctatgacggc 360
aaaatttcca agaccatgtc tggactggaa tgccaggcct gggactctca gagcccacac 420
gctcatggat acattccttc caaatttcca aacaagaacc tgaagaagaa ttactgtcgt 480
aaccccgata gggagctgcg gccttggtgt ttcaccaccg accccaacaa gcgctgggaa 540
ctttgtgaca tcccccgctg cacaacacct ccaccatctt ctggtcccac ctaccagtgt 600
ctgaagggaa caggtgaaaa ctatcgcggg aatgtggctg ttaccgtgtc cgggcacacc 660
tgtcagcact ggagtgcaca gacccctcac acacataaca ggacaccaga aaacttcccc 720
tgcaaaaatt tggatgaaaa ctactgccgc aatcctgacg gaaaaagggc cccatggtgc 780
catacaacca acagccaagt gcggtgggag tactgtaaga taccgtcctg tgactcctcc 840
ccagtatcca cggaacagtt ggcttgaaag cttggatcc 879

<210> 40
<211> 915
<212> DNA
<213> human

<400> 40
ggatccacca tgagccgcct gcccgtcctg ctcctgctcc aactcctggt ccgccccgcc 60
atgtctgaag aggaatgtat gcattgcagt ggagaaaact atgacggcaa aatttccaag 120
accatgtctg gactggaatg ccaggcctgg gactctcaga gcccacacgc tcatggatac 180
attccttcca aatttccaaa caagaacctg aagaagaatt actgtcgtaa ccccgatagg 240
gagctgcggc cttggtgttt caccaccgac cccaacaagc gctgggaact ttgtgacatc 300
ccccgctgca caacacctcc accatcttct ggtcccacct accagtgtct gaagggaaca 360
ggtgaaaact atcgcgggaa tgtggctgtt accgtgtccg ggcacacctg tcagcactgg 420
agtgcacaga cccctcacac acataacagg acaccagaaa acttcccctg caaaaatttg 480
gatgaaaact actgccgcaa tcctgacgga aaaagggccc catggtgcca tacaaccaac 540
agccaagtgc ggtgggagta ctgtaagata ccgtcctgtg actcctcccc agtatccacg 600
gaacagttgg ctcccacagc accacctgag ctaacccctg tggtccagga ctgctaccat 660
ggtgatggac agagctaccg aggcacatcc tccaccacca ccacaggaaa gaagtgtcag 720
tcttggtcat ctatgacacc acaccggcac cagaagaccc cagaaaacta cccaaatgct 780
ggcctgacaa tgaactactg caggaatcca gatgccgata aaggcccctg gtgttttacc 840
acagacccca gcgtcaggtg ggagtactgc aacctgaaaa aatgctcagg aacagaagcg 900
tgaaagcttg gatcc 915

<210> 41
<211> 627
<212> DNA
<213> human

<400> 41
ggatccacca tgagccgcct gcccgtcctg ctcctgctcc aactcctggt ccgccccgcc 60
atgtctgaag aggaatgtat gcattgcagt ggagaaaact atgacggcaa aatttccaag 120
accatgtctg gactggaatg ccaggcctgg gactctcaga gcccacacgc tcatggatac 180
attccttcca aatttccaaa caagaacctg aagaagaatt actgtcgtaa ccccgatagg 240
gagctgcggc cttggtgttt caccaccgac cccaacaagc gctgggaact ttgtgacatc 300
ccccgctgca caacacctcc accatcttct ggtcccacct accagtgtct gaagggaaca 360
ggtgaaaact atcgcgggaa tgtggctgtt accgtgtccg ggcacacctg tcagcactgg 420
agtgcacaga cccctcacac acataacagg acaccagaaa acttcccctg caaaaatttg 480
gatgaaaact actgccgcaa tcctgacgga aaaagggccc catggtgcca tacaaccaac 540
agccaagtgc ggtgggagta ctgtaagata ccgtcctgtg actcctcccc agtatccacg 600
gaacaattgg cttgaaagct tggatcc 627

<210> 42
<211> 1154
<212> DNA
<213> human

<400> 42
ccatgtcact gtttgaaaaa aaagtttatc tctcagagtg caagactggg aatggaaaga 60
attacagagg gacgatgtcc aaaacaaaaa atggcatcac ctgtcaaaaa tggagttcca 120
cttctcccca cagacctaga ttctcacctg ctacacaccc ctcagaggga ctggaggaga 180
actactgcag gaatccagac aacgatccgc aggggccctg gtgctatact actgatccag 240
aaaagagata tgactactgc gacattcttg agtgtgaaga ggaatgtatg cattgcagtg 300
gagaaaacta tgacggcaaa atttccaaga ccatgtctgg actggaatgc caggcctggg 360
actctcagag cccacacgct catggataca ttccttccaa atttccaaac aagaacctga 420
agaagaatta ctgtcgtaac cccgataggg agctgcggcc ttggtgtttc accaccgacc 480
ccaacaagcg ctgggaactt tgtgacatcc cccgctgcac aacacctcca ccatcttctg 540
gtcccaccta ccagtgtctg aagggaacag gtgaaaacta tcgcgggaat gtggctgtta 600
ccgtgtccgg gcacacctgt cagcactgga gtgcacagac ccctcacaca cataacagga 660
caccagaaaa cttcccctgc aaaaatttgg atgaaaacta ctgccgcaat cctgacggaa 720
aaagggcccc atggtgccat acaaccaaca gccaagtgcg gtgggagtac tgtaagatac 780
cgtcctgtga ctcctcccca gtatccacgg aacagttggc tcccacagca ccacctgagc 840
taacccctgt ggtccaggac tgctaccatg gtgatggaca gagctaccga ggcacatcct 900
ccaccaccac cacaggaaag aagtgtcagt cttggtcatc tatgacacca caccggcacc 960
agaagacccc agaaaactac ccaaatgctg gcctgacaat gaactactgc aggaatccag 1020
atgccgataa aggcccctgg tgttttacca cagaccccag cgtcaggtgg gagtactgca 1080
acctgaaaaa atgctcagga acagaagcga gtgttgtagc acctccgcct gttgtcctgc 1140
tttaataaaa gctt 1154

<210> 43
<211> 1106
<212> DNA
<213> human

<400> 43
ccatgtcagt gtatctgtcc gagtgtaaga ctgggaatgg aaagaattac agagggacga 60
tgtccaaaac aaaaaatggc atcacctgtc aaaaatggag ttccacttct ccccacagac 120
ctagattctc acctgctaca cacccctcag agggactgga ggagaactac tgcaggaatc 180
cagacaacga tccgcagggg ccctggtgct atactactga tccagaaaag agatatgact 240
actgcgacat tcttgagtgt gaagaggaat gtatgcattg cagtggagaa aactatgacg 300
gcaaaatttc caagaccatg tctggactgg aatgccaggc ctgggactct cagagcccac 360
acgctcatgg atacattcct tccaaatttc caaacaagaa cctgaagaag aattactgtc 420
gtaaccccga tagggagctg cggccttggt gtttcaccac cgaccccaac aagcgctggg 480

aactttgtga catcccccgc tgcacaacac ctccaccatc ttctggtccc acctaccagt 540
gtctgaaggg aacaggtgaa aactatcgcg ggaatgtggc tgttaccgtg tccgggcaca 600
cctgtcagca ctggagtgca cagacccctc acacacataa caggacacca gaaaacttcc 660
cctgcaaaaa tttggatgaa aactactgcc gcaatcctga cggaaaaagg gccccatggt 720
gccatacaac caacagccaa gtgcggtggg agtactgtaa gataccgtcc tgtgactcct 780
ccccagtatc cacggaacag ttggctccca cagcaccacc tgagctaacc cctgtggtcc 840
aggactgcta ccatggtgat ggacagagct accgaggcac atcctccacc accaccacag 900
gaaagaagtg tcagtcttgg tcatctatga caccacaccg gcaccagaag accccagaaa 960
actacccaaa tgctggcctg acaatgaact actgcaggaa tccagatgcc gataaaggcc 1020
cctggtgttt taccacagac cccagcgtca ggtgggagta ctgcaacctg aaaaaatgct 1080
caggaacaga agcgtaataa aagctt 1106

<210> 44
<211> 566
<212> DNA
<213> human

<400> 44
ccatgtctga agaagaatgc atgcactgca gtggagaaaa ctatgacggc aaaatttcca 60
agaccatgtc tggactggaa tgccaggcct gggactctca gagcccacac gctcatggat 120
acattccttc caaatttcca aacaagaacc tgaagaagaa ttactgtcgt aaccccgata 180
gggagctgcg gccttggtgt ttcaccaccg accccaacaa gcgctgggaa ctttgtgaca 240
tcccccgctg cacaacacct ccaccatctt ctggtcccac ctaccagtgt ctgaagggaa 300
caggtgaaaa ctatcgcggg aatgtggctg ttaccgtgtc cgggcacacc tgtcagcact 360
ggagtgcaca gacccctcac acacataaca ggacaccaga aaacttcccc tgcaaaaatt 420
tggatgaaaa ctactgccgc aatcctgacg gaaaaagggc cccatggtgc catacaacca 480
acagccaagt gcggtgggag tactgtaaga taccgtcctg tgactcctcc ccagtatcca 540
cggaacaatt ggcttaataa aagctt 566

<210> 45
<211> 854
<212> DNA
<213> human

<400> 45
ccatgtctga ggaagaatgc atgcactgca gtggagaaaa ctatgacggc aaaatttcca 60
agaccatgtc tggactggaa tgccaggcct gggactctca gagcccacac gctcatggat 120
acattccttc caaatttcca aacaagaacc tgaagaagaa ttactgtcgt aaccccgata 180
gggagctgcg gccttggtgt ttcaccaccg accccaacaa gcgctgggaa ctttgtgaca 240
tcccccgctg cacaacacct ccaccatctt ctggtcccac ctaccagtgt ctgaagggaa 300
caggtgaaaa ctatcgcggg aatgtggctg ttaccgtgtc cgggcacacc tgtcagcact 360
ggagtgcaca gacccctcac acacataaca ggacaccaga aaacttcccc tgcaaaaatt 420
tggatgaaaa ctactgccgc aatcctgacg gaaaaagggc cccatggtgc catacaacca 480
acagccaagt gcggtgggag tactgtaaga taccgtcctg tgactcctcc ccagtatcca 540
cggaacagtt ggctcccaca gcaccacctg agctaacccc tgtggtccag gactgctacc 600
atggtgatgg acagagctac cgaggcacat cctccaccac caccacagga aagaagtgtc 660
agtcttggtc atctatgaca ccacaccggc accagaagac cccagaaaac tacccaaatg 720
ctggcctgac aatgaactac tgcaggaatc cagatgccga taaaggcccc tggtgtttta 780
ccacagaccc cagcgtcagg tgggagtact gcaacctgaa aaaatgctca ggaacagaag 840
cgtaataaaa gctt 854

<210> 46
<211> 818
<212> DNA

<213> human

<400> 46
ccatgtcagt ttatctttca gagtgtaaga ctgggaatgg aaagaattac agagggacga 60
tgtccaaaac aaaaaatggc atcacctgtc aaaaatggag ttccacttct ccccacagac 120
ctagattctc acctgctaca cacccctcag agggactgga ggagaactac tgcaggaatc 180
cagacaacga tccgcagggg ccctggtgct atactactga tccagaaaag agatatgact 240
actgcgacat tcttgagtgt gaagaggaat gtatgcattg cagtggagaa aactatgacg 300
gcaaaatttc caagaccatg tctggactgg aatgccaggc ctgggactct cagagcccac 360
acgctcatgg atacattcct tccaaatttc caaacaagaa cctgaagaag aattactgtc 420
gtaaccccga tagggagctg cggccttggt gtttcaccac cgaccccaac aagcgctggg 480
aactttgtga catcccccgc tgcacaacac ctccaccatc ttctggtccc acctaccagt 540
gtctgaaggg aacaggtgaa aactatcgcg ggaatgtggc tgttaccgtg tccgggcaca 600
cctgtcagca ctggagtgca cagacccctc acacacataa caggacacca gaaaacttcc 660
cctgcaaaaa tttggatgaa aactactgcc gcaatcctga cggaaaaagg gccccatggt 720
gccatacaac caacagccaa gtgcggtggg agtactgtaa gataccgtcc tgtgactcct 780
ccccagtatc cacggaacaa ttggcttaat aaaagctt 818

<210> 47
<211> 281
<212> DNA
<213> human

<400> 47
ccatgtccgt gtacctgtca gaatgtaaga ctgggaatgg aaagaattac agagggacga 60
tgtccaaaac aaaaaatggc atcacctgtc aaaaatggag ttccacttct ccccacagac 120
ctagattctc acctgctaca cacccctcag agggactgga ggagaactac tgcaggaatc 180
cagacaacga tccgcagggg ccctggtgct atactactga tccagaaaag agatatgact 240
actgcgacat tcttgagtgt gaagaggaat aataaaagct t 281

<210> 48
<211> 345
<212> DNA
<213> human

<400> 48
ggatccacca tgagccgcct gcccgtcctg ctcctgctcc aactcctggt ccgccccgcc 60
atggccgtgt acctgtcaga atgtaagact gggaatggaa agaattacag agggacgatg 120
tccaaaacaa aaaatggcat cacctgtcaa aaatggagtt ccacttctcc ccacagacct 180
agattctcac ctgctacaca cccctcagag ggactggagg agaactactg caggaatcca 240
gacaacgatc cgcaggggcc ctggtgctat actactgatc cagaaaagag atatgactac 300
tgcgacattc ttgagtgtga agaggaataa taaaagcttg gattc 345

<210> 49
<211> 1436
<212> DNA
<213> human

<400> 49
ggatccacca tgagccgcct gcccgtcctg ctcctgctcc aactcctggt ccgccccgcc 60
atggcagttt atctttcaga gtgtaagact gggaatggaa agaattacag agggacgatg 120
tccaaaacaa aaaatggcat cacctgtcaa aaatggagtt ccacttctcc ccacagacct 180
agattctcac ctgctacaca cccctcagag ggactggagg agaactactg caggaatcca 240
gacaacgatc cgcaggggcc ctggtgctat actactgatc cagaaaagag atatgactac 300

tgcgacattc ttgagtgtga agaggaatgt atgcattgca gtggagaaaa ctatgacggc 360
aaaatttcca agaccatgtc tggactggaa tgccaggcct gggactctca gagcccacac 420
gctcatggat acattccttc caaatttcca aacaagaacc tgaagaagaa ttactgtcgt 480
aaccccgata gggagctgcg gccttggtgt ttcaccaccg accccaacaa gcgctgggaa 540
ctttgtgaca tcccccgctg cacaacacct ccaccatctt ctggtcccac ctaccagtgt 600
ctgaagggaa caggtgaaaa ctatcgcggg aatgtggctg ttaccgtgtc cgggcacacc 660
tgtcagcact ggagtgcaca gacccctcac acacataaca ggacaccaga aaacttcccc 720
tgcaaaaatt tggatgaaaa ctactgccgc aatcctgacg gaaaaagggc cccttggtgc 780
catacaacca acagccaagt gcggtgggag tactgtaaga taccgtcctg tgactcctcc 840
ccagtatcca cggaacagtt ggcttacgta gagggcggtg gaggctcccc gggtggtggt 900
tctggcggcg gctccaacat ggcttgtgat ctgccgcaaa ctcatagcct gggtagccgt 960
cgcaccctga tgctgctggc tcagatgcgc cgtatctctc ttttctcctg cttgaaggac 1020
agacatgact ttggatttcc ccaggaggag tttggcaacc agttccaaaa ggctgaaacc 1080
atccctgtcc tccatgagat gatccagcag atcttcaacc tgtttaccac caaagattct 1140
tctgctgctt gggatgagga cctgctggac aaattctgca ccgaactgta ccagcagctg 1200
aacgacctcg aagcctgtgt gatgcaggag gagcgtgtgg gtgaaactcc gctgatgaac 1260
gtggactcca tcctggctgt gaagaaatac ttccgtcgta tcactctgta tctgactgag 1320
aagaaataca gcccgtgtgc ttgggaggtt gttcgtgctg aaatcatgcg ttccctgtct 1380
ctgtctacca acctgcaaga acgtctgcgt cgtaaggaat aataaagctt ggatcc 1436

<210> 50
<211> 552
<212> DNA
<213> human

<400> 50
cacagccacc gcgacttcca gccggtgctc cacctggttg cgctcaacag ccccctgtca 60
ggcggcatgc ggggcatccg cggggccgac ttccagtgct tccagcaggc gcgggccgtg 120
gggctggcgg gcaccttccg cgccttcctg tcctcgcgcc tgcaggacct gtacagcatc 180
gtgcgccgtg ccgaccgcgc agccgtgccc atcgtcaacc tcaaggacga gctgctgttt 240
cccagctggg aggctctgtt ctcaggctct gagggtccgc tgaagcccgg ggcacgcatc 300
ttctcctttg acggcaagga cgtcctgagg caccccacct ggccccagaa gagcgtgtgg 360
catggctcgg accccaacgg gcgcaggctg accgagagct actgtgagac gtggcggacg 420
gaggctccct cggccacggg ccaggcctcc tcgctgctgg ggggcaggct cctggggcag 480
agtgccgcga gctgccatca cgcctacatc gtgctctgca ttgagaacag cttcatgact 540
gcctccaagt ag 552

<210> 51
<211> 2732
<212> DNA
<213> human

<400> 51
aacaacatcc tgggattggg acccactttc tgggcactgc tggccagtcc caaaatggaa 60
cataaggaag tggttcttct acttctttta tttctgaaat caggtcaagg agagcctctg 120
gatgactatg tgaataccca gggggcttca ctgttcagtg tcactaagaa gcagctggga 180
gcaggaagta tagaagaatg tgcagcaaaa tgtgaggagg acgaagaatt cacctgcagg 240
gcattccaat atcacagtaa agagcaacaa tgtgtgataa tggctgaaaa caggaagtcc 300
tccataatca ttaggatgag agatgtagtt ttatttgaaa agaaagtgta tctctcagag 360
tgcaagactg ggaatggaaa gaattacaga gggacgatgt ccaaaacaaa aaatggcatc 420
acctgtcaaa aatggagttc cacttctccc cacagaccta gattctcacc tgctacacac 480
ccctcagagg gactggagga gaactactgc aggaatccag acaacgatcc gcaggggccc 540
tggtgctata ctactgatcc agaaaagaga tatgactact gcgacattct tgagtgtgaa 600
gaggaatgta tgcattgcag tggagaaaac tatgacggca aaatttccaa gaccatgtct 660

ggactggaat gccaggcctg ggactctcag agcccacacg ctcatggata cattccttcc 720
aaatttccaa acaagaacct gaagaagaat tactgtcgta accccgatag ggagctgcgg 780
ccttggtgtt tcaccaccga ccccaacaag cgctgggaac tttgtgacat cccccgctgc 840
acaacacctc caccatcttc tggtcccacc taccagtgtc tgaagggaac aggtgaaaac 900
tatcgcggga atgtggctgt taccgtgtcc gggcacacct gtcagcactg gagtgcacag 960
acccctcaca cacataacag gacaccagaa aacttcccct gcaaaaattt ggatgaaaac 1020
tactgccgca atcctgacgg aaaaagggcc ccatggtgcc atacaaccaa cagccaagtg 1080
cggtgggagt actgtaagat accgtcctgt gactcctccc cagtatccac ggaacagttg 1140
gctcccacag caccacctga gctaacccct gtggtccagg actgctacca tggtgatgga 1200
cagagctacc gaggcacatc ctccaccacc accacaggaa agaagtgtca gtcttggtca 1260
tctatgacac cacaccggca ccagaagacc ccagaaaact acccaaatgc tggcctgaca 1320
atgaactact gcaggaatcc agatgccgat aaaggcccct ggtgttttac cacagacccc 1380
agcgtcaggt gggagtactg caacctgaaa aaatgctcag gaacagaagc gagtgttgta 1440
gcacctccgc ctgttgtcct gcttccagat gtagagactc cttccgaaga agactgtatg 1500
tttgggaatg ggaaaggata ccgaggcaag agggcgacca ctgttactgg gacgccatgc 1560
caggactggg ctgcccagga gccccataga cacagcattt tcactccaga gacaaatcca 1620
cgggcgggtc tggaaaaaaa ttactgccgt aaccctgatg gtgatgtagg tggtccctgg 1680
tgctacacga caaatccaag aaaactttac gactactgtg atgtccctca gtgtgcggcc 1740
ccttcatttg attgtgggaa gcctcaagtg gagccgaaga aatgtcctgg aagggttgtg 1800
ggggggtgtg tggcccaccc acattcctgg ccctggcaag tcagtcttag aacaaggttt 1860
ggaatgcact tctgtggagg caccttgata tccccagagt gggtgttgac tgctgcccac 1920
tgcttggaga agtccccaag gccttcatcc tacaaggtca tcctgggtgc acaccaagaa 1980
gtgaatctcg aaccgcatgt tcaggaaata gaagtgtcta ggctgttctt ggagcccaca 2040
cgaaaagata ttgccttgct aaagctaagc agtcctgccg tcatcactga caaagtaatc 2100
ccagcttgtc tgccatcccc aaattatgtg gtcgctgacc ggaccgaatg tttcatcact 2160
ggctggggag aaacccaagg tacttttgga gctggccttc tcaaggaagc ccagctccct 2220
gtgattgaga ataaagtgtg caatcgctat gagtttctga atggaagagt ccaatccacc 2280
gaactctgtg ctgggcattt ggccggaggc actgacagtt gccagggtga cagtggaggt 2340
cctctggttt gcttcgagaa ggacaaatac attttacaag gagtcacttc ttggggtctt 2400
ggctgtgcac gccccaataa gcctggtgtc tatgttcgtg tttcaaggtt tgttacttgg 2460
attgagggag tgatgagaaa taattaattg gacgggagac agagtgacgc actgactcac 2520
ctagaggctg ggacgtgggt agggatttag catgctggaa ataactggca gtaatcaaac 2580
gaagacactg tccccagcta ccagctacgc caaacctcgg cattttttgt gttattttct 2640
gactgctgga ttctgtagta aggtgacata gctatgacat ttgttaaaaa taaactctgt 2700
acttaacttt gatttgagta aattttggtt tt 2732

<210> 52
<211> 384
<212> DNA
<213> human

<400> 52
ggccctacgg gcaccggtga atccaagtgt cctctgatgg tcaaagttct agatgctgtc 60
cgaggcagtc ctgccatcaa tgtggccgtg catgtgttca gaaaggctgc tgatgacacc 120
tgggagccat ttgcctctgg gaaaaccagt gagtctggag agctgcatgg gctcacaact 180
gaggaggaat ttgtagaagg gatatacaaa gtggaaatag acaccaaatc ttactggaag 240
gcacttggca tctccccatt ccatgagcat gcagaggtgg tattcacagc caacgactcc 300
ggcccccgcc gctacaccat tgccgccctg ctgagcccct actcctattc caccacggct 360
gtcgtcacca atcccaagga atga 384

<210> 53
<211> 165
<212> PRT
<213> human

<400> 53

<210> 54
<211> 165
<212> PRT
<213> human

<400> 54

<210> 55
<211> 274
<212> PRT
<213> human

<400> 55
Met Ala Cys Asp Leu Pro Gln Thr His Ser Leu Gly Ser Arg Arg Thr
1               5                   10                  15
Leu Met Leu Leu Ala Gln Met Arg Arg Ile Ser Leu Phe Ser Cys Leu
            20                  25                  30
Lys Asp Arg His Asp Phe Gly Phe Pro Gln Glu Glu Phe Gly Asn Gln
        35                  40                  45
Phe Gln Lys Ala Glu Thr Ile Pro Val Leu His Glu Met Ile Gln Gln
    50                  55                  60
Ile Phe Asn Leu Phe Thr Thr Lys Asp Ser Ser Ala Ala Trp Asp Glu
65                  70                  75                  80
Asp Leu Leu Asp Lys Phe Cys Thr Glu Leu Tyr Gln Gln Leu Asn Asp
                85                  90                  95
Leu Glu Ala Cys Val Met Gln Glu Glu Arg Val Gly Glu Thr Pro Leu
            100                 105                 110
Met Asn Val Asp Ser Ile Leu Ala Val Lys Lys Tyr Phe Arg Arg Ile
        115                 120                 125
Thr Leu Tyr Leu Thr Glu Lys Lys Tyr Ser Pro Cys Ala Trp Glu Val
    130                 135                 140
Val Arg Ala Glu Ile Met Arg Ser Leu Ser Leu Ser Thr Asn Leu Gln
145                 150                 155                 160
Glu Arg Leu Arg Arg Lys Glu Tyr Val Glu Gly Gly Gly Gly Ser Pro
                165                 170                 175
Gly Gly Gly Ser Gly Gly Gly Ser Asn Met Ala Val Tyr Leu Ser Glu
            180                 185                 190
Cys Lys Thr Gly Asn Gly Lys Asn Tyr Arg Gly Thr Met Ser Lys Thr
        195                 200                 205
Lys Asn Gly Ile Thr Cys Gln Lys Trp Ser Ser Thr Ser Pro His Arg
    210                 215                 220
Pro Arg Phe Ser Pro Ala Thr His Pro Ser Glu Gly Leu Glu Glu Asn
225                 230                 235                 240
Tyr Cys Arg Asn Pro Asp Asn Asp Pro Gln Gly Pro Trp Cys Tyr Thr
                245                 250                 255
Thr Asp Pro Glu Lys Arg Tyr Asp Tyr Cys Asp Ile Leu Glu Cys Glu
            260                 265                 270
Glu Glu

<210> 56
<211> 453
<212> PRT
<213> human

<400> 56
Met Ala Cys Asp Leu Pro Gln Thr His Ser Leu Gly Ser Arg Arg Thr
1               5                   10                  15
Leu Met Leu Leu Ala Gln Met Arg Arg Ile Ser Leu Phe Ser Cys Leu
            20                  25                  30
Lys Asp Arg His Asp Phe Gly Phe Pro Gln Glu Glu Phe Gly Asn Gln

35                  40                  45
Phe Gln Lys Ala Glu Thr Ile Pro Val Leu His Glu Met Ile Gln Gln
    50                  55                  60
Ile Phe Asn Leu Phe Thr Thr Lys Asp Ser Ser Ala Ala Trp Asp Glu
65                  70                  75                  80
Asp Leu Leu Asp Lys Phe Cys Thr Glu Leu Tyr Gln Gln Leu Asn Asp
                85                  90                  95
Leu Glu Ala Cys Val Met Gln Glu Glu Arg Val Gly Glu Thr Pro Leu
            100                 105                 110
Met Asn Val Asp Ser Ile Leu Ala Val Lys Lys Tyr Phe Arg Arg Ile
        115                 120                 125
Thr Leu Tyr Leu Thr Glu Lys Lys Tyr Ser Pro Cys Ala Trp Glu Val
    130                 135                 140
Val Arg Ala Glu Ile Met Arg Ser Leu Ser Leu Ser Thr Asn Leu Gln
145                 150                 155                 160
Glu Arg Leu Arg Arg Lys Glu Tyr Val Glu Gly Gly Gly Gly Ser Pro
                165                 170                 175
Gly Gly Gly Ser Gly Gly Gly Ser Asn Met Ala Val Tyr Leu Ser Glu
            180                 185                 190
Cys Lys Thr Gly Asn Gly Lys Asn Tyr Arg Gly Thr Met Ser Lys Thr
        195                 200                 205
Lys Asn Gly Ile Thr Cys Gln Lys Trp Ser Ser Thr Ser Pro His Arg
    210                 215                 220
Pro Arg Phe Ser Pro Ala Thr His Pro Ser Glu Gly Leu Glu Glu Asn
225                 230                 235                 240
Tyr Cys Arg Asn Pro Asp Asn Asp Pro Gln Gly Pro Trp Cys Tyr Thr
                245                 250                 255
Thr Asp Pro Glu Lys Arg Tyr Asp Tyr Cys Asp Ile Leu Glu Cys Glu
            260                 265                 270
Glu Glu Cys Met His Cys Ser Gly Glu Asn Tyr Asp Gly Lys Ile Ser
        275                 280                 285
Lys Thr Met Ser Gly Leu Glu Cys Gln Ala Trp Asp Ser Gln Ser Pro
    290                 295                 300
His Ala His Gly Tyr Ile Pro Ser Lys Phe Pro Asn Lys Asn Leu Lys
305                 310                 315                 320
Lys Asn Tyr Cys Arg Asn Pro Asp Arg Glu Leu Arg Pro Trp Cys Phe
                325                 330                 335
Thr Thr Asp Pro Asn Lys Arg Trp Glu Leu Cys Asp Ile Pro Arg Cys
            340                 345                 350
Thr Thr Pro Pro Pro Ser Ser Gly Pro Thr Tyr Gln Cys Leu Lys Gly
        355                 360                 365
Thr Gly Glu Asn Tyr Arg Gly Asn Val Ala Val Thr Val Ser Gly His
    370                 375                 380
Thr Cys Gln His Trp Ser Ala Gln Thr Pro His Thr His Asn Arg Thr
385                 390                 395                 400
Pro Glu Asn Phe Pro Cys Lys Asn Leu Asp Glu Asn Tyr Cys Arg Asn
                405                 410                 415
Pro Asp Gly Lys Arg Ala Pro Trp Cys His Thr Thr Asn Ser Gln Val
            420                 425                 430
Arg Trp Glu Tyr Cys Lys Ile Pro Ser Cys Asp Ser Ser Pro Val Ser
        435                 440                 445
Thr Glu Gln Leu Ala
    450

<210> 57
<211> 274
<212> PRT
<213> human

<400> 57
Met Ala Cys Asp Leu Pro Gln Thr His Ser Leu Gly Ser Arg Arg Thr
1               5                   10                  15
Leu Met Leu Leu Ala Gln Met Arg Arg Ile Ser Leu Phe Ser Cys Leu
            20                  25                  30
Lys Asp Arg His Asp Phe Gly Phe Pro Gln Glu Glu Phe Gly Asn Gln
        35                  40                  45
Phe Gln Lys Ala Glu Thr Ile Pro Val Leu His Glu Met Ile Gln Gln
    50                  55                  60
Ile Phe Asn Leu Phe Ser Thr Lys Asp Ser Ser Ala Ala Trp Asp Glu
65                  70                  75                  80
Thr Leu Leu Asp Lys Phe Tyr Thr Glu Leu Tyr Gln Gln Leu Asn Asp
                85                  90                  95
Leu Glu Ala Cys Val Ile Gln Gly Val Gly Val Thr Glu Thr Pro Leu
            100                 105                 110
Met Lys Glu Asp Ser Ile Leu Ala Val Arg Lys Tyr Phe Gln Arg Ile
        115                 120                 125
Thr Leu Tyr Leu Lys Glu Lys Lys Tyr Ser Pro Cys Ala Trp Glu Val
    130                 135                 140
Val Arg Ala Glu Ile Met Arg Ser Phe Ser Leu Ser Thr Asn Leu Gln
145                 150                 155                 160
Glu Ser Leu Arg Ser Lys Glu Tyr Val Glu Gly Gly Gly Gly Ser Pro
                165                 170                 175
Gly Gly Gly Ser Gly Gly Gly Ser Asn Met Ala Val Tyr Leu Ser Glu
            180                 185                 190
Cys Lys Thr Gly Asn Gly Lys Asn Tyr Arg Gly Thr Met Ser Lys Thr
        195                 200                 205
Lys Asn Gly Ile Thr Cys Gln Lys Trp Ser Ser Thr Ser Pro His Arg
    210                 215                 220
Pro Arg Phe Ser Pro Ala Thr His Pro Ser Glu Gly Leu Glu Glu Asn
225                 230                 235                 240
Tyr Cys Arg Asn Pro Asp Asn Asp Pro Gln Gly Pro Trp Cys Tyr Thr
                245                 250                 255
Thr Asp Pro Glu Lys Arg Tyr Asp Tyr Cys Asp Ile Leu Glu Cys Glu
            260                 265                 270
Glu Glu

<210> 58
<211> 453
<212> PRT
<213> human

<400> 58
Met Ala Cys Asp Leu Pro Gln Thr His Ser Leu Gly Ser Arg Arg Thr
1               5                   10                  15
Leu Met Leu Leu Ala Gln Met Arg Arg Ile Ser Leu Phe Ser Cys Leu
            20                  25                  30
Lys Asp Arg His Asp Phe Gly Phe Pro Gln Glu Glu Phe Gly Asn Gln

35                  40                  45
Phe Gln Lys Ala Glu Thr Ile Pro Val Leu His Glu Met Ile Gln Gln
    50                  55                  60
Ile Phe Asn Leu Phe Ser Thr Lys Asp Ser Ser Ala Ala Trp Asp Glu
65                  70                  75                  80
Thr Leu Leu Asp Lys Phe Tyr Thr Glu Leu Tyr Gln Gln Leu Asn Asp
                85                  90                  95
Leu Glu Ala Cys Val Ile Gln Gly Val Gly Val Thr Glu Thr Pro Leu
            100                 105                 110
Met Lys Glu Asp Ser Ile Leu Ala Val Arg Lys Tyr Phe Gln Arg Ile
        115                 120                 125
Thr Leu Tyr Leu Lys Glu Lys Lys Tyr Ser Pro Cys Ala Trp Glu Val
    130                 135                 140
Val Arg Ala Glu Ile Met Arg Ser Phe Ser Leu Ser Thr Asn Leu Gln
145                 150                 155                 160
Glu Ser Leu Arg Ser Lys Glu Tyr Val Glu Gly Gly Gly Gly Ser Pro
                165                 170                 175
Gly Gly Gly Ser Gly Gly Gly Ser Asn Met Ala Val Tyr Leu Ser Glu
            180                 185                 190
Cys Lys Thr Gly Asn Gly Lys Asn Tyr Arg Gly Thr Met Ser Lys Thr
        195                 200                 205
Lys Asn Gly Ile Thr Cys Gln Lys Trp Ser Ser Thr Ser Pro His Arg
    210                 215                 220
Pro Arg Phe Ser Pro Ala Thr His Pro Ser Glu Gly Leu Glu Glu Asn
225                 230                 235                 240
Tyr Cys Arg Asn Pro Asp Asn Asp Pro Gln Gly Pro Trp Cys Tyr Thr
                245                 250                 255
Thr Asp Pro Glu Lys Arg Tyr Asp Tyr Cys Asp Ile Leu Glu Cys Glu
            260                 265                 270
Glu Glu Cys Met His Cys Ser Gly Glu Asn Tyr Asp Gly Lys Ile Ser
        275                 280                 285
Lys Thr Met Ser Gly Leu Glu Cys Gln Ala Trp Asp Ser Gln Ser Pro
    290                 295                 300
His Ala His Gly Tyr Ile Pro Ser Lys Phe Pro Asn Lys Asn Leu Lys
305                 310                 315                 320
Lys Asn Tyr Cys Arg Asn Pro Asp Arg Glu Leu Arg Pro Trp Cys Phe
                325                 330                 335
Thr Thr Asp Pro Asn Lys Arg Trp Glu Leu Cys Asp Ile Pro Arg Cys
            340                 345                 350
Thr Thr Pro Pro Pro Ser Ser Gly Pro Thr Tyr Gln Cys Leu Lys Gly
        355                 360                 365
Thr Gly Glu Asn Tyr Arg Gly Asn Val Ala Val Thr Val Ser Gly His
    370                 375                 380
Thr Cys Gln His Trp Ser Ala Gln Thr Pro His Thr His Asn Arg Thr
385                 390                 395                 400
Pro Glu Asn Phe Pro Cys Lys Asn Leu Asp Glu Asn Tyr Cys Arg Asn
                405                 410                 415
Pro Asp Gly Lys Arg Ala Pro Trp Cys His Thr Thr Asn Ser Gln Val
            420                 425                 430
Arg Trp Glu Tyr Cys Lys Ile Pro Ser Cys Asp Ser Ser Pro Val Ser
        435                 440                 445
Thr Glu Gln Leu Ala
    450

<210> 59
<211> 453
<212> PRT
<213> human

<400> 59
Met Ala Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Cys Met His Cys Ser Gly Glu
                85                  90                  95
Asn Tyr Asp Gly Lys Ile Ser Lys Thr Met Ser Gly Leu Glu Cys Gln
            100                 105                 110
Ala Trp Asp Ser Gln Ser Pro His Ala His Gly Tyr Ile Pro Ser Lys
        115                 120                 125
Phe Pro Asn Lys Asn Leu Lys Lys Asn Tyr Cys Arg Asn Pro Asp Arg
    130                 135                 140
Glu Leu Arg Pro Trp Cys Phe Thr Thr Asp Pro Asn Lys Arg Trp Glu
145                 150                 155                 160
Leu Cys Asp Ile Pro Arg Cys Thr Thr Pro Pro Pro Ser Ser Gly Pro
                165                 170                 175
Thr Tyr Gln Cys Leu Lys Gly Thr Gly Glu Asn Tyr Arg Gly Asn Val
            180                 185                 190
Ala Val Thr Val Ser Gly His Thr Cys Gln His Trp Ser Ala Gln Thr
        195                 200                 205
Pro His Thr His Asn Arg Thr Pro Glu Asn Phe Pro Cys Lys Asn Leu
    210                 215                 220
Asp Glu Asn Tyr Cys Arg Asn Pro Asp Gly Lys Arg Ala Pro Trp Cys
225                 230                 235                 240
His Thr Thr Asn Ser Gln Val Arg Trp Glu Tyr Cys Lys Ile Pro Ser
                245                 250                 255
Cys Asp Ser Ser Pro Val Ser Thr Glu Gln Leu Ala Tyr Val Glu Gly
            260                 265                 270
Gly Gly Gly Ser Pro Gly Gly Gly Ser Gly Gly Gly Ser Asn Met Ala
        275                 280                 285
Cys Asp Leu Pro Gln Thr His Ser Leu Gly Ser Arg Arg Thr Leu Met
    290                 295                 300
Leu Leu Ala Gln Met Arg Arg Ile Ser Leu Phe Ser Cys Leu Lys Asp
305                 310                 315                 320
Arg His Asp Phe Gly Phe Pro Gln Glu Glu Phe Gly Asn Gln Phe Gln
                325                 330                 335
Lys Ala Glu Thr Ile Pro Val Leu His Glu Met Ile Gln Gln Ile Phe
            340                 345                 350
Asn Leu Phe Ser Thr Lys Asp Ser Ser Ala Ala Trp Asp Glu Thr Leu
        355                 360                 365
Leu Asp Lys Phe Tyr Thr Glu Leu Tyr Gln Gln Leu Asn Asp Leu Glu
    370                 375                 380

Ala Cys Val Ile Gln Gly Val Gly Val Thr Glu Thr Pro Leu Met Lys
385                 390                 395                 400
Glu Asp Ser Ile Leu Ala Val Arg Lys Tyr Phe Gln Arg Ile Thr Leu
                405                 410                 415
Tyr Leu Lys Glu Lys Lys Tyr Ser Pro Cys Ala Trp Glu Val Val Arg
            420                 425                 430
Ala Glu Ile Met Arg Ser Phe Ser Leu Ser Thr Asn Leu Gln Glu Ser
        435                 440                 445
Leu Arg Ser Lys Glu
    450

<210> 60
<211> 274
<212> PRT
<213> human

<400> 60
Met Ala Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Tyr Val Glu Gly Gly Gly Gly
                85                  90                  95
Ser Pro Gly Gly Gly Ser Gly Gly Gly Ser Asn Met Ala Cys Asp Leu
            100                 105                 110
Pro Gln Thr His Ser Leu Gly Ser Arg Arg Thr Leu Met Leu Leu Ala
        115                 120                 125
Gln Met Arg Arg Ile Ser Leu Phe Ser Cys Leu Lys Asp Arg His Asp
    130                 135                 140
Phe Gly Phe Pro Gln Glu Glu Phe Gly Asn Gln Phe Gln Lys Ala Glu
145                 150                 155                 160
Thr Ile Pro Val Leu His Glu Met Ile Gln Gln Ile Phe Asn Leu Phe
                165                 170                 175
Thr Thr Lys Asp Ser Ser Ala Ala Trp Asp Glu Asp Leu Leu Asp Lys
            180                 185                 190
Phe Cys Thr Glu Leu Tyr Gln Gln Leu Asn Asp Leu Glu Ala Cys Val
        195                 200                 205
Met Gln Glu Glu Arg Val Gly Glu Thr Pro Leu Met Asn Val Asp Ser
    210                 215                 220
Ile Leu Ala Val Lys Lys Tyr Phe Arg Arg Ile Thr Leu Tyr Leu Thr
225                 230                 235                 240
Glu Lys Lys Tyr Ser Pro Cys Ala Trp Glu Val Val Arg Ala Glu Ile
                245                 250                 255
Met Arg Ser Leu Ser Leu Ser Thr Asn Leu Gln Glu Arg Leu Arg Arg
            260                 265                 270
Lys Glu

<210> 61
<211> 274
<212> PRT
<213> human

<400> 61
Met Ala Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Tyr Val Glu Gly Gly Gly Gly
                85                  90                  95
Ser Pro Gly Gly Gly Ser Gly Gly Gly Ser Asn Met Ala Cys Asp Leu
            100                 105                 110
Pro Gln Thr His Ser Leu Gly Ser Arg Arg Thr Leu Met Leu Leu Ala
        115                 120                 125
Gln Met Arg Arg Ile Ser Leu Phe Ser Cys Leu Lys Asp Arg His Asp
    130                 135                 140
Phe Gly Phe Pro Gln Glu Glu Phe Gly Asn Gln Phe Gln Lys Ala Glu
145                 150                 155                 160
Thr Ile Pro Val Leu His Glu Met Ile Gln Gln Ile Phe Asn Leu Phe
                165                 170                 175
Ser Thr Lys Asp Ser Ser Ala Ala Trp Asp Glu Thr Leu Leu Asp Lys
            180                 185                 190
Phe Tyr Thr Glu Leu Tyr Gln Gln Leu Asn Asp Leu Glu Ala Cys Val
        195                 200                 205
Ile Gln Gly Val Gly Val Thr Glu Thr Pro Leu Met Lys Glu Asp Ser
    210                 215                 220
Ile Leu Ala Val Arg Lys Tyr Phe Gln Arg Ile Thr Leu Tyr Leu Lys
225                 230                 235                 240
Glu Lys Lys Tyr Ser Pro Cys Ala Trp Glu Val Val Arg Ala Glu Ile
                245                 250                 255
Met Arg Ser Phe Ser Leu Ser Thr Asn Leu Gln Glu Ser Leu Arg Ser
            260                 265                 270
Lys Glu

<210> 62
<211> 453
<212> PRT
<213> human

<400> 62
Met Ala Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro

35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Cys Met His Cys Ser Gly Glu
                85                  90                  95
Asn Tyr Asp Gly Lys Ile Ser Lys Thr Met Ser Gly Leu Glu Cys Gln
            100                 105                 110
Ala Trp Asp Ser Gln Ser Pro His Ala His Gly Tyr Ile Pro Ser Lys
        115                 120                 125
Phe Pro Asn Lys Asn Leu Lys Lys Asn Tyr Cys Arg Asn Pro Asp Arg
    130                 135                 140
Glu Leu Arg Pro Trp Cys Phe Thr Thr Asp Pro Asn Lys Arg Trp Glu
145                 150                 155                 160
Leu Cys Asp Ile Pro Arg Cys Thr Thr Pro Pro Pro Ser Ser Gly Pro
                165                 170                 175
Thr Tyr Gln Cys Leu Lys Gly Thr Gly Glu Asn Tyr Arg Gly Asn Val
            180                 185                 190
Ala Val Thr Val Ser Gly His Thr Cys Gln His Trp Ser Ala Gln Thr
        195                 200                 205
Pro His Thr His Asn Arg Thr Pro Glu Asn Phe Pro Cys Lys Asn Leu
    210                 215                 220
Asp Glu Asn Tyr Cys Arg Asn Pro Asp Gly Lys Arg Ala Pro Trp Cys
225                 230                 235                 240
His Thr Thr Asn Ser Gln Val Arg Trp Glu Tyr Cys Lys Ile Pro Ser
                245                 250                 255
Cys Asp Ser Ser Pro Val Ser Thr Glu Gln Leu Ala Tyr Val Glu Gly
            260                 265                 270
Gly Gly Gly Ser Pro Gly Gly Gly Ser Gly Gly Gly Ser Asn Met Ala
        275                 280                 285
Cys Asp Leu Pro Gln Thr His Ser Leu Gly Ser Arg Arg Thr Leu Met
    290                 295                 300
Leu Leu Ala Gln Met Arg Arg Ile Ser Leu Phe Ser Cys Leu Lys Asp
305                 310                 315                 320
Arg His Asp Phe Gly Phe Pro Gln Glu Glu Phe Gly Asn Gln Phe Gln
                325                 330                 335
Lys Ala Glu Thr Ile Pro Val Leu His Glu Met Ile Gln Gln Ile Phe
            340                 345                 350
Asn Leu Phe Thr Thr Lys Asp Ser Ser Ala Ala Trp Asp Glu Asp Leu
        355                 360                 365
Leu Asp Lys Phe Cys Thr Glu Leu Tyr Gln Gln Leu Asn Asp Leu Glu
    370                 375                 380
Ala Cys Val Met Gln Glu Glu Arg Val Gly Glu Thr Pro Leu Met Asn
385                 390                 395                 400
Val Asp Ser Ile Leu Ala Val Lys Lys Tyr Phe Arg Arg Ile Thr Leu
                405                 410                 415
Tyr Leu Thr Glu Lys Lys Tyr Ser Pro Cys Ala Trp Glu Val Val Arg
            420                 425                 430
Ala Glu Ile Met Arg Ser Leu Ser Leu Ser Thr Asn Leu Gln Glu Arg
        435                 440                 445
Leu Arg Arg Lys Glu
    450

<210> 63
<211> 566
<212> PRT
<213> human

<400> 63
Met Glu His Lys Glu Val Val Leu Leu Leu Leu Leu Phe Leu Lys Ser
1               5                   10                  15
Gly Gln Gly Glu Pro Leu Asp Asp Tyr Val Asn Thr Gln Gly Ala Ser
            20                  25                  30
Leu Phe Ser Val Thr Lys Lys Gln Leu Gly Ala Gly Ser Ile Glu Glu
        35                  40                  45
Cys Ala Ala Lys Cys Glu Glu Asp Glu Glu Phe Thr Cys Arg Ala Phe
    50                  55                  60
Gln Tyr His Ser Lys Glu Gln Gln Cys Val Ile Met Ala Glu Asn Arg
65                  70                  75                  80
Lys Ser Ser Ile Ile Ile Arg Met Arg Asp Val Val Leu Phe Glu Lys
                85                  90                  95
Lys Val Tyr Leu Ser Glu Cys Lys Thr Gly Asn Gly Lys Asn Tyr Arg
            100                 105                 110
Gly Thr Met Ser Lys Thr Lys Asn Gly Ile Thr Cys Gln Lys Trp Ser
        115                 120                 125
Ser Thr Ser Pro His Arg Pro Arg Phe Ser Pro Ala Thr His Pro Ser
    130                 135                 140
Glu Gly Leu Glu Glu Asn Tyr Cys Arg Asn Pro Asp Asn Asp Pro Gln
145                 150                 155                 160
Gly Pro Trp Cys Tyr Thr Thr Asp Pro Glu Lys Arg Tyr Asp Tyr Cys
                165                 170                 175
Asp Ile Leu Glu Cys Glu Glu Glu Cys Met His Cys Ser Gly Glu Asn
            180                 185                 190
Tyr Asp Gly Lys Ile Ser Lys Thr Met Ser Gly Leu Glu Cys Gln Ala
        195                 200                 205
Trp Asp Ser Gln Ser Pro His Ala His Gly Tyr Ile Pro Ser Lys Phe
    210                 215                 220
Pro Asn Lys Asn Leu Lys Lys Asn Tyr Cys Arg Asn Pro Asp Arg Glu
225                 230                 235                 240
Leu Arg Pro Trp Cys Phe Thr Thr Asp Pro Asn Lys Arg Trp Glu Leu
                245                 250                 255
Cys Asp Ile Pro Arg Cys Thr Thr Pro Pro Pro Ser Ser Gly Pro Thr
            260                 265                 270
Tyr Gln Cys Leu Lys Gly Thr Gly Glu Asn Tyr Arg Gly Asn Val Ala
        275                 280                 285
Val Thr Val Ser Gly His Thr Cys Gln His Trp Ser Ala Gln Thr Pro
    290                 295                 300
His Thr His Asn Arg Thr Pro Glu Asn Phe Pro Cys Lys Asn Leu Asp
305                 310                 315                 320
Glu Asn Tyr Cys Arg Asn Pro Asp Gly Lys Arg Ala Pro Trp Cys His
                325                 330                 335
Thr Thr Asn Ser Gln Val Arg Trp Glu Tyr Cys Lys Ile Pro Ser Cys
            340                 345                 350
Asp Ser Ser Pro Val Ser Thr Glu Gln Leu Ala Pro Thr Ala Pro Pro
        355                 360                 365
Glu Leu Thr Pro Val Val Gln Asp Cys Tyr His Gly Asp Gly Gln Ser
    370                 375                 380

Tyr Arg Gly Thr Ser Ser Thr Thr Thr Thr Gly Lys Lys Cys Gln Ser
385                 390                 395                 400
Trp Ser Ser Met Thr Pro His Arg His Gln Lys Thr Pro Glu Asn Tyr
                405                 410                 415
Pro Asn Ala Gly Leu Thr Met Asn Tyr Cys Arg Asn Pro Asp Ala Asp
            420                 425                 430
Lys Gly Pro Trp Cys Phe Thr Thr Asp Pro Ser Val Arg Trp Glu Tyr
        435                 440                 445
Cys Asn Leu Lys Lys Cys Ser Gly Thr Glu Ala Ser Val Val Ala Pro
    450                 455                 460
Pro Pro Val Val Leu Leu Pro Asp Val Glu Thr Pro Ser Glu Glu Asp
465                 470                 475                 480
Cys Met Phe Gly Asn Gly Lys Gly Tyr Arg Gly Lys Arg Ala Thr Thr
                485                 490                 495
Val Thr Gly Thr Pro Cys Gln Asp Trp Ala Ala Gln Glu Pro His Arg
            500                 505                 510
His Ser Ile Phe Thr Pro Glu Thr Asn Pro Arg Ala Gly Leu Glu Lys
        515                 520                 525
Asn Tyr Cys Arg Asn Pro Asp Gly Asp Val Gly Gly Pro Trp Cys Tyr
    530                 535                 540
Thr Thr Asn Pro Arg Lys Leu Tyr Asp Tyr Cys Asp Val Pro Gln Cys
545                 550                 555                 560
Ala Ala Pro Ser Phe Asp
                565

<210> 64
<211> 381
<212> PRT
<213> human

<400> 64
Met Ser Arg Leu Pro Val Leu Leu Leu Leu Gln Leu Leu Val Arg Pro
1               5                   10                  15
Ala Met Ser Val Tyr Leu Ser Glu Cys Lys Thr Gly Asn Gly Lys Asn
            20                  25                  30
Tyr Arg Gly Thr Met Ser Lys Thr Lys Asn Gly Ile Thr Cys Gln Lys
        35                  40                  45
Trp Ser Ser Thr Ser Pro His Arg Pro Arg Phe Ser Pro Ala Thr His
    50                  55                  60
Pro Ser Glu Gly Leu Glu Glu Asn Tyr Cys Arg Asn Pro Asp Asn Asp
65                  70                  75                  80
Pro Gln Gly Pro Trp Cys Tyr Thr Thr Asp Pro Glu Lys Arg Tyr Asp
                85                  90                  95
Tyr Cys Asp Ile Leu Glu Cys Glu Glu Glu Cys Met His Cys Ser Gly
            100                 105                 110
Glu Asn Tyr Asp Gly Lys Ile Ser Lys Thr Met Ser Gly Leu Glu Cys
        115                 120                 125
Gln Ala Trp Asp Ser Gln Ser Pro His Ala His Gly Tyr Ile Pro Ser
    130                 135                 140
Lys Phe Pro Asn Lys Asn Leu Lys Lys Asn Tyr Cys Arg Asn Pro Asp
145                 150                 155                 160
Arg Glu Leu Arg Pro Trp Cys Phe Thr Thr Asp Pro Asn Lys Arg Trp
                165                 170                 175
Glu Leu Cys Asp Ile Pro Arg Cys Thr Thr Pro Pro Pro Ser Ser Gly

            180                 185                 190
Pro Thr Tyr Gln Cys Leu Lys Gly Thr Gly Glu Asn Tyr Arg Gly Asn
        195                 200                 205
Val Ala Val Thr Val Ser Gly His Thr Cys Gln His Trp Ser Ala Gln
    210                 215                 220
Thr Pro His Thr His Asn Arg Thr Pro Glu Asn Phe Pro Cys Lys Asn
225                 230                 235                 240
Leu Asp Glu Asn Tyr Cys Arg Asn Pro Asp Gly Lys Arg Ala Pro Trp
                245                 250                 255
Cys His Thr Thr Asn Ser Gln Val Arg Trp Glu Tyr Cys Lys Ile Pro
            260                 265                 270
Ser Cys Asp Ser Ser Pro Val Ser Thr Glu Gln Leu Ala Pro Thr Ala
        275                 280                 285
Pro Pro Glu Leu Thr Pro Val Val Gln Asp Cys Tyr His Gly Asp Gly
    290                 295                 300
Gln Ser Tyr Arg Gly Thr Ser Ser Thr Thr Thr Thr Gly Lys Lys Cys
305                 310                 315                 320
Gln Ser Trp Ser Ser Met Thr Pro His Arg His Gln Lys Thr Pro Glu
                325                 330                 335
Asn Tyr Pro Asn Ala Gly Leu Thr Met Asn Tyr Cys Arg Asn Pro Asp
            340                 345                 350
Ala Asp Lys Gly Pro Trp Cys Phe Thr Thr Asp Pro Ser Val Arg Trp
        355                 360                 365
Glu Tyr Cys Asn Leu Lys Lys Cys Ser Gly Thr Glu Ala
    370                 375                 380

<210> 65
<211> 285
<212> PRT
<213> human

<400> 65
Met Ser Arg Leu Pro Val Leu Leu Leu Leu Gln Leu Leu Val Arg Pro
1               5                   10                  15
Ala Met Ser Val Tyr Leu Ser Glu Cys Lys Thr Gly Asn Gly Lys Asn
            20                  25                  30
Tyr Arg Gly Thr Met Ser Lys Thr Lys Asn Gly Ile Thr Cys Gln Lys
        35                  40                  45
Trp Ser Ser Thr Ser Pro His Arg Pro Arg Phe Ser Pro Ala Thr His
    50                  55                  60
Pro Ser Glu Gly Leu Glu Glu Asn Tyr Cys Arg Asn Pro Asp Asn Asp
65                  70                  75                  80
Pro Gln Gly Pro Trp Cys Tyr Thr Thr Asp Pro Glu Lys Arg Tyr Asp
                85                  90                  95
Tyr Cys Asp Ile Leu Glu Cys Glu Glu Glu Cys Met His Cys Ser Gly
            100                 105                 110
Glu Asn Tyr Asp Gly Lys Ile Ser Lys Thr Met Ser Gly Leu Glu Cys
        115                 120                 125
Gln Ala Trp Asp Ser Gln Ser Pro His Ala His Gly Tyr Ile Pro Ser
    130                 135                 140
Lys Phe Pro Asn Lys Asn Leu Lys Lys Asn Tyr Cys Arg Asn Pro Asp
145                 150                 155                 160
Arg Glu Leu Arg Pro Trp Cys Phe Thr Thr Asp Pro Asn Lys Arg Trp
                165                 170                 175

Glu Leu Cys Asp Ile Pro Arg Cys Thr Thr Pro Pro Pro Ser Ser Gly
            180                 185                 190
Pro Thr Tyr Gln Cys Leu Lys Gly Thr Gly Glu Asn Tyr Arg Gly Asn
        195                 200                 205
Val Ala Val Thr Val Ser Gly His Thr Cys Gln His Trp Ser Ala Gln
    210                 215                 220
Thr Pro His Thr His Asn Arg Thr Pro Glu Asn Phe Pro Cys Lys Asn
225                 230                 235                 240
Leu Asp Glu Asn Tyr Cys Arg Asn Pro Asp Gly Lys Arg Ala Pro Trp
                245                 250                 255
Cys His Thr Thr Asn Ser Gln Val Arg Trp Glu Tyr Cys Lys Ile Pro
            260                 265                 270
Ser Cys Asp Ser Ser Pro Val Ser Thr Glu Gln Leu Ala
        275                 280                 285

<210> 66
<211> 297
<212> PRT
<213> human

<400> 66
Met Ser Arg Leu Pro Val Leu Leu Leu Leu Gln Leu Leu Val Arg Pro
1               5                   10                  15
Ala Met Ser Glu Glu Glu Cys Met His Cys Ser Gly Glu Asn Tyr Asp
            20                  25                  30
Gly Lys Ile Ser Lys Thr Met Ser Gly Leu Glu Cys Gln Ala Trp Asp
        35                  40                  45
Ser Gln Ser Pro His Ala His Gly Tyr Ile Pro Ser Lys Phe Pro Asn
    50                  55                  60
Lys Asn Leu Lys Lys Asn Tyr Cys Arg Asn Pro Asp Arg Glu Leu Arg
65                  70                  75                  80
Pro Trp Cys Phe Thr Thr Asp Pro Asn Lys Arg Trp Glu Leu Cys Asp
                85                  90                  95
Ile Pro Arg Cys Thr Thr Pro Pro Pro Ser Ser Gly Pro Thr Tyr Gln
            100                 105                 110
Cys Leu Lys Gly Thr Gly Glu Asn Tyr Arg Gly Asn Val Ala Val Thr
        115                 120                 125
Val Ser Gly His Thr Cys Gln His Trp Ser Ala Gln Thr Pro His Thr
    130                 135                 140
His Asn Arg Thr Pro Glu Asn Phe Pro Cys Lys Asn Leu Asp Glu Asn
145                 150                 155                 160
Tyr Cys Arg Asn Pro Asp Gly Lys Arg Ala Pro Trp Cys His Thr Thr
                165                 170                 175
Asn Ser Gln Val Arg Trp Glu Tyr Cys Lys Ile Pro Ser Cys Asp Ser
            180                 185                 190
Ser Pro Val Ser Thr Glu Gln Leu Ala Pro Thr Ala Pro Pro Glu Leu
        195                 200                 205
Thr Pro Val Val Gln Asp Cys Tyr His Gly Asp Gly Gln Ser Tyr Arg
    210                 215                 220
Gly Thr Ser Ser Thr Thr Thr Thr Gly Lys Lys Cys Gln Ser Trp Ser
225                 230                 235                 240
Ser Met Thr Pro His Arg His Gln Lys Thr Pro Glu Asn Tyr Pro Asn
                245                 250                 255
Ala Gly Leu Thr Met Asn Tyr Cys Arg Asn Pro Asp Ala Asp Lys Gly

            260                 265                 270
Pro Trp Cys Phe Thr Thr Asp Pro Ser Val Arg Trp Glu Tyr Cys Asn
        275                 280                 285
Leu Lys Lys Cys Ser Gly Thr Glu Ala
    290                 295

<210> 67
<211> 201
<212> PRT
<213> human

<400> 67
Met Ser Arg Leu Pro Val Leu Leu Leu Leu Gln Leu Leu Val Arg Pro
1               5                   10                  15
Ala Met Ser Glu Glu Glu Cys Met His Cys Ser Gly Glu Asn Tyr Asp
            20                  25                  30
Gly Lys Ile Ser Lys Thr Met Ser Gly Leu Glu Cys Gln Ala Trp Asp
        35                  40                  45
Ser Gln Ser Pro His Ala His Gly Tyr Ile Pro Ser Lys Phe Pro Asn
    50                  55                  60
Lys Asn Leu Lys Lys Asn Tyr Cys Arg Asn Pro Asp Arg Glu Leu Arg
65                  70                  75                  80
Pro Trp Cys Phe Thr Thr Asp Pro Asn Lys Arg Trp Glu Leu Cys Asp
                85                  90                  95
Ile Pro Arg Cys Thr Thr Pro Pro Pro Ser Ser Gly Pro Thr Tyr Gln
            100                 105                 110
Cys Leu Lys Gly Thr Gly Glu Asn Tyr Arg Gly Asn Val Ala Val Thr
        115                 120                 125
Val Ser Gly His Thr Cys Gln His Trp Ser Ala Gln Thr Pro His Thr
    130                 135                 140
His Asn Arg Thr Pro Glu Asn Phe Pro Cys Lys Asn Leu Asp Glu Asn
145                 150                 155                 160
Tyr Cys Arg Asn Pro Asp Gly Lys Arg Ala Pro Trp Cys His Thr Thr
                165                 170                 175
Asn Ser Gln Val Arg Trp Glu Tyr Cys Lys Ile Pro Ser Cys Asp Ser
            180                 185                 190
Ser Pro Val Ser Thr Glu Gln Leu Ala
        195                 200

<210> 68
<211> 380
<212> PRT
<213> human

<400> 68
Met Ser Leu Phe Glu Lys Lys Val Tyr Leu Ser Glu Cys Lys Thr Gly
1               5                   10                  15
Asn Gly Lys Asn Tyr Arg Gly Thr Met Ser Lys Thr Lys Asn Gly Ile
            20                  25                  30
Thr Cys Gln Lys Trp Ser Ser Thr Ser Pro His Arg Pro Arg Phe Ser
        35                  40                  45
Pro Ala Thr His Pro Ser Glu Gly Leu Glu Glu Asn Tyr Cys Arg Asn
    50                  55                  60
Pro Asp Asn Asp Pro Gln Gly Pro Trp Cys Tyr Thr Thr Asp Pro Glu

65                  70                  75                  80
Lys Arg Tyr Asp Tyr Cys Asp Ile Leu Glu Cys Glu Glu Glu Cys Met
                85                  90                  95
His Cys Ser Gly Glu Asn Tyr Asp Gly Lys Ile Ser Lys Thr Met Ser
            100                 105                 110
Gly Leu Glu Cys Gln Ala Trp Asp Ser Gln Ser Pro His Ala His Gly
        115                 120                 125
Tyr Ile Pro Ser Lys Phe Pro Asn Lys Asn Leu Lys Lys Asn Tyr Cys
    130                 135                 140
Arg Asn Pro Asp Arg Glu Leu Arg Pro Trp Cys Phe Thr Thr Asp Pro
145                 150                 155                 160
Asn Lys Arg Trp Glu Leu Cys Asp Ile Pro Arg Cys Thr Thr Pro Pro
                165                 170                 175
Pro Ser Ser Gly Pro Thr Tyr Gln Cys Leu Lys Gly Thr Gly Glu Asn
            180                 185                 190
Tyr Arg Gly Asn Val Ala Val Thr Val Ser Gly His Thr Cys Gln His
        195                 200                 205
Trp Ser Ala Gln Thr Pro His Thr His Asn Arg Thr Pro Glu Asn Phe
    210                 215                 220
Pro Cys Lys Asn Leu Asp Glu Asn Tyr Cys Arg Asn Pro Asp Gly Lys
225                 230                 235                 240
Arg Ala Pro Trp Cys His Thr Thr Asn Ser Gln Val Arg Trp Glu Tyr
                245                 250                 255
Cys Lys Ile Pro Ser Cys Asp Ser Ser Pro Val Ser Thr Glu Gln Leu
            260                 265                 270
Ala Pro Thr Ala Pro Pro Glu Leu Thr Pro Val Val Gln Asp Cys Tyr
        275                 280                 285
His Gly Asp Gly Gln Ser Tyr Arg Gly Thr Ser Ser Thr Thr Thr Thr
    290                 295                 300
Gly Lys Lys Cys Gln Ser Trp Ser Ser Met Thr Pro His Arg His Gln
305                 310                 315                 320
Lys Thr Pro Glu Asn Tyr Pro Asn Ala Gly Leu Thr Met Asn Tyr Cys
                325                 330                 335
Arg Asn Pro Asp Ala Asp Lys Gly Pro Trp Cys Phe Thr Thr Asp Pro
            340                 345                 350
Ser Val Arg Trp Glu Tyr Cys Asn Leu Lys Lys Cys Ser Gly Thr Glu
        355                 360                 365
Ala Ser Val Val Ala Pro Pro Pro Val Val Leu Leu
    370                 375                 380

<210> 69
<211> 364
<212> PRT
<213> human

<400> 69
Met Ser Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60

Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Cys Met His Cys Ser Gly Glu
                85                  90                  95
Asn Tyr Asp Gly Lys Ile Ser Lys Thr Met Ser Gly Leu Glu Cys Gln
            100                 105                 110
Ala Trp Asp Ser Gln Ser Pro His Ala His Gly Tyr Ile Pro Ser Lys
        115                 120                 125
Phe Pro Asn Lys Asn Leu Lys Lys Asn Tyr Cys Arg Asn Pro Asp Arg
    130                 135                 140
Glu Leu Arg Pro Trp Cys Phe Thr Thr Asp Pro Asn Lys Arg Trp Glu
145                 .150                155                 160
Leu Cys Asp Ile Pro Arg Cys Thr Thr Pro Pro Pro Ser Ser Gly Pro
                165                 170                 175
Thr Tyr Gln Cys Leu Lys Gly Thr Gly Glu Asn Tyr Arg Gly Asn Val
            180                 185                 190
Ala Val Thr Val Ser Gly His Thr Cys Gln His Trp Ser Ala Gln Thr
        195                 200                 205
Pro His Thr His Asn Arg Thr Pro Glu Asn Phe Pro Cys Lys Asn Leu
    210                 215                 220
Asp Glu Asn Tyr Cys Arg Asn Pro Asp Gly Lys Arg Ala Pro Trp Cys
225                 230                 235                 240
His Thr Thr Asn Ser Gln Val Arg Trp Glu Tyr Cys Lys Ile Pro Ser
                245                 250                 255
Cys Asp Ser Ser Pro Val Ser Thr Glu Gln Leu Ala Pro Thr Ala Pro
            260                 265                 270
Pro Glu Leu Thr Pro Val Val Gln Asp Cys Tyr His Gly Asp Gly Gln
        275                 280                 285
Ser Tyr Arg Gly Thr Ser Ser Thr Thr Thr Thr Gly Lys Lys Cys Gln
    290                 295                 300
Ser Trp Ser Ser Met Thr Pro His Arg His Gln Lys Thr Pro Glu Asn
305                 310                 315                 320
Tyr Pro Asn Ala Gly Leu Thr Met Asn Tyr Cys Arg Asn Pro Asp Ala
                325                 330                 335
Asp Lys Gly Pro Trp Cys Phe Thr Thr Asp Pro Ser Val Arg Trp Glu
            340                 345                 350
Tyr Cys Asn Leu Lys Lys Cys Ser Gly Thr Glu Ala
        355                 360

<210> 70
<211> 184
<212> PRT
<213> human

<400> 70
Met Ser Glu Glu Glu Cys Met His Cys Ser Gly Glu Asn Tyr Asp Gly
1               5                   10                  15
Lys Ile Ser Lys Thr Met Ser Gly Leu Glu Cys Gln Ala Trp Asp Ser
            20                  25                  30
Gln Ser Pro His Ala His Gly Tyr Ile Pro Ser Lys Phe Pro Asn Lys
        35                  40                  45
Asn Leu Lys Lys Asn Tyr Cys Arg Asn Pro Asp Arg Glu Leu Arg Pro
    50                  55                  60
Trp Cys Phe Thr Thr Asp Pro Asn Lys Arg Trp Glu Leu Cys Asp Ile

65                  70                  75                  80
Pro Arg Cys Thr Thr Pro Pro Pro Ser Ser Gly Pro Thr Tyr Gln Cys
                85                  90                  95
Leu Lys Gly Thr Gly Glu Asn Tyr Arg Gly Asn Val Ala Val Thr Val
            100                 105                 110
Ser Gly His Thr Cys Gln His Trp Ser Ala Gln Thr Pro His Thr His
        115                 120                 125
Asn Arg Thr Pro Glu Asn Phe Pro Cys Lys Asn Leu Asp Glu Asn Tyr
    130                 135                 140
Cys Arg Asn Pro Asp Gly Lys Arg Ala Pro Trp Cys His Thr Thr Asn
145                 150                 155                 160
Ser Gln Val Arg Trp Glu Tyr Cys Lys Ile Pro Ser Cys Asp Ser Ser
                165                 170                 175
Pro Val Ser Thr Glu Gln Leu Ala
            180

<210> 71
<211> 280
<212> PRT
<213> human

<400> 71
Met Ser Glu Glu Glu Cys Met His Cys Ser Gly Glu Asn Tyr Asp Gly
1               5                   10                  15
Lys Ile Ser Lys Thr Met Ser Gly Leu Glu Cys Gln Ala Trp Asp Ser
            20                  25                  30
Gln Ser Pro His Ala His Gly Tyr Ile Pro Ser Lys Phe Pro Asn Lys
        35                  40                  45
Asn Leu Lys Lys Asn Tyr Cys Arg Asn Pro Asp Arg Glu Leu Arg Pro
    50                  55                  60
Trp Cys Phe Thr Thr Asp Pro Asn Lys Arg Trp Glu Leu Cys Asp Ile
65                  70                  75                  80
Pro Arg Cys Thr Thr Pro Pro Pro Ser Ser Gly Pro Thr Tyr Gln Cys
                85                  90                  95
Leu Lys Gly Thr Gly Glu Asn Tyr Arg Gly Asn Val Ala Val Thr Val
            100                 105                 110
Ser Gly His Thr Cys Gln His Trp Ser Ala Gln Thr Pro His Thr His
        115                 120                 125
Asn Arg Thr Pro Glu Asn Phe Pro Cys Lys Asn Leu Asp Glu Asn Tyr
    130                 135                 140
Cys Arg Asn Pro Asp Gly Lys Arg Ala Pro Trp Cys His Thr Thr Asn
145                 150                 155                 160
Ser Gln Val Arg Trp Glu Tyr Cys Lys Ile Pro Ser Cys Asp Ser Ser
                165                 170                 175
Pro Val Ser Thr Glu Gln Leu Ala Pro Thr Ala Pro Pro Glu Leu Thr
            180                 185                 190
Pro Val Val Gln Asp Cys Tyr His Gly Asp Gly Gln Ser Tyr Arg Gly
        195                 200                 205
Thr Ser Ser Thr Thr Thr Thr Gly Lys Lys Cys Gln Ser Trp Ser Ser
    210                 215                 220
Met Thr Pro His Arg His Gln Lys Thr Pro Glu Asn Tyr Pro Asn Ala
225                 230                 235                 240
Gly Leu Thr Met Asn Tyr Cys Arg Asn Pro Asp Ala Asp Lys Gly Pro
                245                 250                 255

Trp Cys Phe Thr Thr Asp Pro Ser Val Arg Trp Glu Tyr Cys Asn Leu
            260                 265                 270
Lys Lys Cys Ser Gly Thr Glu Ala
        275                 280

<210> 72
<211> 268
<212> PRT
<213> human

<400> 72
Met Ser Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu Cys Met His Cys Ser Gly Glu
                85                  90                  95
Asn Tyr Asp Gly Lys Ile Ser Lys Thr Met Ser Gly Leu Glu Cys Gln
            100                 105                 110
Ala Trp Asp Ser Gln Ser Pro His Ala His Gly Tyr Ile Pro Ser Lys
        115                 120                 125
Phe Pro Asn Lys Asn Leu Lys Lys Asn Tyr Cys Arg Asn Pro Asp Arg
    130                 135                 140
Glu Leu Arg Pro Trp Cys Phe Thr Thr Asp Pro Asn Lys Arg Trp Glu
145                 150                 155                 160
Leu Cys Asp Ile Pro Arg Cys Thr Thr Pro Pro Pro Ser Ser Gly Pro
                165                 170                 175
Thr Tyr Gln Cys Leu Lys Gly Thr Gly Glu Asn Tyr Arg Gly Asn Val
            180                 185                 190
Ala Val Thr Val Ser Gly His Thr Cys Gln His Trp Ser Ala Gln Thr
        195                 200                 205
Pro His Thr His Asn Arg Thr Pro Glu Asn Phe Pro Cys Lys Asn Leu
    210                 215                 220
Asp Glu Asn Tyr Cys Arg Asn Pro Asp Gly Lys Arg Ala Pro Trp Cys
225                 230                 235                 240
His Thr Thr Asn Ser Gln Val Arg Trp Glu Tyr Cys Lys Ile Pro Ser
                245                 250                 255
Cys Asp Ser Ser Pro Val Ser Thr Glu Gln Leu Ala
            260                 265

<210> 73
<211> 89
<212> PRT
<213> human

<400> 73
Met Ser Val Tyr Leu Ser Glu Cys Lys Thr Gly Asn Gly Lys Asn Tyr
1               5                   10                  15

Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu
                85

<210> 74
<211> 106
<212> PRT
<213> human

<400> 74
Met Ser Arg Leu Pro Val Leu Leu Leu Leu Gln Leu Leu Val Arg Pro
1               5                   10                  15
Ala Met Ala Val Tyr Leu Ser Glu Cys Lys Thr Gly Asn Gly Lys Asn
            20                  25                  30
Tyr Arg Gly Thr Met Ser Lys Thr Lys Asn Gly Ile Thr Cys Gln Lys
        35                  40                  45
Trp Ser Ser Thr Ser Pro His Arg Pro Arg Phe Ser Pro Ala Thr His
    50                  55                  60
Pro Ser Glu Gly Leu Glu Glu Asn Tyr Cys Arg Asn Pro Asp Asn Asp
65                  70                  75                  80
Pro Gln Gly Pro Trp Cys Tyr Thr Thr Asp Pro Glu Lys Arg Tyr Asp
                85                  90                  95
Tyr Cys Asp Ile Leu Glu Cys Glu Glu Glu
            100                 105

<210> 75
<211> 470
<212> PRT
<213> human

<400> 75
Met Ser Arg Leu Pro Val Leu Leu Leu Leu Gln Leu Leu Val Arg Pro
1               5                   10                  15
Ala Met Ala Val Tyr Leu Ser Glu Cys Lys Thr Gly Asn Gly Lys Asn
            20                  25                  30
Tyr Arg Gly Thr Met Ser Lys Thr Lys Asn Gly Ile Thr Cys Gln Lys
        35                  40                  45
Trp Ser Ser Thr Ser Pro His Arg Pro Arg Phe Ser Pro Ala Thr His
    50                  55                  60
Pro Ser Glu Gly Leu Glu Glu Asn Tyr Cys Arg Asn Pro Asp Asn Asp
65                  70                  75                  80
Pro Gln Gly Pro Trp Cys Tyr Thr Thr Asp Pro Glu Lys Arg Tyr Asp
                85                  90                  95
Tyr Cys Asp Ile Leu Glu Cys Glu Glu Glu Cys Met His Cys Ser Gly
            100                 105                 110
Glu Asn Tyr Asp Gly Lys Ile Ser Lys Thr Met Ser Gly Leu Glu Cys
        115                 120                 125

Gln Ala Trp Asp Ser Gln Ser Pro His Ala His Gly Tyr Ile Pro Ser
    130                 135                 140
Lys Phe Pro Asn Lys Asn Leu Lys Lys Asn Tyr Cys Arg Asn Pro Asp
145                 150                 155                 160
Arg Glu Leu Arg Pro Trp Cys Phe Thr Thr Asp Pro Asn Lys Arg Trp
                165                 170                 175
Glu Leu Cys Asp Ile Pro Arg Cys Thr Thr Pro Pro Pro Ser Ser Gly
            180                 185                 190
Pro Thr Tyr Gln Cys Leu Lys Gly Thr Gly Glu Asn Tyr Arg Gly Asn
        195                 200                 205
Val Ala Val Thr Val Ser Gly His Thr Cys Gln His Trp Ser Ala Gln
    210                 215                 220
Thr Pro His Thr His Asn Arg Thr Pro Glu Asn Phe Pro Cys Lys Asn
225                 230                 235                 240
Leu Asp Glu Asn Tyr Cys Arg Asn Pro Asp Gly Lys Arg Ala Pro Trp
                245                 250                 255
Cys His Thr Thr Asn Ser Gln Val Arg Trp Glu Tyr Cys Lys Ile Pro
            260                 265                 270
Ser Cys Asp Ser Ser Pro Val Ser Thr Glu Gln Leu Ala Tyr Val Glu
        275                 280                 285
Gly Gly Gly Gly Ser Pro Gly Gly Gly Ser Gly Gly Gly Ser Asn Met
    290                 295                 300
Ala Cys Asp Leu Pro Gln Thr His Ser Leu Gly Ser Arg Arg Thr Leu
305                 310                 315                 320
Met Leu Leu Ala Gln Met Arg Arg Ile Ser Leu Phe Ser Cys Leu Lys
                325                 330                 335
Asp Arg His Asp Phe Gly Phe Pro Gln Glu Glu Phe Gly Asn Gln Phe
            340                 345                 350
Gln Lys Ala Glu Thr Ile Pro Val Leu His Glu Met Ile Gln Gln Ile
        355                 360                 365
Phe Asn Leu Phe Thr Thr Lys Asp Ser Ser Ala Ala Trp Asp Glu Asp
    370                 375                 380
Leu Leu Asp Lys Phe Cys Thr Glu Leu Tyr Gln Gln Leu Asn Asp Leu
385                 390                 395                 400
Glu Ala Cys Val Met Gln Glu Glu Arg Val Gly Glu Thr Pro Leu Met
                405                 410                 415
Asn Val Asp Ser Ile Leu Ala Val Lys Lys Tyr Phe Arg Arg Ile Thr
            420                 425                 430
Leu Tyr Leu Thr Glu Lys Lys Tyr Ser Pro Cys Ala Trp Glu Val Val
        435                 440                 445
Arg Ala Glu Ile Met Arg Ser Leu Ser Leu Ser Thr Asn Leu Gln Glu
    450                 455                 460
Arg Leu Arg Arg Lys Glu
465                 470

<210> 76
<211> 183
<212> PRT
<213> human

<400> 76
His Ser His Arg Asp Phe Gln Pro Val Leu His Leu Val Ala Leu Asn
1               5                   10                  15
Ser Pro Leu Ser Gly Gly Met Arg Gly Ile Arg Gly Ala Asp Phe Gln

20                  25                  30
Cys Phe Gln Gln Ala Arg Ala Val Gly Leu Ala Gly Thr Phe Arg Ala
        35                  40                  45
Phe Leu Ser Ser Arg Leu Gln Asp Leu Tyr Ser Ile Val Arg Arg Ala
    50                  55                  60
Asp Arg Ala Ala Val Pro Ile Val Asn Leu Lys Asp Glu Leu Leu Phe
65                  70                  75                  80
Pro Ser Trp Glu Ala Leu Phe Ser Gly Ser Glu Gly Pro Leu Lys Pro
                85                  90                  95
Gly Ala Arg Ile Phe Ser Phe Asp Gly Lys Asp Val Leu Arg His Pro
            100                 105                 110
Thr Trp Pro Gln Lys Ser Val Trp His Gly Ser Asp Pro Asn Gly Arg
        115                 120                 125
Arg Leu Thr Glu Ser Tyr Cys Glu Thr Trp Arg Thr Glu Ala Pro Ser
    130                 135                 140
Ala Thr Gly Gln Ala Ser Ser Leu Leu Gly Gly Arg Leu Leu Gly Gln
145                 150                 155                 160
Ser Ala Ala Ser Cys His His Ala Tyr Ile Val Leu Cys Ile Glu Asn
                165                 170                 175
Ser Phe Met Thr Ala Ser Lys
            180

<210> 77
<211> 810
<212> PRT
<213> human

<400> 77

<210> 78
<211> 127
<212> PRT
<213> human

<400> 78
Gly Pro Thr Gly Thr Gly Glu Ser Lys Cys Pro Leu Met Val Lys Val
1               5                   10                  15
Leu Asp Ala Val Arg Gly Ser Pro Ala Ile Asn Val Ala Val His Val
            20                  25                  30
Phe Arg Lys Ala Ala Asp Asp Thr Trp Glu Pro Phe Ala Ser Gly Lys
        35                  40                  45
Thr Ser Glu Ser Gly Glu Leu His Gly Leu Thr Thr Glu Glu Glu Phe
    50                  55                  60
Val Glu Gly Ile Tyr Lys Val Glu Ile Asp Thr Lys Ser Tyr Trp Lys
65                  70                  75                  80
Ala Leu Gly Ile Ser Pro Phe His Glu His Ala Glu Val Val Phe Thr
                85                  90                  95
Ala Asn Asp Ser Gly Pro Arg Arg Tyr Thr Ile Ala Ala Leu Leu Ser
            100                 105                 110
Pro Tyr Ser Tyr Ser Thr Thr Ala Val Val Thr Asn Pro Lys Glu
        115                 120                 125

<210> 79
<211> 18
<212> PRT
<213> human

<400> 79
Tyr Val Glu Gly Gly Gly Gly Ser Pro Gly Gly Gly Ser Gly Gly Gly
1               5                   10                  15

Ser Asn

<210> 80
<211> 4
<212> PRT
<213> human

<400> 80
Gly Gly Gly Ser
1

<210> 81
<211> 8
<212> PRT
<213> human

<400> 81
Gly Gly Gly Ser Gly Gly Gly Ser
1               5

<210> 82
<211> 12
<212> PRT
<213> human

<400> 82
Gly Gly Gly Ser Gly Gly Gly Ser Gly Gly Gly Ser
1               5                   10

<210> 83
<211> 7
<212> PRT
<213> human

<400> 83
Ser Gly Gly Ser Gly Gly Ser
1               5

<210> 84
<211> 6
<212> PRT
<213> human

<400> 84
Glu Phe Gly Asn Met Ala
1               5

<210> 85
<211> 7
<212> PRT
<213> human

<400> 85

Glu Phe Gly Gly Asn Met Ala
1               5

<210> 86
<211> 10
<212> PRT
<213> human

<400> 86
Glu Phe Gly Gly Asn Gly Gly Asn Met Ala
1               5                   10

<210> 87
<211> 7
<212> PRT
<213> human

<400> 87
Gly Gly Ser Asp Met Ala Gly
1               5

<210> 88
<211> 4
<212> PRT
<213> human

<400> 88
Gly Gly Gly Ser
1

<210> 89
<211> 5
<212> PRT
<213> human

<400> 89
Gly Gly Gly Gly Ser
1               5

<210> 90
<211> 6
<212> PRT
<213> human

<400> 90
Gly Gly Gly Gly Gly Ser
1               5

<210> 91
<211> 3
<212> PRT
<213> human

<400> 91

Ala Gly Ser
1

<210> 92
<211> 7
<212> PRT
<213> human

<400> 92
Gly Gly Gly Gly Gly Gly Ser
1               5

<210> 93
<211> 36
<212> PRT
<213> human

<400> 93
Gly Gly Gly Ser Gly Gly Gly Ser Gly Gly Gly Ser Glu Gly Gly Gly
1               5                   10                  15
Ser Glu Gly Gly Gly Ser Glu Gly Gly Gly Ser Glu Gly Gly Gly Ser
            20                  25                  30
Gly Gly Gly Ser
        35

<210> 94
<211> 24
<212> PRT
<213> human

<400> 94
Ile Ser Glu Pro Ser Gly Pro Ile Ser Thr Ile Asn Pro Ser Pro Pro
1               5                   10                  15
Ser Lys Glu Ser His Lys Ser Pro
            20

<210> 95
<211> 28
<212> PRT
<213> human

<400> 95
Ile Glu Gly Arg Ile Ser Glu Pro Ser Gly Pro Ile Ser Thr Ile Asn
1               5                   10                  15
Pro Ser Pro Pro Ser Lys Glu Ser His Lys Ser Pro
            20                  25

<210> 96
<211> 28
<212> DNA
<213> human

<400> 96
catgccatgg cagtgtatct ctcagtgc                                    28

<210> 97
<211> 30
<212> DNA
<213> human

<400> 97
catgccatgg cagtttatct ttcagagtgt 30

<210> 98
<211> 34
<212> DNA
<213> human

<400> 98
gatcaagctt ttattaagcc aattgttccg tgga 34

<210> 99
<211> 34
<212> DNA
<213> human

<400> 99
gatcaagctt ttattacgct tctgttcctg agca 34

<210> 100
<211> 34
<212> DNA
<213> human

<400> 100
gatcaagctt ttattaaagc aggacaacag gcgg 34

<210> 101
<211> 48
<212> DNA
<213> human

<400> 101
gatcacatgt chgargarga rtgyatgcay tgcagtggag aaaactat 48

<210> 102
<211> 48
<212> DNA
<213> human

<400> 102
gatcacatgt chgtbtayct btchgartgy aagactggga atggaaag 48

<210> 103
<211> 48
<212> DNA
<213> human

<400> 103
gatcacatgt chctbttyga raaraargtb tatctctcag agtgcaag 48

<210> 104
<211> 34
<212> DNA
<213> human

<400> 104
gatcggatcc gccaccatgg aacataagga agtg 34

<210> 105
<211> 31
<212> DNA
<213> human

<400> 105
gatcggatcc ttaatcaaat gaaggggccg c 31

<210> 106
<211> 25
<212> DNA
<213> human

<400> 106
gatcccatgg cagtgtatct gtccg 25

<210> 107
<211> 40
<212> DNA
<213> human

<400> 107
gatcaagctt tccagactcg agcgcttctg ttcctgagca 40

<210> 108
<211> 27
<212> DNA
<213> human

<400> 108
tgtccatcac cgtggtagca gtcctgg 27

<210> 109
<211> 33
<212> DNA
<213> human

<400> 109
tgccatacaa cgaattccca agtgcggtgg gag 33

<210> 110
<211> 30
<212> DNA

<213> numan

<400> 110
catgccatgg cagtttatct ttcagagtgt                                        30

<210> 111
<211> 34
<212> DNA
<213> human

<400> 111
gatcaagctt ttattattcc tcttcacact caag                                   34

<210> 112
<211> 281
<212> DNA
<213> human

<400> 112
ccatggcagt ttatctttca gagtgtaaga ctgggaatgg aaagaattac agagggacga      60
tgtccaaaac aaaaaatggc atcacctgtc aaaaatggag ttccacttct ccccacagac     120
ctagattctc acctgctaca cacccctcag agggactgga ggagaactac tgcaggaatc     180
cagacaacga tccgcagggg ccctggtgct atactactga tccagaaaag agatatgact     240
actgcgacat tcttgagtgt gaagaggaat aataaaagct t                         281

<210> 113
<211> 89
<212> PRT
<213> human

<400> 113
Met Ala Val Tyr Leu Ser Glu Cys Lys Thr Gly Asn Gly Lys Asn Tyr
1               5                   10                  15
Arg Gly Thr Met Ser Lys Thr Lys Asn Gly Ile Thr Cys Gln Lys Trp
            20                  25                  30
Ser Ser Thr Ser Pro His Arg Pro Arg Phe Ser Pro Ala Thr His Pro
        35                  40                  45
Ser Glu Gly Leu Glu Glu Asn Tyr Cys Arg Asn Pro Asp Asn Asp Pro
    50                  55                  60
Gln Gly Pro Trp Cys Tyr Thr Thr Asp Pro Glu Lys Arg Tyr Asp Tyr
65                  70                  75                  80
Cys Asp Ile Leu Glu Cys Glu Glu Glu
                85

## Description

### Priority

The present application claims priority under Title 35, United States Code, § 119 of United States Provisional application Serial No. 60/060,609 filed October 2, 1997.

### Field of the Invention

The present invention relates to the development of novel multi-functional proteins (MFPs) of the formula: R₁-L₁-R₂, R₂-L₁-R₁, R₁-L₁-R₁, R₁-R₂, R₂-R₁, or R₁-R₁ where R₁ is angiostatin, R₂ is selected from endostatin, human type I interferon, thrombospondin, interferon-inducible protein 10, platelet factor 4, and L₁ is a linker capable of linking R₁ to R₂. The invention is also drawn to nucleic acids that encode MFPs, procedures for manufacturing MFPs, methods of using MFPs, methods of treating angiogenic-mediated disease with MFPs including methods of treating tumors with MFPs, and treating tumors by gene therapy using vectors that encode MFPs.

### Background of the Invention

Angiogenesis, the growth of new blood vessels, plays an important role in cancer growth and metastasis. In humans, the extent of vasculature in a tumor has been shown to correlate with the patient prognosis for a variety of cancers (Folkman, J., Seminars in Medicine of the Beth Israel Hospital, Boston 333(26): 1757-1763, 1995; Gasparini, G., European Journal of Cancer 32A(14): 2485-2493, 1996; Pluda, J. M., Seminars in Oncology 24(2): 203-218, 1997). In normal adults, angiogenesis is limited to well controlled situations, such as wound healing and the female reproductive system (Battegay, E.J., J Mol Med 73:-333-346, 1995; Dvorak, H.F, New Engl J Med, 315: 1650-1659, 1986).

Animal studies suggest that tumors can exist in a dormant state, in which tumor growth is limited by a balance between high rates of proliferation and high rates of apoptosis (Holmgren, L. et al., Nat. Med. (N. Y.) 1(2): 149-153, 1995; Hanahan, D. et al., Cell (Cambridge, Mass.) 86(3): 353-364, 1996). The switch to an angiogenic phenotype allows tumor cells to escape from dormancy and to grow rapidly, presumably as the result of a decrease in the apoptotic rate of the tumor cells (Bouck, Cancer Cells, 2(6): 179-185, 1990; Dameron et al, Cold Spring Harb Symp Quant Biol, 59: 483-489, 1994). The control of angiogenesis is thought to be a balance between factors which promote new vessel formation and anti-angiogenic factors with suppress the formation of a neovasculature (Bouck, N. et al., Advances in Cancer Research 69: 135-173, 1996; O'Reilly et al., Cell (Cambridge, Mass) 79(2): 315-328, 1994).

A variety of pro-angiogenic factors have been characterized including basic and acid fibroblast growth factors (bFGF and aFGF) and vascular permeability factor/vascular endothelial growth factor (VPF/VEGF) (Potgens, A. J. G. et al., Biol. Chem. Hoppe-Seyler 376: 57-70, 1995; Ferrara, N., European Journal of Cancer 32A(14): 2413-2442, 1996; Bikfalvi, A. et al., Endocrine Reviews 18: 26-45, 1997). Several endogenous anti-angiogenic factors have also been characterized, including angiostatin (O'Reilly et al., Cell (Cambridge, Mass) 79(2): 315-328, 1994), endostatin (O'Reilly et al, Cell (Cambridge, Mass) 88(2): 277-285, 1997), interferon alpha (Ezekowitz et al, N. Engl. J. Med., May 28, 326(22) 1456-1463, 1992), thrombospondin (Good et al, Proc Natl Acad Sci USA 87(17): 6624-6628, 1990; Tolsma et al, J Cell Biol 122(2): 497-511, 1993), and platelet factor 4 (PF4) (Maione et al, Science 247:(4938): 77-79, 1990).

Angiostatin is a 38 kD protein comprising the first three or four kringle domains of plasminogen and was first described in 1994 (O'Reilly, M. S. et al., Cell (Cambridge, Mass.) 79(2): 315-328, 1994). Mice bearing primary (Lewis lung carcinoma-low metastatic) tumors did not respond to angiogenic stimuli such as bFGF in a corneal micropocket assay and the growth of metastatic tumors in these mice was suppressed until the primary tumor was excised. The factor responsible for the inhibition of angiogenesis and tumor growth was designated mouse angiostatin. Angiostatin was also shown to inhibit the growth of endothelial cells in vitro.

Human angiostatin can be prepared by digestion of plasminogen by porcine elastase (O'Reilly et al., Cell 79(2): 315-328, 1994) or with human metalloelastase (Dong et al., Cell 88, 801-810, 1997). The angiostatin produced via porcine elastase digestion inhibited the growth of metastases and primary tumors in mice. O'Reilly et al (Cell 79(2): 315-328, 1994) demonstrated that human angiostatin inhibited metastasis of Lewis lung carcinoma in SCID mice. The same group (O'Reilly, M. S. et al., Nat. Med. (N. Y.) 2(6): 689-692, 1996) subsequently showed that human angiostatin inhibited the growth of the human tumors PC3 prostate carcinoma, clone A colon carcinoma, and MDA-MB breast carcinoma in SCID mice. Human angiostatin also inhibited the growth of the mouse tumors Lewis lung carcinoma, T241 fibrosarcoma and M5076 reticulum cell carcinoma in C57B1 mice. Because these enzymatically-prepared angiostatins are not well characterized biochemically, the precise composition of the molecules is not known.

Angiostatins of known composition can be prepared by means of recombinant DNA technology and expression in heterologous cell systems. Recombinant human angiostatin comprising Kringle domains one through four (K1-4) has been produced in the yeast *Pichia pastoris* (Sim et al., Cancer Res 57: 1329-1334, 1997). The recombinant human protein inhibited growth of endothelial cells in vitro and inhibited metastasis of Lewis lung carcinoma in C57Bl mice. Recombinant murine angiostatin (K1-4) has been produced in insect cells (Wu et al., Biochem Biophys Res Comm 236: 651-654, 1997). The recombinant mouse protein inhibited endothelial cell growth in vitro and growth of primary Lewis lung carcinoma *in vivo*. These experiments demonstrated that the first four kringle domains are sufficient for angiostatin activity but did not determine which kringle domains are necessary.

Cao et al. (J. Biol. Chem. 271: 29461-29467, 1996), produced fragments of human plasminogen by proteolysis and by expression of recombinant proteins in *E. coli*. These authors showed that kringle one and to a lesser extent kringle four of plasminogen were responsible for the inhibition of endothelial cell growth in vitro. Specifically, kringles 1-4 and 1-3 inhibited at similar concentrations, while K1 alone inhibited endothelial cell growth at four-fold higher concentrations. Kringles two and three inhibited to a lesser extent. More recently Cao et al. (*J Biol Chem* **272**: 22924-22928, 1997), showed that recombinant mouse or human kringle five inhibited endothelial cell growth at lower concentrations than angiostatin (K1-4). These experiments demonstrated in vitro angiostatin-like activity but did not address in vivo action against tumors and their metastases.

World patent applications WO 95/29242 A1, WO 96/41194 A1, and WO 96/35774 A2 describe the expression, purification, and characterization of angiostatin. WO 95/29242 A1 951102 discloses purification of a protein from blood and urine by HPLC that inhibits proliferation of endothelial cells. The protein has a molecular weight between 38 kilodaltons and 45 kilodaltons and an amino acid sequence substantially similar to that of a murine plasminogen fragment beginning at amino acid number 79 of a murine plasminogen molecule. WO 96/41194 A1 961219, discloses compounds and methods for the diagnosis and monitoring of angiogenesis-dependent diseases. WO 96/35774 A2 961114 discloses the structure of protein fragments, generally corresponding to kringle structures occurring within angiostatin. It also discloses aggregate forms of angiostatin, which have endothelial cell inhibiting activity, and provides a means for inhibiting angiogenesis of tumors and for treating angiogenic-mediated diseases.

Endostatin, a 20-kDa (184 amino acid) carboxy fragment of collagen XVIII, is an angiogenesis inhibitor produced by a hemangioendothelioma (O'Reilly, M. S. et al., Cell (Cambridge, Mass.) 88(2): 277-285, 1997); and WO 97/15666). Endostatin specifically inhibits endothelial proliferation and inhibits angiogenesis and tumor growth. Primary tumors treated with non-refolded suspensions of *E. coli*-derived endostatin regressed to dormant microscopic lesions. Toxicity was not observed and immunohistochemical studies revealed a blockage of angiogenesis accompanied by high proliferation balanced by apoptosis in tumor cells.

Interferon alpha (IFN alpha) is a family of highly homologous, species-specific proteins that possess complex antiviral, antineoplastic and immunomodulating activities (Extensively reviewed in the monograph "Antineoplastic agents, interferon alfa", American Society of Hospital Pharmacists, Inc., 1996). Interferon alpha also has anti-proliferative, and anti-angiogenic properties, and has specific effects on cellular differentiation (Sreevalsan, in "Biologic Therapy of Cancer", pp. 347-364, (eds. V.T. DeVita Jr., S. Hellman, and S.A. Rosenberg), J.B. Lippincott Co, Philadelphia, PA, 1995).

Interferon alpha is effective against a variety of cancers including hairy cell leukemia, chronic myelogenous leukemia, malignant melanoma, and Kaposi's sarcoma. The precise mechanism by which IFN alpha exerts its anti-tumor activity is not entirely clear, and may differ based on the tumor type or stage of disease. The anti-proliferative properties of IFN alpha, which may result from the modulation of the expression of oncogenes and/or proto-oncogenes, have been demonstrated on both tumor cell lines and human tumors growing in nude mice (Gutterman, J. U., Proc. Natl. Acad. Sci., USA 91: 1198-1205, 1994).

Interferon is also considered an anti-angiogenic factor, as demonstrated through the successful treatment of hemangiomas in infants (Ezekowitz et al, N. Engl. J. Med., May 28, 326(22) 1456-1463, 1992) and the effectiveness of IFN alpha against Kaposi's sarcoma (Krown, Semin Oncol 14(2 Suppl 3): 27-33, 1987). The mechanism underlying these anti-angiogenic effects is not clear, and may be the result of IFN alpha action on the tumor (decreasing the secretion of pro-angiogenic factors) or on the neo-vasculature. IFN receptors have been identified on a variety of cell types (Navarro et al., Modern Pathology 9(2): 150-156, 1996).

United States Patent 4,530,901, by Weissmann, describes the cloning and expression of IFN-alpha-type molecules in transformed host strains. United States Patent 4,503,035, Pestka, describes an improved processes for purifying 10 species of human leukocyte interferon using preparative high performance liquid chromatography. United States Patent 5,231,176, Goeddel, describes the cloning of a novel distinct family of human leukocyte interferons containing in their mature form greater than 166 and no more than 172 amino acids.

United States Patent 5,541,293, by Stabinsky, describes the synthesis, cloning, and expression of consensus human interferons. These are non-naturally occurring analogues of human (leukocyte) interferon-alpha assembled from synthetic oligonucleotides. The sequence of the consensus interferon was determined by comparing the sequences of 13 members of the IFN-alpha family of interferons and selecting the preferred amino acid at each position. These variants differ from naturally occurring forms in terms of the identity and/or location of one or more amino acids, and one or more biological and pharmacological properties (e.g., antibody reactivity, potency, or duration effect) but retain other such properties.

Thrombospondin-1 (TSP-1) is a trimer containing three copies of a 180 kDa polypeptide. TSP-1 is produced by many cell types including platelets, fibroblasts, and endothelial cells (see Frazier, Curr Opin Cell Biol 3(5): 792-799, 1991) and the cDNA encoding the subunit has been cloned (Hennessy et al., 1989, J Cell Biol 108(2): 729-736; Lawler and Hynes, J Cell Biol 103(5): 1635-1648, 1986). Native TSP-1 has been shown to block endothelial cell migration *in vitro* and neovascularization *in vivo* (Good et al, Proc Natl Acad Sci USA 87(17): 6624-6628, 1990). Expression of TSP-1 in tumor cells also suppresses tumorigenesis and tumor-induced angiogenesis (Sheibani and Frazier, Proc Natl Acad Sci USA 92(15) 6788-6792, 1995; Weinstat-Saslow et al., Cancer Res 54(24):6504-6511, 1994). The anti-angiogenic activity of TSP-1 has been shown to reside in two distinct domains of this protein (Tolsma et al, J Cell Biol 122(2): 497-511, 1993). One of these domains consists of residues 303 to 309 of native TSP-1 and the other consists of residues 481 to 499 of TSP-1. Another important domain consists of the sequence CSVTCG which appears to mediate the binding of TSP-1 to some tumor cell types (Tuszynski and Nicosia, Bioessays 18(1): 71-76, 1996). These results suggest that CSVTCG, or related sequences, can be used to target other moieties to tumor cells. Taken together, the available data indicate that TSP-1 plays a role in the growth and vascularization of tumors. Subfragments of TSP-1, then, may be useful as anti-angiogenic components of chimeras and/or in targeting other proteins to specific tumor cells. Subfragments may be generated by standard procedures (such as proteolytic fragmentation, or by DNA amplification, cloning, expression, and purification of specific TSP-1 domains or subdomains) and tested for anti-angiogenic or anti-tumor activities by methods known in the art (Tolsma et al, J Cell Biol 122(2): 497-511, 1993; Tuszynski and Nicosia, Bioessays 18(1): 71-76, 1996).

A variety of *in vivo* experiments demonstrate that chemokines platelet factor 4 (PF-4) and interferon inducible protein 10 (IP-10) retard the growth of tumors. PF-4 blocks the growth of primary tumors in mice (mouse melanoma and human colon cancer) (Sharpe et al., J. Natl. Cancer Inst. 82, 848-853; Maione et al. Cancer Res. 51, 2077-2083, 1991). IP-10 administration blocks non-small cell lung tumorigenesis and Burkitt's lymphoma subcutaneous tumors in mice (Arenberg et al., J. Exp. Med. 184, 981-992, 1996; Sgardari et al. Proc. Natl. Acad. Sci. USA 93, 13791-13796, 1996). PF-4 is currently in clinical trials for Kaposi's sarcoma, glioblastoma, melanoma, renal cell and colon cancer. IP-10 promotes the migration of activated lymphocytes to the site of injection thereby suggesting that IP-10 may have immuno-modulatory properties. The immuno-modulatory property may contribute to the anti-tumor capacity of IP-10.

Anti-angiogenic therapy may offer several advantages over conventional chemotherapy for the treatment of cancer. Anti-angiogenic agents have low toxicity in preclinical trials and development of drug resistance has not been observed (Folkman, J., Seminars in Medicine of the Beth Israel Hospital, Boston 333(26): 1757-1763, 1995). As angiogenesis is a complex process, made up of many steps including invasion, proliferation and migration of endothelial cells, it can be anticipated that combination therapies may be most effective. In fact, combinations of chemotherapy with anti-angiogenic therapy have already shown promising results in pre-clinical models (Teicher, B. A. et al., Breast Cancer Research and Treatment 36: 227-236, 1995; Teicher, B. A. et al., European Journal of Cancer 32A(14): 2461-2466, 1996).

Chimeras of angiostatin with other anti-angiogenic proteins such as endostatin may have improved biological properties by virtue of acting through several mechanisms. The two proteins may have distinct mechanisms, allowing increased efficacy by virtue of blocking the angiogenic process at two separate points. Alternatively, the two proteins may act on the same cell type causing an increased binding, or other action, by virtue of increased avidity. In this case, each molecule would also have the effect of targeting the other domain to the appropriate site of action. Dimers or higher order multimers of angiostatin, endostatin, or other anti-angiogenic proteins with themselves or other proteins could have increased efficacy and/or potency by virtue of either of these mechanisms.

One class of mechanisms for improved activity of angiostatin chimeras would result from the ability to bind to two distinct receptors on the same cell (e.g., receptor agonists) and thus demonstrate improved activity due to interactions with receptors on a single cell. These chimeras may have improved therapeutic properties through a variety of mechanisms such as: 1). alterations in the overall on- or off-rates or Kₐ or K_{d} of the ligand(s) on the target cell, 2). activation or blockade of complementary receptor signaling pathways, and/or 3). more specific targeting of one or both of the components to the cell of interest. Examples of this class are endothelial cell binding dual receptor agonists (or antagonists) such as: AᵥB₃-angiostatin, angiostatin-IP-10, and angiostatin-IFN alpha. For example, formation of a chimera between angiostatin and a non-ELR-C-X-C chemokine is expected to generate a receptor agonist which has anti-angiogenic effects on the endothelium. In addition, an IP-10-angiostatin chimera would be expected to contain an immunomodulatory component affecting the recruitment of white blood cells to the tumor. IP-10 would also be expected to have an effect on the primary tumor and on metastasis. Further, the chimeric proteins are expected to possess a unique pharmacokinetic distribution and clearance profile (Dehmer et al. Circulation, 91, 2188-2194, 1995; Tanaka et al. Nature Medicine, 3, 437-442, 1997).

A second class of mechanisms does not depend on receptor binding on the same cell, but where the chimeric protein has improved properties *in vivo* compared to the two components individually. This may be a result of alterations in biodistribution or half-life. Alternatively, the binding of the chimera to one or more of the receptors, pharmacokinetics, or uptake of the chimera is altered in a favorable manner. These proteins are likely to act through complementary mechanisms. Therefore, chimeric proteins containing anti-proliferative and anti-angiogenic activities may provide improved anti-tumor activity by using two distinct mechanisms to decrease tumor growth. The anti-proliferative moiety should act directly on the tumor to decrease its growth while the anti-angiogenic factor should act indirectly by preventing the growth of the neo-vasculature required for rapid tumor growth. One example of this mechanism might include angiostatin/IFN alpha chimeras.

### Summary of the Invention

Novel proteins of this invention are represented by the formulas:

R₁-L₁-R₂, R₂-L₁-R₁, R₁-L₁-R₁, R₁-R₂, R₂-R₁, and R₁-R₁

wherein R₁ is a modified human angiostatin amino acid sequence,
and R₂ is a modified endostatin, human type I interferon, thrombospondin or a fragment thereof, interferon-inducible protein 10, platelet factor 4, and proteins with similar activity,
and wherein L₁ is a linker capable of linking R₁ to R₂,
and said protein can optionally be immediately preceded by (methionine⁻¹), (alanine⁻¹), (methionine⁻², alanine⁻¹), (serine⁻¹), (methionine⁻², serine⁻¹), (cysteine⁻¹), or (methionine⁻², cysteine⁻¹).

Preferably, R₁ is selected from the from the group consisting of angiostatin K1, angiostatin K5, angiostatin K1-K3, angiostatin K1-K4, or a functional homologue thereof.

Preferably R₂ is selected from the from the group consisting of interferon alpha A/D and interferon alpha 2b.

Even more preferably, R, is selected from the group consisting of interferon alpha 2a, interferon alpha 2b, interferon alpha hybrid A/D, type I interferon variants, and consensus interferon, or a functional homologue or variant protein thereof.

Additionally, the present invention relates to recombinant expression vectors comprising nucleotide sequences encoding the multi-functional proteins, related microbial and eukaryotic expression systems, and processes for making the multi-functional proteins. The invention also relates to pharmaceutical compositions containing the multi-functional proteins, and methods for using the multi-functional proteins.

### Definitions

The term "multi-functional protein" means a single polypeptide which inherently possesses two distinct activities. In the context of the current invention, anti-angiogenic and/or anti-tumor activities are contemplated. The polypeptide may be formed by the covalent union of two distinct proteins or portions thereof, or two copies of the same protein, or portions thereof.

The term "anti-tumor" means possessing an activity which slows or abolishes the growth of, or which kills, or otherwise harms tumors *in vivo*.

The term "native sequence" refers to an amino acid or nucleic acid sequence which is identical to a wild-type or native form of a gene or protein.

The terms "mutant amino acid sequence," "mutant protein", "variant protein", "mutein", or "mutant polypeptide" refer to a polypeptide having an amino acid sequence which varies from a native sequence due to amino acid additions, deletions, substitutions, or all three, or is encoded by a nucleotide sequence from an intentionally-made variant derived from a native sequence.

The term "angiostatin" means a protein fragment of the heavy chain (SEQ ID NO: 37) of plasminogen having anti-angiogenic activity. The activity of said fragments can be determined by the mouse corneal micropocket assay of angiogenesis or by inhibition of endothelial cell growth *in vitro*.

The term "kringle" or "K" means a fragment or substructure of plasminogen or angiostatin bounded by disulfide-linked cysteine residues, and which retain a structure similar to that which it will adopt within the whole of plasminogen. A kringle domain may also include interkringle sequences which serve to connect it with other kringle domains. The terms K1, K2, K3, K4, K5 refer to specific kringle domains, and terms like K1-3 or K2-4, for example, refer to contiguous protein segments from the first kringle through the last kringle in a series, including the interkringle sequences.

The term "interferon" includes one of a group of proteins having anti-viral and anti-tumor activities including type I interferon, type I interferon variants, interferon alpha 2a, interferon alpha 2b , interferon alpha hybrid A/D, consensus interferon, functional homologues thereof, and those encoded by a DNA sequences related to those in SEQ ID NO: 27, SEQ ID NO: 28, SEQ ID NO: 29, and SEQ ID NO: 31.

The term "thrombospondin" means the proteins identical or essentially identical to thrombospondin and internal fragments of thrombospondin which possess anti-angiogenic activity.

### Brief Description of the Figures

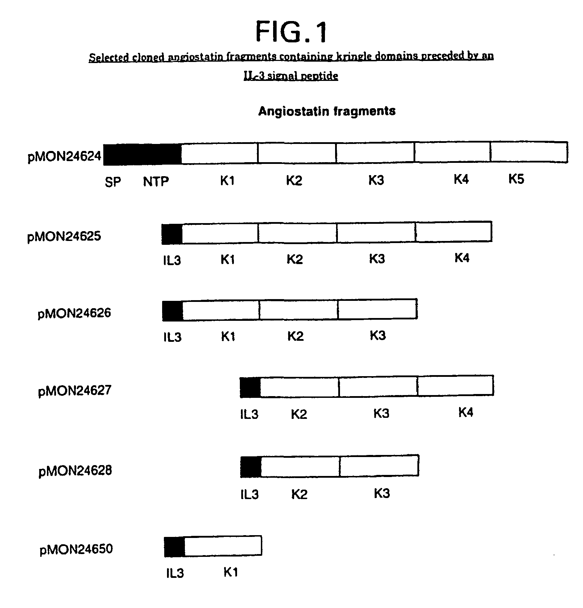

### Figure 1 shows a schematic of selected cloned angiostatin fragments containing kringle domains preceded by an IL-3 signal peptide

The labels K1, K2, K3, K4, and K5, refer to specific kringle domains within human plasminogen or angiostatin. Plasmid pMON24624 contains the sequence of the heavy chain of human plasminogen including the N-terminal peptide (NTP) and the signal peptide (SP). Plasmids pMON24625 (K1-K4), pMON24626 (K1-K3), pMON24627 (K2-K4), pMON24628 (K2-K3), and pMON24650 (K1), each contain various kringle domains, preceded by the IL-3 signal peptide (IL3). These plasmids also contain sequences necessary for expression and secretion of the proteins from mammalian (e.g., BHK) cells.

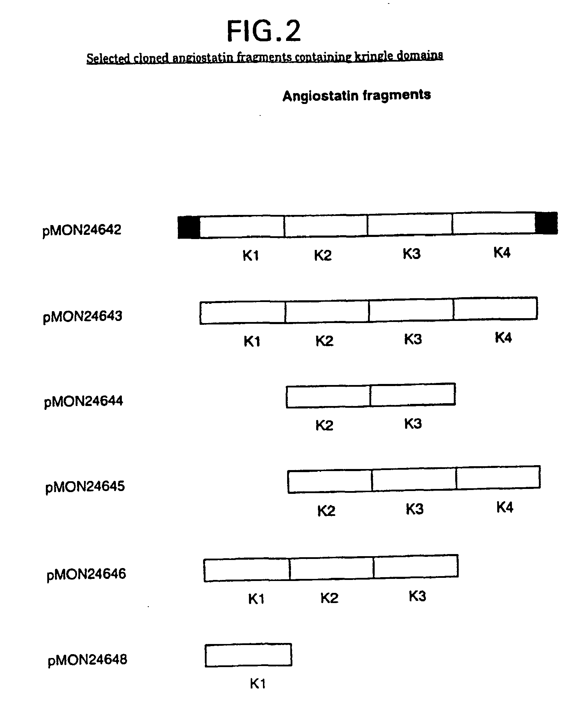

### Figure 2 shows a schematic of selected cloned angiostatin fragments containing kringle domains

The plasmid pMON24642 encodes kringles K1-K4 plus additional human plasminogen sequences at its 5' and 3' ends shown in the diagram by solid areas. Plasmids pMON24643 (K1-K4), pMON24644 (K2-K3), pMON24645 (K2-K3), pMON24646 (K1-K3), and pMON24648 (K1) contain specific kringle domains of human plasminogen. These plasmids also contain sequences necessary for the expression of the encoded polypeptides in *E. coli.*

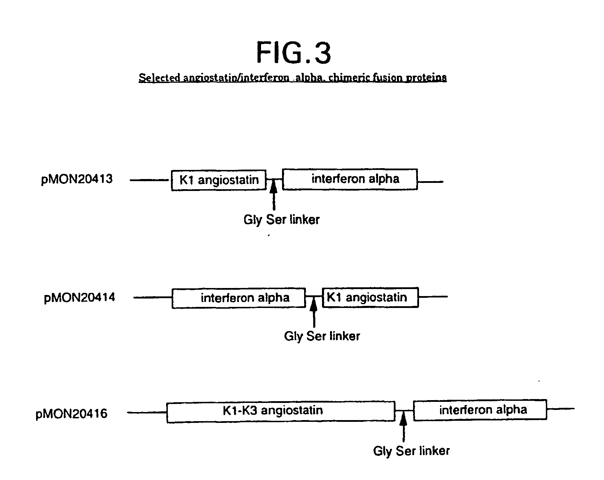

### Figure 3 shows a schematic of selected angiostatin/interferon alpha 2b chimeric fusion proteins

Plasmid pMON20413 encodes the K1 kringle domain fused to interferon alpha through a Glycine-Serine (Gly-Ser) linker peptide. Plasmid pMON20414 is similar to PMON20413, but encodes a chimeric fusion protein containing interferon alpha at its N-terminus fused to the Gly-Ser linker, which is fused to the K1 kringle domain of plasminogen (angiostatin). Plasmid pMON20416 encodes a fusion protein consisting of kringlc domains K1-K3 fused to interferon alpha with a Gly-Ser linker.

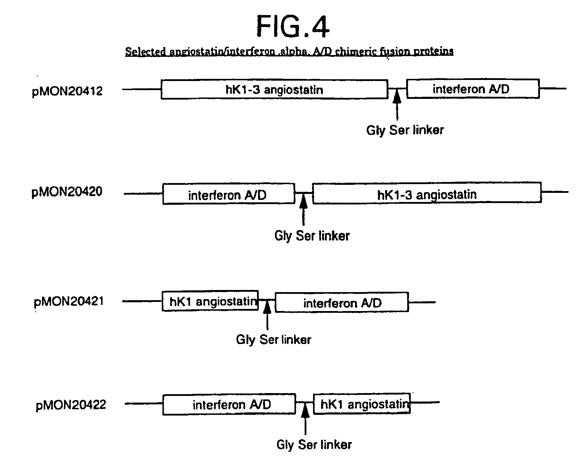

### Figure 4 shows a schematic of selected angiostatin/interferon alpha A/D chimeric fusion proteins

Plasmid pMON20412 encodes kringles K1-K3 fused to the hybrid A/D interferon with a Gly-Ser linker. Plasmid pMON20420 encodes a fusion protein containing the hybrid A/D interferon fused to kringles K1-K3 through a Gly-Ser linker. Plasmid pMON20421 encodes kringle K1 fused to the hybrid A/D interferon with a Gly-Ser linker. Plasmid pMON20422 encodes a fusion protein containing the hybrid A/D interferon fused to kringle K1 through a Gly-Ser linker.

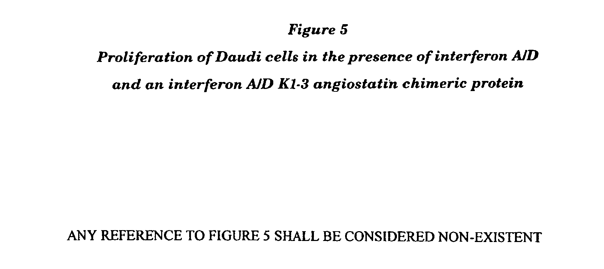

### Figure 5 shows the proliferation of Daudi cells in the presence of interferon A/D and an interferon A/D K1-3 angiostatin chimeric protein

Two replicates of assays depicting the proliferation of Daudi cells in the presence of interferon A/D (standard, from Genzyme) and an interferon A/D K1-3 angiostatin chimeric protein are shown. Protein samples were serially diluted, beginning at a concentration of 1 mM.

### Detailed Description of the Invention

The present invention encompasses multi-functional proteins (MFPs) which inherently possess two distinct activities. In the context of this invention, anti-angiogenic and/or anti-tumor activities are contemplated. The polypeptide may be formed by the covalent union of two distinct proteins, or portions thereof. Each distinct protein segment which may act through a different and specific cell receptor to initiate complementary biological activities. The MFPs may also be formed from covalently-linked polypeptides derived from the same protein.

### Peptide-bonded chimeric fusion proteins

Novel compounds of this invention are represented by a formula selected from the group consisting of:

R₁-L₁-R₂,

R₂-L₁-R₁,

R₁-L₁-R₁,

R₁-R₂,

R₂-R₁,

R₁-R₁

Where R₁ and R₂ are as defined above.

R₂ is preferably a protein or protein fragment with a different but complementary activity than R₁. Complementary activity is meant to be activity which enhances or changes the response to another cell modulator. The R₁ polypeptide is joined either directly or through a linker segment to the R₂ polypeptide. The term "directly" defines multi-functional proteins in which the polypeptides are joined without a peptide linker. Thus L₁ represents a chemical bond or polypeptide segment to which both R₁ and R₂ are joined in frame. Most commonly L₁ is a linear peptide in which R₁ and R₂ are joined by amide bonds, linking the carboxy terminus of R₁ to the amino terminus of L₁ and carboxy terminus of L₁ to the amino terminus of R₂. "Joined in frame" means that there is no translation termination or disruption between the reading frames of the DNA encoding R₁ and R₂.

Additionally, this invention encompasses the use of modified R₁ or R₂ molecules or mutated or modified DNA sequences encoding these R₁ or R₂ molecules. The present invention also includes multi-functional proteins in which R₁ or R₂ is a variant.

The linking group (L₁) is generally a polypeptide of between 1 and 500 amino acids in length. The linkers joining the two molecules are preferably designed to (1) allow the two molecules to fold and act independently of each other, (2) not have a propensity for developing an ordered secondary structure which could interfere with the functional domains of the two proteins, (3) have minimal hydrophobic characteristics which could interact with the functional protein domains and (4) provide steric separation of R₁ and R₂ such that R₁ and R₂ could interact simultaneously with their corresponding receptors on a single cell. Typically surface amino acids in flexible protein regions include Gly, Asn and Ser. Virtually any permutation of amino acid sequences containing Gly, Asn and Ser would be expected to satisfy the above criteria for a linker sequence. Other neutral amino acids, such as Thr and Ala, may also be used in the linker sequence. Additional amino acids may also be included in the linkers due to the addition of unique restriction sites in the linker sequence to facilitate construction of the multi-functional proteins.

Preferred L, linkers of the present invention include sequences selected from the group of formulas:
(Gly³Ser)ⁿ (SEQ ID NO. 88).
(Gly⁴Ser)ⁿ (SEQ ID NO. 89).
(Gly⁵Ser)ⁿ (SEQ ID NO. 90),
(GlyⁿSer)ⁿ (SEQ ID NO. 92) or
(AlaGlySer)ⁿ (SEQ ID NO. 91).

One example of a highly-flexible linker is the glycine and serine-rich spacer region present within the pIII protein of the filamentous bacteriophages, e.g. bacteriophages M13 or fd (Schaller et al., PNAS USA 72: 737-741, 1975). This region provides a long, flexible spacer region between two domains of the pIII surface protein. The spacer region consists of the amino acid sequence:

GlyGlyGlySerGlyGlyGlySerGlyGlyGlySerGluGlyGlyGlySerGlu
GlyGlyGlySerGluGlyGlyGlySerGluGlyGlyGlySerGlyGlyGlySer (SEQ ID NO. 93)

The present invention also includes linkers in which an endopeptidase recognition sequence is included. Such a cleavage site may be valuable to separate the individual components of the multi-functional protein to determine if they are properly folded and active *in vitro*. Examples of various endopeptidases include, but are not limited to, plasmin, enterokinase, kallikrein, urokinase, tissue plasminogen activator, clostripain, chymosin, collagenase, Russell's viper venom protease, post-proline cleavage enzyme, V8 protease, Thrombin and factor Xa.

Peptide linker segments from the hinge region of heavy chain immunoglobulins IgG, IgA, IgM, IgD or IgE provide an angular relationship between the attached polypeptides. Especially useful are those hinge regions where the cysteines are replaced with serines. Preferred linkers of the present invention include sequences derived from murine IgG gamma 2b hinge region in which the cysteines have been changed to serines. These linkers may also include an endopeptidase cleavage site. Examples of such linkers include the following sequences:

IleSerGluProSerGlyProIleSerThrIleAsnProSerProProSerLys
GluSerHisLysSerPro (SEQ ID NO. 94) and

IleGluGlyArgIleSerGluProSerGlyProIleSerThrIleAsnProSer
ProProSerLysGluSerHisLysSerPro (SEQ ID NO. 95).

The present invention is, however, not limited by the form, size or number of linker sequences employed. The only requirement of the linker is that it does not functionally interfere with the folding and function of the individual molecules of the multi-functional protein.

Additional peptide sequences may also be added to facilitate purification or identification of multi-functional proteins (e.g., poly-His). A highly antigenic peptide may also be added that would enable rapid assay and facile purification of the multi-functional protein by a specif monoclonal antibody.

Multi-functional proteins of the present invention may exhibit useful properties such as having similar or greater biological activity when compared to a single factor or by having improved half-life or decreased adverse side effects, or a combination of these properties.

Multi-functional proteins which have little or no activity maybe useful as antigens for the production of antibodies for use in immunology or immunotherapy, as genetic probes or as intermediates used to construct other useful muteins.

Biological activity of the multi-functional proteins of the present invention can be determined by tumor cell proliferation assays, endothelial cell proliferation assays, endothelial cell migration assays, endothelial cell tube formation assays, mouse corneal micro-pocket angiogenesis assays, and tumor growth assays. Syngeneic models of mouse tumor growth, such as the Lewis Lung carcinoma assay (Sugiura and Stock, Cancer Res., 15: 38-51, 1955; O'Reilly et al., Cell (Cambridge, Mass) 79(2): 315-328, 1994), and xenograft models of human tumors in nude or SCID mice using human breast cancer, prostate carcinoma, or melanoma cell lines are also used (Price, Breast Cancer Research and Treatment, 39: 93-102, 1996; Sasaki et al., Can. Res. 55: 3551-3557, 1995;Pretlow et al, Can. Res. 51: 3814-3817, 1991 and Passaniti et al., Int. J. Cancer, 51: 318-324, 1992; Felding-Habermann et al., J. Clin. Invest., 89: 2018-2022, 1992).

The biological activity of individual protein subunits can be performed using specific assays. The antiviral activity of interferon, for example, can be carried out by titering the potency of interferon preparations on Madin Darby bovine kidney cells infected with vesicular stomatitis virus (Rubinstein et al., *J. Virol.* **37**(2): 755-758, 1981).

The multi-functional proteins of the present invention may have an improved therapeutic profile as compared to single-acting anti-angiogenic or anti-tumor proteins. For example, some multi-functional proteins of the present invention may have a similar or more potent anti-tumor activity relative to other anti-tumor proteins without having a similar or corresponding increase in side-effects.

### Therapeutic targets

The multi-functional proteins of the present invention may be useful in the treatment of angiogenic-mediated diseases such as cancer, diabetic retinopathy, and macular degeneration. Among the cancers susceptible to treatment with the polypeptides of the present invention are lung cancer, breast cancer, ovarian cancer, prostate cancer, pancreatic cancer, gastric cancer, colon cancer, renal cancer, bladder cancer, melanoma, hepatoma, sarcoma, and lymphoma.

The present invention provides an improvement to the existing methods of treating solid tumors, in that it provides methods utilizing multi-functional proteins that have improved biological activities. Therapeutic treatment of tumors with these multi-functional proteins of the present invention may avoid undesirable side effects caused by treatment with presently available drugs. The treatment of solid tumors may include administration of a pharmaceutical composition containing the multi-functional proteins to a patient.

Other aspects of the present invention are methods and therapeutic compositions for treating the conditions referred to above. Such compositions comprise a therapeutically effective amount of one or more of the multi-functional proteins of the present invention in a mixture with a pharmaceutically acceptable carrier. The compositions can also be admixtures containing adjunctive agents, such as chemotherapeutic or immunotherapeutic agents. This composition can be administered either parenterally, intravenously, or subcutaneously. Other routes of administration are also contemplated, including intranasal and transdermal routes, and by inhalation. When administered, the therapeutic composition for use in this invention is preferably in the form of a pyrogen-free, parenterally-acceptable aqueous solution. The preparation of such a parenterally-acceptable protein solution, having due regard to pH, isotonicity, stability and the like, is within the skill of the art.

The dosage regimen involved in a method for treating the above-described conditions will be determined by the attending physician considering various factors which modify the action of drugs, e.g., the condition, body weight, sex, and diet of the patient, the severity of any infection, time of administration and other clinical factors. Generally, a daily regimen may be about 1.0 µg/kg of multi-functional proteins per kilogram of body weight. Dosages would be adjusted relative to the activity of a given multi-functional proteins and it would not be unreasonable to note that dosage regimens may include doses as low as 0.1 microgram and as high as 100 milligrams per kilogram of body weight per day.

In addition, there may exist specific circumstances where dosages of multi-functional proteins would be adjusted higher or lower than the range of 0.2 - 100,000 micrograms per kilogram of body weight. These include co-administration with other anti-angiogenic or antitumor proteins or variants; co-administration with adjunctive agents such as chemotherapeutic or immunotherapeutic agents, co-administration with chemotherapeutic drugs and/or radiation; the use of glycosylated multi-functional proteins; and various patient-related issues mentioned earlier in this section. As indicated above, the therapeutic method and compositions may also include co-administration with other human anti-tumor proteins.

A non-exclusive list of other appropriate anti-angiogenic or anti-tumor agents or treatments includes chemotherapy, radiation therapy, hormonal therapy, or interleukin-2, or combinations thereof. The dosage recited above would be adjusted to compensate for such additional components in the therapeutic composition.

Delivery of multi-functional proteins to a patient could also be performed through gene therapy. There are a variety of methods, known to those with skill in the art, for introducing genetic material into a host cell. A number of vectors, both viral and non-viral have been developed for transferring therapeutic genes into primary cells. Viral based vectors include; 1) replication deficient recombinant retrovirus (Boris-Lawrie and Temin, Curr. Opin. Genet. Dev. 3:102-109, 1993, Boris-Lawrie and Temin, Annal. New York Acad. Sci. 716:59-71, 1994, Miller, Current Top. Microbiol. Immunol. 158:1-24. 1992) and replication-deficient recombinant adenovirus (Berkner, BioTechniques 6:616-629, 1988, Berkner, Current Top. Microbiol. Immunol. 158:39-66, 1992, Brody and Crystal, Annal. New York Acad. Sci. 716:90-103, 1994). Non-viral based vectors include protein/DNA complexes (Cristiano et al., PNAS USA. 90:2122-2126, 1993, Curiel et al., PNAS USA 88:8850-8854, 1991, Curiel, Annal. New York Acad. Sci. 716:36-58, 1994), electroporation and liposome mediated delivery such as cationic liposomes (Farhood et al., Annal. New York Acad. Sci. 716:23-35, 1994).

### Cloning and expression of genes encoding multi-functional proteins

The present invention also includes the DNA sequences which code for the multi-functional proteins, DNA sequences which are substantially similar and perform substantially the same function, and DNA sequences which differ from the DNAs encoding the multi-functional proteins of the invention only due to the degeneracy of the genetic code. Also included in the present invention are the oligonucleotide intermediates used to construct the mutant DNAs and the polypeptides coded for by these oligonucleotides.

Genetic engineering techniques now standard in the art (United States Patent 4,935,233 and Sambrook et al., "Molecular Cloning A Laboratory Manual", Cold Spring Harbor Laboratory, 1989) may be used in the construction of the DNA sequences of the present invention. One such method is cassette mutagenesis (Wells et al., Gene 34:315-323, 1985) in which a portion of the coding sequence in a plasmid is replaced with synthetic oligonucleotides that encode the desired amino acid substitutions in a portion of the gene between two restriction sites.

Pairs of complementary synthetic oligonucleotides encoding the desired gene can be made and annealed to each other. The DNA sequence of the oligonucleotide would encode sequence for amino acids of desired gene with the exception of those substituted and/or deleted from the sequence.

Plasmid DNA can be treated with the chosen restriction endonucleases then ligated to the annealed oligonucleotides. The ligated mixtures can be used to transform competent *E. coli* cells to resistance to an appropriate antibiotic. Single colonies can be picked and the plasmid DNA examined by restriction analysis and/or DNA sequencing to identify plasmids with the desired genes.

Cloning of DNA sequences encoding novel multi-functional proteins may be accomplished by the use of intermediate vectors. Alternatively, one gene can be cloned directly into a vector containing the other gene. Linkers and adapters can be used for joining the DNA sequences, as well as replacing lost sequences, where a restriction site was internal to the region of interest. Thus genetic material (DNA) encoding one polypeptide, peptide linker, and the other polypeptide is inserted into a suitable expression vector which is used to transform bacteria, yeast, insect cells or mammalian cells. The transformed organism or cell line is grown and the protein isolated by standard techniques. The resulting product is therefore a new protein which has all or a portion of one protein joined by a linker region to a all or a portion of second protein.

Another aspect of the present invention includes plasmid DNA vectors for use in the expression of these novel multi-functional proteins. These vectors contain the novel DNA sequences described above which code for the novel polypeptides of the invention. Appropriate vectors which can transform microorganisms or cell lines capable of expressing the multi-functional proteins include expression vectors comprising nucleotide sequences coding for the multi-functional proteins joined to transcriptional and translational regulatory sequences which are selected according to the host cells used.

Vectors incorporating modified sequences as described above are included in the present invention and are useful in the production of the multi-functional proteins. The vector employed in the method also contains selected regulatory sequences in operative association with the DNA coding sequences of the invention and which are capable of directing the replication and expression thereof in selected host cells.

Methods for producing the novel multi-functional proteins is another aspect of the present invention. The method of the present invention involves culturing suitable cells or cell lines, which has been transformed with a vector containing a DNA sequence coding for expression of a novel multi-functional protein. Suitable cells or cell lines may be bacterial cells. For example, the various strains of *E. coli* are well-known as host cells in the field of biotechnology. Examples of such strains include *E. coli* strains JM101 (Yanisch-Perron et al. Gene 33: 103-119, 1985) and MON105 (Obukowicz et al., Applied Environmental Microbiology 58: 1511-1523, 1992). Also included in the present invention is the expression of the multi-functional proteins utilizing a chromosomal expression vector for *E. coli* based on the bacteriophage Mu (Weinberg et al., Gene 126: 25-33, 1993). Various strains of *B. subtilis* may also be employed in this method. Many strains of yeast cells known to those skilled in the art are also available as host cells for expression of the polypeptides of the present invention.

When expressed in the *E. coli* cytoplasm, the gene encoding the multi-functional proteins of the present invention may also be constructed such that at the 5' end of the gene codons are added to encode Met⁻²-Ala⁻¹, Met⁻²-Ser⁻¹, Met⁻²-Cys⁻¹, or Met⁻¹ at the N-terminus of the protein. The N termini of proteins made in the cytoplasm of *E. coli* are affected by post-translational processing by methionine aminopeptidase (Ben Bassat et al., J. Bacteriol. 169:751-757, 1987) and possibly by other peptidases so that upon expression the methionine is cleaved off the N-terminus. The multi-functional proteins of the present invention may include multi-functional proteins polypeptides having Met⁻¹, Ala⁻¹, Ser⁻¹, Cys⁻¹, Met⁻²-Ala⁻¹, Met⁻²-Ser⁻¹, or Met⁻²-Cys⁻¹ at the N-terminus. These mutant multi-functional proteins may also be expressed in *E. coli* by fusing a secretion signal peptide to the N-terminus. This signal peptide is cleaved from the polypeptide as part of the secretion process.

Also suitable for use in the present invention are mammalian cells, such as Chinese hamster ovary (CHO) cells. General methods for expression of foreign genes in mammalian cells are reviewed in Kaufman, R. J., 1987, "Genetic Engineering, Principles and Methods", Vol. 9, J. K. Setlow, editor, Plenum Press, New York. An expression vector is constructed in which a strong promoter capable of functioning in mammalian cells drives transcription of a eukaryotic secretion signal peptide coding region, which is translationally joined to the coding region for the multi-functional proteins. For example, plasmids such as pcDNA I/Neo, pRc/RSV, and pRdCMV (obtained from Invitrogen Corp., San Diego, California) can be used. The eukaryotic secretion signal peptide coding region can be from the gene itself or it can be from another secreted mammalian protein (Bayne, M. L. et al., Proc. Natl. Acad. Sci. USA 84: 2638-2642, 1987). After construction of the vector containing the gene, the vector DNA is transfected into mammalian cells such as the COS7, HeLa, BHK, CHO, or mouse L lines. The cells can be cultured, for example, in DMEM media (JRH Scientific). The polypeptide secreted into the media can be recovered by standard biochemical approaches following transient expression for 24 - 72 hours after transfection of the cells or after establishment of stable cell lines following selection for antibiotic resistance. The selection of suitable mammalian host cells and methods for transformation, culture, amplification, screening and product production and purification are known in the art. See, e.g., Gething and Sambrook, Nature, 293:620-625, 1981, or alternatively, Kaufman et al, Mol. Cell. Biol., 5(7):1750-1759, 1985) or Howley et al., and U.S. Pat. No. 4,419,446. Other suitable mammalian cell lines are the monkey COS-1 cell line and the CV-1 cell line.

Where desired, insect cells may be utilized as host cells in the method of the present invention. See, e.g., Luckow, V.A., Protein Eng. J. L. Cleland., Wiley-Liss, New York, NY: 183-218, 1996 and references cited therein. In addition, general methods for expression of foreign genes in insect cells using baculovirus vectors are described in: O'Reilly, D. R., L. K. Miller et al. Baculovirus Expression Vectors: A Laboratory Manual. New York, W.H. Freeman and Company, 1992, and King, L. A. and R. D. Possee, The Baculovirus Expression System: A Laboratory Guide, London, Chapman & Hall. An expression vector is constructed comprising a baculovirus transfer vector, in which a strong baculovirus promoter (such as the polyhedrin promoter) drives transcription of a eukaryotic secretion signal peptide coding region, which is translationally joined to the coding region for the multi-functional protein. For example, the plasmid pVL1393 (obtained from Invitrogen Corp., San Diego, California) can be used. After construction of the vector carrying the gene encoding the multi-functional protein, two micrograms of this DNA is co-transfected with one microgram of baculovirus DNA into *Spodoptera frugiperda* insect cells, strain Sf9. Alternatively, recombinant baculoviruses can be created using a baculovirus shuttle vector system (Luckow, VA. et al., J. Virol. 67(8): 4566-4579, 1993) now marketed as the Bac-To-Bac™ Expression System (Life Technologies, Inc. Rockville, MD). Pure recombinant baculovirus carrying the multi-functional protein is used to infect cells cultured, for example, in Excell 401 serum-free medium (JRH Biosciences, Lenexa, Kansas) or Sf900-II (Life Technologies, Inc.). The multi-functional protein secreted into the medium can be recovered by standard biochemical approaches. Supernatants from mammalian or insect cells expressing the multi-functional proteins can be first concentrated using any of a number of commercial concentration units.

The following examples will illustrate the invention in greater detail although it will be understood that the invention is not limited to these specific examples. Various other examples will be apparent to the person skilled in the art after reading the present disclosure without departing from the spirit and scope of the invention. It is intended that all such other examples be included within the scope of the appended claims.

### Materials and Methods

### General methods

General methods of cloning, expressing, and characterizing proteins are found in T. Maniatis et al., Molecular Cloning, A Laboratory Manual, Cold Spring Harbor Laboratory, 1982, and references cited therein, incorporated herein by reference; and in J. Sambrook et al., Molecular Cloning, A Laboratory Manual, 2nd edition, Cold Spring Harbor Laboratory, 1989, and references cited therein, incorporated herein by reference.

Unless noted otherwise, all specialty chemicals were obtained from Sigma, Co. (St. Louis, MO). Restriction endonucleases and T4 DNA ligase were obtained from New England Biolabs (Beverly, MA) or Boehringer Mannheim (Indianapolis, IN).

### Transformation of E. coli strains

*E. coli* strains (Table 1), such as DH5α™ (Life Technologies, Gaithersburg, MD) and TG1 (Amersham Corp., Arlington Heights, IL) are used for transformation of ligation reactions and are the hosts used to prepare plasmid DNA for transfecting mammalian cells. *E. coli* strains, such as JM101 (Yanisch-Perron et al., Gene, 33: 103-119, 1985) and MON105 (Obukowicz et al., Appl. and Envir. Micr., 58: 1511-1523, 1992) can be used for expressing the multi-functional proteins of the present invention in the cytoplasm or periplasmic space.

**TABLE 1 Strains**

| **Designation** | **Description** | **Reference/Source** |
|---|---|---|
| DH5α™ | F, phi80 d*lac*ZdeltaM15, delta(*lac*ZYA-*arg*F)U169, *deo*R, *rec*A1, *end*A1, *hsd*R17 (rk,mk⁺), *pho*A, *sup*E44, lambda-, *thi*-1, *gyr*A96, relA1 | Life Technologies, Rockville, Maryland |
| JM101 (ATCC#33876) | delta (*pro lac), sup*E, *thi*,F'(*tra*D36, *pro*A'B', *lac*I^{q}, *lac*ZdeltaM15) | Yanisch-Perron et al., Gene, 33: 103-119, 1985 |
| MON105 (ATCC#55204) | F, lambda-,IN (rrnD, rrnE)1, rpoD', *rpo*H358 | Obukowicz et al., Appl. and Envir. Micr., 58: 1511-1523,1992 |
| MON208 | W3110 *rpo*H358, *lac*I^{Q}, *omp*T::*kan* | Alan Easton, Monsanto Company |
| TG1 | delta(lac-pro), *sup*E, *thi*-1, *hsd*D5/F'(*tra*D36, *pro*A⁺B⁺, *lac*I^{q}, *lac*ZdeltaM15) | Amersham Corp., Arlington Heights, Illinois |

DH5α™ Subcloning efficiency cells are purchased as competent cells and are ready for transformation using the manufacturer's protocol, while both *E. coli* strains TG1 and MON105 are rendered competent to take up DNA using a CaCl₂ method. Typically, 20 to 50 mL of cells are grown in LB medium (1% Bacto-tryptone, 0.5% Bacto-yeast extract, 150 mM NaCl) to a density of approximately 1.0 optical density unit at 600 nanometers (OD600) as measured by a Baush & Lomb Spectronic spectrophotometer (Rochester, NY). The cells are collected by centrifugation and resuspended in one-fifth culture volume of CaCl₂ solution (50 mM CaCl₂, 10 mM Tris-Cl, pH7.4) and are held at 4°C for 30 minutes. The cells are again collected by centrifugation and resuspended in one-tenth culture volume of CaCl₂ solution. Ligated DNA is added to 0.2 mL of these cells, and the samples are held at 4°C for 30-60 minutes. The samples are shifted to 42°C for two minutes and 1.0 mL of LB is added prior to shaking the samples at 37°C for one hour. Cells from these samples are spread on plates (LB medium plus 1.5% Bacto-agar) containing either ampicillin (100 micrograms/mL, ug/mL) when selecting for ampicillin-resistant transformants, or spectinomycin (75 ug/mL) when selecting for spectinomycin-resistant transformants. The plates are incubated overnight at 37°C.

Colonies are picked and inoculated into LB plus appropriate antibiotic (100 ug/mL ampicillin or 75 ug/mL spectinomycin) and are grown at 37°C while shaking.

### Cloning and Expression

### DNA isolation and characterization

Plasmid DNA can be isolated by a number of different methods and using commercially available kits known to those skilled in the art. Plasmid DNA is isolated using the Promega Wizard™ Miniprep kit (Madison, WI), the Qiagen QlAwell Plasmid isolation kits (Chatsworth, CA) or Qiagen Plasmid Midi or Mini kit. These kits follow the same general procedure for plasmid DNA isolation. Briefly, cells are pelleted by centrifugation (5000 x g), the plasmid DNA released with sequential NaOH/acid treatment, and cellular debris is removed by centrifugation (10000 x g). The supernatant (containing the plasmid DNA) is loaded onto a column containing a DNA-binding resin, the column is washed, and plasmid DNA eluted. After screening for the colonies with the plasmid of interest, the *E. coli* cells are inoculated into 50-100 ml of LB plus appropriate antibiotic for overnight growth at 37°C in an air incubator while shaking. The purified plasmid DNA is used for DNA sequencing, further restriction enzyme digestion, additional subcloning of DNA fragments and transfection into *E. coli*, mammalian cells, or other cell types.

### Sequence confirmation

Purified plasmid DNA is resuspended in dH₂O and its concentration is determined by measuring the absorbance at 260/280 nm in a Bausch and Lomb Spectronic 601 UV spectrometer. DNA samples are sequenced using ABI PRISM™ DyeDeoxy™ terminator sequencing chemistry (Applied Biosystems Division of Perkin Elmer Corporation, Lincoln City, CA) kits (Part Number 401388 or 402078) according to the manufacturer's suggested protocol usually modified by the addition of 5% DMSO to the sequencing mixture. Sequencing reactions are performed in a DNA thermal cycler (Perkin Elmer Corporation, Norwalk, CT) following the recommended amplification conditions. Samples are purified to remove excess dye terminators with Centri-Sep™ spin columns (Princeton Separations, Adelphia, NJ) and lyophilized. Fluorescent dye labeled sequencing reactions are resuspended in deionized formamide, and sequenced on denaturing 4.75% polyacrylamide-8M urea gels using ABI Model 373A and Model 377 automated DNA sequencers. Overlapping DNA sequence fragments are analyzed and assembled into master DNA contigs using Sequencher DNA analysis software (Gene Codes Corporation, Ann Arbor, MI).

### Mammalian Cell Transfection / Production of Conditioned Media

The BHK-21 cell line can be obtained from the ATCC (Rockville, MD). The cells are cultured in Dulbecco's modified Eagle media (DMM/bigh-glucose), supplemented to 2 mM (mM) L-glutamine and 10% fetal bovine serum (FBS). This formulation is designated growth media. Selective media is BHK growth media supplemented with 453 units/mL hygromycin B (CalBiochem, San Diego, CA). The BHK-21 cell line was previously stably transfected with the HSV transactivating protein VP16, which transactivates the IE110 promoter found on the plasmid pMON3359 and pMON3633 and the IE175 promoter found in the plasmid pMON3360B (See Hippenmeyer et al., Bio/Technology, pp.1037-1041, 1993). The VP16 protein drives expression of genes inserted behind the IE110 or IE175 promoter. BHK-21 cells expressing the transactivating protein VP16 are designated BHK-VP16. The plasmid pMON1118 (See Highkin et al., Poultry Sci., 70: 970-981, 1991) expresses the hygromycin resistance gene from the SV40 promoter. A similar plasmid, pSV2-hph, is available from ATCC.

BHK-VP16 cells are seeded into a 60 millimeter (mm) tissue culture dish at 3 x 10⁵ cells per dish 24 hours prior to transfection. Cells are transfected for 16 hours in 3 mL of "OPTIMEM"™ (Gibco-BRL, Gaithersburg, MD) containing 10 ug of plasmid DNA containing the gene of interest, 3 ug hygromycin resistance plasmid, pMON1118, and 80 ug of Gibco-BRL "LIPOFECTAMINE"™ per dish. The media is subsequently aspirated and replaced with 3 mL of growth media. At 48 hours post-transfection, media from each dish is collected and assayed for activity (transient conditioned media). The cells are removed from the dish by trypsin-EDTA, diluted 1:10, and transferred to 100 mm tissue culture dishes containing 10 mL of selective media. After approximately 7 days in selective media, resistant cells grow into colonies several millimeters in diameter. The colonies are removed from the dish with filter paper (cut to approximately the same size as the colonies and soaked in trypsin/EDTA) and transferred to individual wells of a 24 well plate containing 1 mL of selective media. After the clones are grown to confluence, the conditioned media is re-assayed, and positive clones are expanded into growth media.

### Expression of chimeras in E. coli

*E. coli* strain MON105 or JM101 harboring the plasmid of interest are grown at 37°C in M9 plus casamino acids medium with shaking in an air incubator Model G25 from New Brunswick Scientific (Edison, NJ). Growth is monitored at OD₆₀₀ until it reaches a value of 1.0 at which time nalidixic acid (10 mg/mL) in 0.1 N NaOH is added to a final concentration of 50 µg/mL. The cultures are then shaken at 37°C for three to four additional hours. A high degree of aeration is maintained throughout culture period in order to achieve maximal production of the desired gene product. The cells are examined under a light microscope for the presence of inclusion bodies (IB). One mL aliquots of the culture are removed for analysis of protein content by boiling the pelleted cells, treating them with reducing buffer and electrophoresis via SDS-PAGE (see Maniatis et al., "Molecular Cloning: A Laboratory Manual", 1982). The culture is centrifuged (5000 x g) to pellet the cells.

### Purification

### Isolation of Inclusion Bodies

The cell pellet from a 330 mL *E. coli* culture is resuspended in 15 mL of sonication buffer (10 mM 2-amino-2-(hydroxymethyl) 1,3-propanediol hydrochloride (Tris-HCl), pH 8.0 + 1 mM ethylenediaminetetraacetic acid (EDTA). These resuspended cells are sonicated using the microtip probe of a Sonicator Cell Disruptor (Model W-375, Heat Systems-Ultrasonics, Inc., Farmingdale, New York). Three rounds of sonication in sonication buffer followed by centrifugation are employed to disrupt the cells and wash the inclusion bodies (IB). The first round of sonication is a 3 minute burst followed by a 1 minute burst, and the final two rounds of sonication are for 1 minute each.

### Extraction and refolding of proteins from inclusion body pellets

All steps are at 4°C. Angiostatin K1-4 inclusion bodies were dissolved in 6M urea, 5 mM DTT, 50 mM Bis-Tris Propane, pH 10.8 at 1.2 mg/ml. This solution was stirred for 2 hours and then 6-amino hexanoic acid (stock 1M) was added to achieve a 6 mM concentration, followed by addition of cystine (stock 0.2M, pH 10.5) to 1 mM. This was stirred for 5 minutes and then the K1-4 angiostatin was diluted to 0.2 mg/ml by addition of 75 mM Bis-Tris propane, pH 10.5. It was then stirred for 72 hours to complete the refolding of the protein.

### Purification

Purification of angiostatin K1-4 was achieved using a Sepharose-Lys affinity column followed by Q-Sepharose chromatography. The sample was next dialyzed against 50 mM NaPO₄, pH 8.0 and then filtered through a 1 uM filter to clarify. This sample was loaded onto a Sepharose-Lys column equilibrated in 50 mM NaPO₄, pH 8.0. The load was at approximately 1.5 gm of K1-4 per ml of resin. After loading the column, it was washed with 1 column volume of equilibrating buffer and then K1-4 was eluted using a 10 column volume gradient from 0-8 mM 6-amino hexanoic acid in 50 mM NaPO₄, pH 8.0.

Fractions were analyzed by SDS-gel electrophoresis and/or RP-HPLC and pooled. This pool was made 40 mM Tris-Cl, and adjusted to pH 9.0. It was then dialyzed extensively vs. 40 mM Tris-Cl, pH 9.0 and applied to a Q-Sepharose HP column (-5 mgs protein/ml resin) equilibrated in the same buffer. K1-4 was then eluted using a NaCl gradient from 0-0.1M in the equilibrating buffer. Fractions were analyzed by RP-HPLC and/or SDS gel electrophoresis and pooled.

In some cases the folded proteins can be affinity-purified using affinity reagents such as monoclonal antibodies or receptor subunits attached to a suitable matrix. Purification can also be accomplished using any of a variety of chromatographic methods such as: ion exchange, gel filtration or hydrophobic chromatography or reversed phase HPLC. These and other protein purification methods are described in detail in Methods in Enzymology, Volume 182 "Guide to Protein Purification" edited by Murray Deutscher, Academic Press, San Diego, California, 1990.

### Protein Characterization

The purified protein is analyzed by RP-HPLC, electrospray mass spectrometry, and SDS-PAGE. The protein quantitation is done by amino acid composition, RP-HPLC, and Bradford protein determination. In some cases tryptic peptide mapping is performed in conjunction with electrospray mass spectrometry to confirm the identity of the protein.

### Biological Assays

### Assay for Human Interferon-Induced Antiviral Activity

Antiviral activity induced by human interferon is measured spectrophotometrically as inhibition of the cytopathic effect that normally results from infection of Madin Darby bovine kidney cells, (ATCC CCL #22), with vesicular stomatitis virus (VSV) (ATCC VR-158) (Rubinstein et al., J. Virol. 37(2): 755-758, 1981). VSV stocks are prepared on mouse L cells (L929) (ATCC CCL# 1). Samples of interferon are serially titrated in a 96-well plate format and incubated with 4 x 10⁴ cells per well, 6 hours prior to addition of virus at a multiplicity of infection of 0.1 plaque forming units per cell. The cells are stained with crystal violet at 20-24 hours postinfection and staining is measured spectrophotometrically at 680 nm. The relative potencies of the samples are compared with Intron A, a recombinant interferon produced by Schering Plough and obtained by prescription.

### Inhibitory effect on Proliferation of Daudi Cells

Human Burkitt's lymphoma Daudi cells (ATCC) are seeded at 2 x 10⁴ cells /well into 96-well tissue culture plates and cells are cultured in the presence or absence of serial doses of rhIFN alpha 2b for 3 days. Cultures are pulsed with ³H-thymidine for the last hour of the culture period, and the ³H-thymidine uptake, counts per minute (cpm), measured on a Beta-plate reader. All samples are assayed in triplicate.

### Endothelial cell proliferation assay

The endothelial cell proliferation assay was performed as described by Cao et al. (J. Biol. Chem. 271: 29461-29467, 1996). Briefly, human dermal microvascular endothelial cells (HdMVEC, Clonetics) or bovine microvascular endothelial cells (BacEnd, Incell) were maintained in MCDB131 containing 5% heat-inactivated fetal bovine serum (FBS, Hyclone), antibiotics, 100 ug/ml heparin (Sigma) and 100 ug/ml endothelial mitogen (Biomedical Technologies). Confluent monolayers at passages 2-5 were dispersed in .05% trypsin and resuspended in complete medium. Five hundred ul of complete media containing 1.25 x 10⁴ cells were seeded into wells of a 24-well tissue culture plate coated with 0.1% gelatin (Sigma). The cells were incubated overnight at 37°/5% CO₂ at which time the media was replaced with 250 ul of media containing 5% FBS and various concentrations of inhibitors. After 30 minutes of incubation, 250 ul of media containing 1 ng/ml bFGF (R&D Systems) was added and the cells were incubated for an additional 72 hours, at which time they were trypsinized and counted with a Coulter counter.

### Endothelial cell in vitro tube formation assay

The tube formation assay is performed as previous described (Gately et al., Cancer Res. 56:4887-4890, 1996). The effect of angiogenesis inhibitors can be tested in an in vitro angiogenesis assay; endothelial cell tube formation on matrix. Matrigel (Becton Dickson) was thawed on ice and diluted 1:1 with MCDB 131 medium supplemented with 2 mM L-glutamine, containing either buffer control or testing compounds. Aliquots of 0.5 ml of 1:1 diluted matrigel were plated into individual wells of 2-chambered Tissue-Tek polystyrene slides (Nunc) and allowed to polymerize at 37°C for at least 30 min. Primary bovine capillary endothelial cells derived from the bovine adrenal cortex (purchased from Incell) were cultured (as described for bovine endothelial cell proliferation). Following trypsinization, the cells were washed three times, then resuspended at 3.5 x 10⁵ cells/ml in MCDB131 medium supplemented with L-glutamine and 1% fetal bovine serum containing either the same buffer control or testing compounds. The endothelial cells (0.5 ml at 3.5 x 10⁶ cells /ml) were plated onto matrigel coated wells containing the same additives. After incubation for 16-24 hours at 37°C in a 5% CO2 incubator, cultures were evaluated for tube formation (sprouting). Random fields of each experimental condition were photographed using a Nikon N6006 camera under a Nikon Diaphot microscope. The photographs were then quantitated by measuring the total length of the tubes. Tube formation can also be quantitated using image analysis software. Endothelial cell tube networks were fixed in 100% methanol at -20°C for 7-10 min, rinsed 4 times with phosphate buffered saline (PBS) and incubated overnight at 4°C with rabbit polyclonal anti-human Factor VIII-related antigen (von Willibrand factor, vWF) (Dako). The next day, the cells were stained with a secondary antibody, Cy3-conjugated goat anti-rabbit IgG F(ab')2 specific (Jackson ImmunoResearch Laboratories). Tube formation was visualized using a Nikon microphot-FXA with a fluorescence filter linked to a computer containing image analysis software. Tube formation was measured as follows: Total area encompassed by endothelial cell tubes/Total endothelial cell surface area.

### Endothelial cell migration assay

The endothelial cell migration assay is performed essentially as previous described (Gately et al., Cancer Res. 56:4887-4890, 1996). To determine the ability of angiostatin to inhibit the migration of endothelial cells, migration assays were performed in a transwell chamber (Costar) containing 8 mm pore size polycarbonate membranes. The cells utilized in the assay were either human microvascular endothelial cells from Emory or bovine endothelial cells (kindly provided by Gately Northwestern University, Evanston, IL). The cells were starved overnight before use in MCDB131 + 0.1% BSA (human cells) or DMEM+0.1%BSA(bovine cells), harvested, and resuspended in the same media at 10⁵ cells/ml. The lower side of the transwells were coated with 0.1% gelatin for 30 minutes at 37°C before addition of 2 x 10⁶ cells to the upper chamber. The transwell was moved to a well containing the chemoattractant (bFGF or VEGF) in the lower chamber. Migration was allowed to occur overnight at 37°C. The membranes were then fixed and stained, and the number of cells that migrated to the lower side of the membrane counted in 3 high powered fields.

### Micro-pocket assay to evaluate anti-angiogenic activity

The corneal micropocket assay has been developed to evaluate the anti-angiogenic activity of test compounds in mice. BALBc or C57BL strains of mice are anesthetized with avertin (tribromoethanol, 125 mpk, 0.3-.4 ml/mouse, i.p., 25 ga needle). The eyes are topically anesthetized with 0.5% proparacaine. Only one eye is used. The eye is proptosed with a small forceps and under an operating microscope, a central, intrastromal linear keratotomy is performed with a #15 blade parallel to the insertion of the lateral rectus muscle. A modified cataract knife (1 x 20 mm) is then inserted to dissect a lamellar micropocket to within 1 mm of the temporal limbus. A single Hydron pellet containing either basic fibroblastic growth factor or vascular endothelial growth factor (bFGF or VEGF) is placed on the eye and pushed into the pocket with one arm of the forceps. The flap is self-sealing. Antibiotic ointment (Neobacimyx) is applied once to prevent infection and to decrease irritation of the irregular ocular surface.

Compounds are administered immediately post-operatively. They can be administered either orally, intraperitoneally, subcutaneously or intravenously, depending on bioavailability and potency of the compound. Dosing is from one to three times daily for oral compounds, one or two per day for i.p. or s.q., and once per day via the tail vein for i.v. delivery. Volumes do not exceed 5 ml/kg orally, 10 ml/kg i.p. or s.q. or 2.5 ml/kg i.v. All injections are done with a 25 guage needle.

On post-operative day 5 or 6 the mice are anesthetized with avertin (125 mpk, i.p.), the eyes proptosed, and the degree of neovascularization assessed by determining the maximum vessel length, and the contiguous circumferential zone involved. Using the formula for the area of an ellipse, the neovascular area is measured. The animals receive a thoracotomy while still anesthetized, to assure euthanasia. Some of the eyes are removed for histology. If blood samples are required for compound blood levels, the mice are bled by cardiac puncture immediately following the corneal neovascularization assessment. This is done via a substernal approach with a 1 inch, 23 guage needle, and the animal is subsequently euthanized.

Animals are monitored daily following surgery. Topical proparicaine is used as necessary to relieve irritation of the affected eye. The maximum number of bleeds per rat is four, every third day, although typically only two are required, one at day 4 or 6 and one at completion.

### Mouse models for anti-tumor activity

Several mouse models can be used to evaluate the anti-tumor activity of the chimeric proteins; either direct effects on the growth of the primary tumor or effects on metastasis. These can be divided into two broad classes: syngeneic models of mouse tumor in mice, such as the Lewis Lung Carcinoma (Sugiura and Stock, Cancer Res., 15: 38-51, 1955; O'Reilly et al., Cell (Cambridge, Mass) 79(2): 315-328, 1994) and xenograft models of human tumors in nude or severe combined immunodeficiency (SCID) mice. Examples of the human tumor xenografts include: the breast cancer cell lines, MDA-MB-435 (Price, Breast Cancer Research and Treatment, 39: 93-102, 1996) and MDA-231 (Sasaki et al., Can. Res. 55: 3551-3557, 1995), the human prostate carcinoma cell line, PC-3 (Pretlow et al, Can. Res. 51: 3814-3817, 1991; Passaniti et al., Int. J. Cancer, 51: 318-324, 1992) and the human melanoma line M21 (Felding-Habermann et al., J. Clin. Invest., 89: 2018-2022, 1992).

### Examples

### Example 1 Construction of plasmids encoding interferon alpha 2b

### Construction of pMON30422

An interferon alpha 2b (IFNα-2b) gene was amplifed from plasmid DNA from ATCC clone no. 67979 using the primer set, IF start (SEQ ID NO. 1) and IF stop (SEQ ID NO. 2).
Oligo Ifstart (SEQ ID NO: 1)
GATCGACCAT GGCTTGTGAT CTGCCTCAAA CC 32
Oligo Ifstop (SEQ ID NO:2)
CGATCGAAGC TTATTATTCC TTACTTCTTA AACTTT 36
The primers were designed to include the appropriate restriction enzyme recognition sites which allow cloning of the gene into expression plasmids. Conditions for polymerase chain reaction (PCR) amplification were 35 cycles 92°C degree. denaturation for one minute, 40°C annealing for one minute and 72°C extension for one minute. A 100 ul reaction contained 100 pmol of each primer and one ug of template DNA (isolated by Qiagen Miniprep); and 1x PCR reaction buffer, 200 uM dNTPs and 0.6 unit Taq DNA polymerase (Boehringer Mannheim). The PCR product was digested with restriction endonucleases *Nco*I and *Hin*dIII and gel-purified. The vector pMON6875, encoding a ptac promoter, G10L ribosome binding site and P22 terminator, was digested with restriction endonucleases *Nco*I and *Hin*dIII. The digested PCR product and vector fragment were combined and ligated. A portion of the ligation reaction was used to transform *E. coli* strain MON208. Transformant bacteria were selected on spectinomycin-containing plates. Plasmid DNA was isolated and sequenced to confirm the correct insert. The resulting plasmid was designated pMON30422.

### Construction of pMON30426

To optimize expression of IFNα-2b in *E. coli,* a new gene with an optimized N-terminus was amplified from plasmid DNA from pMON30422 using the primer set, New IF-A (SEQ ID NO. 3) and IF stop (SEQ ID NO. 2).
Oligo IFstop (SEQ ID NO:2)
CGATCGAAGC TTATTATTCC TTACTTCTTA AACTTT 36
Oligo NewIF-A (SEQ ID NO:3)
GATCGACCAT GGCTTGTGAT CTGCCGCAAA CTCATAGCCT GGGTAGCCGT CGCACCCTGA 60
TGCTGCTGGC TCAGATGCGC CGTATCTCTC TTTTCTCCTG CTTGAAGGAC AGACA 115
The primers were designed to include the appropriate restriction enzyme recognition sites which allow cloning of the gene into expression plasmids. Conditions for polymerase chain reaction (PCR) amplification were 35 cycles 92°C denaturation for one minute, 40°C annealing for one minute and 72°C extension for one minute. A 100 ul reaction contained 100 pmol of each primer and one ug of template DNA; and 1x PCR reaction buffer, 200 uM dNTPs and 0.6 unit Taq DNA polymerase (Boehringer Mannheim). The PCR product was digested with restriction endonucleases Ncol and HindIII and gel-purified. Vector DNA encoding a *ptac* promoter, G10L ribosome binding site and P22 terminator, pMON6875, was digested with restriction endonucleases *Nco*I and *Hind*III. The digested PCR product and vector fragment were combined and ligated. A portion of the ligation reaction was used to transform *E. coli* strain MON208. Transformed bacteria were selected on spectinomycin-containing plates. Plasmid DNA was isolated and sequenced to confirm the correct insert. The resulting plasmid was designated pMON30426. Both pMON30422 and pMON30426 encode the same peptide.

### Example 2. Creation of human interferon alpha A/D chimeras

### Construction of pMON20405

Plasmid, pMON30426, DNA was digested with the restriction enzymes *Nco*I and *Hind*III resulting in a 3207 bp vector fragment. Plasmid DNA from pMON30426 was digested with *Nco*I and *Bgl*II resulting in a 192 bp fragment. The 192 bp fragment along with a 315 bp *Bgl*II/*Hind*III fragment that was assembled from synthetic oligonucleotides IFND1 (SEQ ID NO. 11), IFND2 (SEQ ID NO. 12), IFND3X (SEQ ID NO. 13), IFND4X (SEQ ID NO. 14), IFND5 (SEQ ID NO. 15), IFND6 (SEQ ID NO. 16), IFND7 (SEQ ID NO. 17), and IFND8 (SEQ ID NO. 18) was ligated to the vector fragment. A portion of the ligation reaction was used to transform *E. coli* K-12 strain JM101. Transformant bacteria were selected on spectinomycin-containing plates. Plasmid DNA was isolated, analyzed by restriction analysis, and sequenced to confirm the correct insert. The genetic elements derived from plasmid pMON20405 are the pBR327 origin of replication, the *tac* promoter, the gene 10 leader (g10-L) ribosome binding site joined to human interferon (hIFN) alpha A/D hybrid, the P22 transcriptional terminator, and the streptomycin adenyltransferase gene.

### Construction of pMON20407

Plasmid, pMON31236, DNA was digested with the restriction enzymes *Nco*l and *Hind*III resulting in a 3159 bp vector fragment. Plasmid DNA from pMON20405 was digested with *Nco*l and *Hind*III resulting in a 507 bp fragment that was ligated to the vector fragment. A portion of the ligation reaction was used to transform *E. coli* K-12 strain JM101. Transformant bacteria were selected on spectinomycin-containing plates. Plasmid DNA was isolated from a colony grown in LB broth containing spectinomycin and analyzed by restriction analysis. The genetic elements derived from plasmid pMON20407 are the pBR327 origin of replication, the recA promoter, the gene 10 leader (g10-L) ribosome binding site joined to human interferon (hIFN) alpha A/D hybrid, the P22 transcriptional terminator, and the streptomycin adenyltransferase gene.

### Construction of pMON20408

Plasmid DNA from pMON24646-A7 was mutagenized using the QuikChange Site-Directed Mutagenesis Kit from Stratagene (La Jolla, CA). The *Nco*I site that is present in the K3 domain of human angiostatin was removed by changing base 714 from A to T with synthetic oligonucleotides HPLGN-CD1 (SEQ ID NO. 19) AND HPLGN-CD2 (SEQ ID NO. 20). The genetic elements derived from plasmid pMON20408 are the pBR327 origin of replication, the *rec*A promoter, the gene 10 leader (g10-L) ribosome binding site joined to human angiostatin K1-3, the T7 transcriptional terminator and the streptomycin adenyltransferase gene.

### Construction of pMON20409

An *Nco*I site was created at the 5' end of the K1-3 angiostatin DNA from plasmid pMON20408 by PCR using Stratagene's *Pfu* DNA polymerase and synthetic oligonucleotides HANGECSKI (SEQ ID NO. 21) and ANGECN3 (SEQ ID NO. 22). The PCR reaction was then treated with Taq polymerase (Boehringer Mannheim, Indianapolis, IN), extracted with phenol/chloroform and ligated into the pCR2.1 vector using the TA cloning kit from Invitrogen (San Diego, CA). A portion of the ligation reaction was used to transform competent *E. coli* K-12 strain INVα F'. Transformant bacteria were selected on ampicillin-containing plates. Plasmid DNA was isolated, analyzed by restriction analysis, and sequenced to confirm the correct insert.

### Construction of pMON20410

Construction of pMON20410, an intermediate plasmid used for constructing plasmids containing DNA sequences encoding fusion proteins. pMON20410 encodes an IL-3 variant fused 5' to K1-3 angiostatin with the Gly Ser polypeptide linker. The 3551 bp *Afl*III, *Hind*III restriction fragment from pMON31250 was ligated with the 812bp *Nco*I, *Hind*III restriction fragment from pMON20409. A portion of the ligation reaction was used to transform competent *E. coli* K-12 strain JM101. Transformed bacteria were selected on spectinomycin-containing plates. Plasmid DNA was isolated from a colony grown in LB broth containing spectinomycin and was analyzed by restriction analysis and sequenced to confirm the correct insert. The genetic elements derived from the intermediate plasmid pMON20410 are the pBR327 origin of replication, the recA promoter, the gene 10 leader (g10-L) ribosome binding site, an IL-3 variant joined to human angiostatin K1-3 with the GlySer polypeptide linker, the T7 transcription terminator and the streptomycin adenyltransferase gene.

### Construction of pMON20411

The 3552 bp Aflll/*Hind*III restriction fragment from pMON31250 was ligated with the 507 bp *Nco*I/*Hind*III bp fragment from pMON20407. A portion of the ligation reaction was used to transform competent *E. coli* K-12 strain JM101. Transformant bacteria were selected on spectinomycin-containing plates. Plasmid DNA was isolated from a colony grown in LB broth containing spectinomycin and was analyzed by restriction analysis. The genetic elements derived from the intermediate plasmid pMON20411 are the pBR327 origin of replication, the recA promoter, the gene 10 leader (g10-L) ribosome binding site, IL-3 variant joined to human interferon alpha A/D hybrid with the GlySer polypeptide linker, the T7 transcription terminator and the streptomycin adenyltransferase gene.

### Construction of pMON20412

Construction of pMON20412, which encodes K1-3 angiostatin fused 5' to the interferon alpha A/D hybrid. Plasmid DNA from pMON20411 was digested with the restriction enzymes *Nco*I and *Xma*I resulting in a 3695 bp vector fragment. Plasmid DNA from pMON20409 was digested with *Nco*I and *Mfe*I resulting in a 797 bp fragment that was ligated with an annealed pair of synthetic oligonucleotides K1K3INTU.REQ (SEQ ID NO. 23) and K1K3INTL.REQ (SEQ ID NO. 24) and the vector fragment. A portion of the ligation reaction was used to transform *E. coli* K-12 strain JM101. Transformed bacteria were selected on spectinomycin plates. Plasmid DNA was isolated from a colony grown in LB broth and was analyzed by restriction analysis, and sequenced to confirm the correct insert. Other genetic elements include the pBR327 origin of replication, the recA promoter, the gene 10 leader (g10-L) ribosome binding site, the T7 transcription terminator and the streptomycin adenyltransferase gene.

### Construction of pMON20420

Construction of pMON20420, which encodes interferon alpha A/D hybrid fused 5' to the K1-3 angiostatin with the Gly Ser polypeptide linker. Plasmid DNA from pMON20410 is digested with the restriction enzymes *Nco*I and *Xma*I resulting in a 4000 bp vector fragment. Plasmid DNA from pMON20407 is digested with *Nco*I and AccI resulting in a 467 bp fragment that is ligated with an annealed pair of synthetic oligonucleotides IFADINTU.REQ (SEQ ID NO. 25) and IFADINTL.REQ (SEQ ID NO. 26) and the vector fragment. A portion of the ligation reaction is used to transform *E. coli* K-12 strain JM101. Transformant bacteria are selected on spectinomycin-containing plates. Plasmid DNA was isolated from a colony grown in LB broth and was analyzed by restriction analysis, and sequenced to confirm the correct insert. Other genetic elements include the pBR327 origin of replication, the recA promoter, the gene 10 leader (g10-L) ribosome binding site, the T7 transcription terminator and the streptomycin adenyltransferase gene.

### Construction of pMON20421

Construction of pMON20421, which encodes K1 angiostatin fused 5' to the interferon alpha A/D hybrid with the Gly Ser polypeptide linker. The 3480 bp Aflll/*Hind*III restriction fragment from pMON31251 is ligated with the 507 bp *Nco*I, *Hind*III bp fragment from pMON20407. A portion of the ligation reaction is used to transform competent *E. coli* K-12 strain JM101. Transformant bacteria are selected on spectinomycin-containing plates. Plasmid DNA was isolated from a colony grown in LB broth containing spectinomycin and analyzed by restriction analysis. Other genetic elements include the pBR327 origin of replication, the recA promoter, the gene 10 leader (g10-L) ribosome binding site, the T7 transcription terminator and the streptomycin adenyltransferase gene.

### Construction of pMON20422

Construction of pMON20422, which encodes interferon alpha A/D hybrid fused 5' to the K1 angiostatin with the Gly Ser polypeptide linker. Plasmid DNA from pMON31252 is digested with the restriction enzymes *Nco*I and *Xma*I resulting in a 3391 bp vector fragment. Plasmid DNA from pMON20407 is digested with *Nco*I and AccI resulting in a 467 bp fragment that is ligated with an annealed pair of synthetic oligonucleotides IFADINTU.REQ (SEQ ID NO. 25) and IFADINTL.REQ (SEQ ID NO. 26) and the vector fragment. A portion of the ligation reaction is used to transform competent *E. coli* K-12 strain JM101. Transformed bacteria were selected on spectinomycin plates. Plasmid DNA was isolated from a colony grown in LB broth and is analyzed by restriction analysis, and sequenced to confirm the correct insert. Other genetic elements include the pBR327 origin of replication, the recA promoter, the gene 10 leader (g10-L) ribosome binding site, the T7 transcription terminator and the streptomycin adenyltransferase gene.

### Construction of pMON31250

pMON31250, an intermediate plasmid, was used for the construction of plasmids containing DNA sequences encoding multi-functional proteins. It encodes an IL-3 variant fused 5' to the GlySer polypeptide linker, and contains *Afl*III and *Hind*III cloning sites at the 3' end of the polypeptide linker. The 3159 bp *Nco*I, *Hind*III restriction fragment from pMON31236 was ligated with the 413 bp NcoI, *Hind*III fragment from pMON13180. A portion of the ligation reaction was used to transform competent *E. coli* K-12 strain JM101. Transformant bacteria were selected on spectinomycin-containing plates. Plasmid DNA was isolated from a colony grown in LB broth containing spectinomycin and analyzed by restriction analysis. Other genetic elements include the pBR327 origin of replication, the recA promoter, the gene 10 leader (g10-L) ribosome binding site, the T7 transcription terminator and the streptomycin adenyltransferase gene.

### Example 3. Creation of angiostatin / interferon alpha 2b chimeras

### Construction of pMON20413

Construction of plasmid pMON20413, which encodes K1 angiostatin fused 5' to the interferon alpha 2b sequence. Plasmid pMON31251 was digested with restriction enzymes *Afl*III and *Hind*III, resulting in a 3477 bp *Afl*III/*Hind*III vector fragment. Plasmid pMON30426 which encodes interferon alpha 2b, was digested with *Nco*I and *Hind*III, resulting in release of a 507 bp fragment. The restriction fragments were ligated and the ligation reaction mixture was used to transform *E. coli* strain JM101. Transformant bacteria were selected on spectinomycin-containing plates. Plasmid DNA was isolated from colonies and sequenced to confirm the correct insert.

### Construction of pMON20414

Construction of pMON20414, which encodes interferon alpha 2b fused 5' to the K1 angiostatin sequence. Plasmid pMON31252 was digested with the restriction enzymes *Nco*I and *Xma*I, resulting in a 3447 bp *Nco*I/*Xma*I vector fragment. Plasmid pMON30426, which encodes interferon alpha 2b, was digested with the restriction enzymes *Nco*I and *Bsp*HI, resulting in a 447 bp *Nco*I/*Bsp*HI fragment. The 447 bp *Nco*I/*Bsp*HI fragment from pMON30426 was ligated for 3 h with the following pair of annealed complementary oligonucleotides:
Oligo #IFNAintU (SEQ ID NO. 6)
   CATGAGATCT TTTTCTTTGT CAACAAACTT GCAAGAAAGT TTAAGAAGTA AGGAATACGT 60
   AGAGGGCGGT GGAGGCTCC 79
Oligo #IFNAintL (SEQ ID NO. 5)
   CCGGGGAGCC TCCACCGCCC TCTACGTATT CCTTACTTCT TAAACTTTCT TGCAAGTTTG 60
   TTGACAAAGA AAAAGATCT 79
The assembled oligonucleotides create *Bsp*HI and *Xma*I restriction ends and remove the termination codons from the interferon alpha 2b sequence. This fuses the interferon alpha 2b sequence 5' to the GlySer linker sequence (SEQ ID NO. 79). After 3 h ligation, the mixture was added to the 3447 bp *Nco*I, *Xma*I pMON31252 DNA fragment and the ligation continued for 15 h. This was used to transform *E. coli* strain JM101. Transformant bacteria were selected on spectinomycin-containing plates. Plasmid DNA was isolated from colonies and sequenced to confirm the correct insert.

### Construction of pMON20416

Construction of pMON20416, which encodes K1-K3 angiostatin fused 5' to the interferon alpha 2b sequence. Plasmid pMON31259 was digested with restriction enzymes *Afl*III and *Hind*III, resulting in a 4017 bp *Afl*III, *Hind*III vector fragment. Plasmid pMON30426 which encodes interferon alpha 2b, was digested with *Nco*I and *Hind*III, resulting in a 507 bp *Nco*I, *Hind*III fragment. The restriction fragments were ligated and the ligation reaction mixture was used to transform *E. coli* K-12 strain JM101. Transformed bacteria were selected on spectinomycin-containing plates. Plasmid DNA was isolated from colonies and sequenced to confirm the correct insert.

### Construction of pMON31251

Construction of pMON31251, an intermediate plasmid used for constructing plasmids containing DNA sequences encoding fusion proteins. pMON31261 encodes K1 angiostatin fused 6' to the GlySer linker sequence, and contains *Afl*III and *Hind*III cloning sites at the 3' end of the insert DNA. Plasmid pMON31250 was digested with restriction enzymes *Nco*I and *Xma*I, resulting in a 3208 bp *Nco*I, *Xma*I vector fragment. Plasmid pMON24649 which encodes the K1 domain of angiostatin was digested with *Nco*I and *Ear*l, resulting in a 255 bp *Nco*I/*Ear*I fragment. The 255 bp *Nco*I/*Ear*I fragment from pMON24649 was ligated for 3 h with the following pair of annealed complementary oligonucleotides:
Oligo #KlintU (SEQ ID NO. 8)
   GTGTGAAGAG GAATACGTAG AGGGCGGTGG AGGCTCC 37
Oligo #KlintL (SEQ ID NO. 7)
   CCGGGGAGCC TCCACCGCCC TCTACGTATT CCTCTTCA 38
The assembled oligonucleotides create *Ear*I and *Xma*I restriction ends and removes the termination codons from the angiostatin K1 sequence. This fuses the angiostatin K1 sequence to the GlySer linker sequence (SEQ ID NO. 79). After 3 h ligation, the mixture was added to the 3208 bp *Nco*I, *Xma*I pMON31250 DNA fragment and the ligation continued for 15 h. This was used to transform *E. coli* K-12 strain JM101. Transformant bacteria were selected on spectinomycin-containing plates. Plasmid DNA was isolated from colonies and sequenced to confirm the correct insert.

### Construction of pMON31252

Construction of pMON31252, an intermediate plasmid used for constructing plasmids containing DNA sequences encoding fusion proteins. pMON31252 encodes K1 angiostatin fused 3' to the GlySer coding sequence (SEQ ID NO. 79). Plasmid pMON31250 was digested with restriction enzymes *Afl*III and *Hind*III, resulting in a 275 bp *Afl*III, *Hind*III vector fragment. Plasmid pMON24649 was digested with the restriction enzymes *Nco*I and *Hind*III, resulting in a 3552 bp *Nco*I/*Hind*III fragment. The restriction fragments were ligated and the ligation reaction mixture was used to transform *E. coli* K-12 strain JM101. Transformant bacteria were selected on spectinomycin-containing plates. Plasmid DNA was isolated from colonies and sequenced to confirm the correct insert.

### Construction of pMON31259

Construction of pMON31259, an intermediate plasmid used for constructing plasmids containing DNA sequences encoding fusion proteins. pMON31259 encodes domains K1 through K3 of angiostatin fused 5' to the GlySer linker sequence (SEQ ID NO. 79 ), and contains *Afl*III and *Hind*III cloning sites at the 3' end of the insert DNA. Plasmid pMON31250 was digested with restriction enzymes *Nco*I and *Xma*I, resulting in a 3208 bp *Nco*I, *Xma*I vector fragment. Plasmid pMON20409 was digested with *Nco*I and *Mfe*I, resulting in release of a 798 bp *Nco*I/*Mfe*I fragment. The 798 bp *Nco*I/*Mfe*I fragment from pMON20409 was ligated for 3 h with the following pair of annealed complementary oligonucleotides:
Oligo #K1K3intU (SEQ ID NO. 10)
   AATTGGCTTA CGTAGAGCCC GGTGGAGGCT CC 32
Oligo #K1K3intL (SEQ ID NO. 9)
   CCGGGGAGCC TCCACCGCCC TCTACGTAAG CC 32
The assembled oligonucleotides create *Mfe*I and *Xma*I sites and remove the termination codons from the K1-K3 angiostatin sequence. This fuses the angiostatin K1-K3 sequence to the GlySer linker sequence. After 3 h ligation, the mixture was added to the 3208 bp *Nco*I, *Xma*I pMON31250 DNA fragment and the ligation continued for 15 h. This was used to transform *E. coli* K-12 strain JM101. Transformant bacteria were selected on spectinomycin-containing plates. Plasmid DNA was isolated from colonies and sequenced to confirm the correct insert.

### Example 4. Construction of plasmids encoding Kringle domains of angiostatin

### Construction of pMON24624

A cDNA encoding the heavy chain of human plasminogen (pMON24624, SEQ ID NO: 37) was synthesized by polymerase chain reaction (PCR) using the oligonucleotide primers hplgn-s1 (SEQ ID NO: 104) and hplgn-n1 (SEQ ID NO: 105), and using cDNA synthesized from human liver mRNA as a template. This cDNA encodes the signal peptide, N-terminal peptide and the five Kringle domains of human plasminogen. The resulting DNA was cleaved with the restriction endonuclease *Bam*HI and ligated into the mammalian expression vector pMON3360B, which had been similarly cleaved. A portion of the ligation reaction was used to transform *E. coli* strain DH10B. Plasmid DNA was isolated from ampicillin-resistant bacterial colonies and analyzed by restriction endonuclease cleavage and DNA sequencing to confirm the identity of the cDNA.

### Construction of pMON24625

A cDNA encoding the first four Kringle domains (K1-4) of human plasminogen (24625.seq, SEQ ID NO: 38) was synthesized by polymerase chain reaction (PCR) using the oligonucleotide primers angpcr-s1 (SEQ ID NO: 106) and angpcr-n1 (SEQ ID NO: 107). The resulting DNA was cleaved with the restriction endonucleases *Nco*I and *Hin*dIII and ligated into the mammalian expression vector pMON3633 which had been similarly cleaved. A portion of the ligation reaction was used to transform *E. coli* strain DH10B. Plasmid DNA was isolated from ampicillin-resistant bacterial colonies and analyzed by restriction endonuclease cleavage and DNA sequencing to confirm the identity of the cDNA. The resulting protein expressed from pMON24625 includes the human interleukin 3 signal peptide (encoded by vector sequences) fused at the amino terminus of the plasminogen Kringle domains in such a way as to insure secretion of the protein product.

### Construction of pMON24626

A cDNA encoding the first three Kringle domains (K1-3) of human plasminogen (24626.seq, SEQ ID NO: 39) was synthesized by polymerase chain reaction (PCR) using the oligonucleotide primers angpcr-s1 (SEQ ID NO: 106) and angpcr-n2 (SEQ ID NO: 108). The resulting DNA was cleaved with the restriction endonucleases *Nco*I and *Hin*dIII and ligated into the mammalian expression vector pMON3633 which had been similarly cleaved. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity of the cDNA. The resulting protein expressed from pMON24626 includes the human interleukin 3 signal peptide (encoded by vector sequences) fused at the amino terminus of the plasminogen Kringle domains in such a way as to insure secretion of the protein product.

### Construction of pMON24627

A cDNA encoding Kringle domains two through four (K2-4) of human plasminogen (24627.seq, SEQ ID NO: 40) was synthesized by polymerase chain reaction (PCR) using the oligonucleotide primers angpcr-s2 (SEQ ID NO: 109) and angpcr-n1 (SEQ ID NO:107). The resulting DNA was cleaved with the restriction endonucleases *Nco*I and *Hin*dIII and ligated into the mammalian expression vector pMON3633 which had been similarly cleaved. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity of the cDNA. The resulting protein expressed from pMON24627 includes the human interleukin 3 signal peptide (encoded by vector sequences) fused at the amino terminus of the plasminogen Kringle domains in such a way as to insure secretion of the protein product.

### Construction of pMON24628

A cDNA encoding Kringle domains two and three (K2-3) of human plasminogen (24628.seq, SEQ ID NO: 41) was synthesized by polymerase chain reaction (PCR) using the oligonucleotide primers angpcr-s2 (SEQ ID NO: 109) and angpcr-n2 (SEQ ID NO: 108). The resulting DNA was cleaved with the restriction endonucleases *Nco*I and *Hin*dIII and ligated into the mammalian expression vector pMON3633 which had been similarly cleaved. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity of the cDNA. The resulting protein expressed from pMON24628 includes the human interleukin 3 signal peptide (encoded by vector sequences) fused at the amino terminus of the plasminogen Kringle domains in such a way as to insure secretion of the protein product.

### Construction of pMON24630

Plasmid pMON24625 was cleaved with restriction endonuclease *Bam*HI. The 1161 bp cDNA released by such digestion encodes the first four Kringle (K1-4) domains of human plasminogen fused to the human interleukin 3 signal peptide (24625.seq, SEQ ID NO: 38). The baculovirus expression vector pFastBac1 was cleaved with *Bam*HI and digested with Shrimp Alkaline Phosphatase to prevent recircularization. The cleaved vector and the cDNA were ligated together. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity of the cDNA.

### Construction of pMON24631

Plasmid pMON24626 was cleaved with restriction endonuclease *Bam*HI. The 873 bp cDNA released by such digestion encodes the first three Kringle domains (K1-3) of human plasminogen fused to the human interleukin 3 signal peptide (24626.seq, SEQ ID NO: 39). The baculovirus expression vector pFastBac1 was cleaved with *Bam*HI and digested with Shrimp Alkaline Phosphatase to prevent recircularization. The cleaved vector and the cDNA were ligated together. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity of the cDNA.

### Construction of pMON24632

Plasmid pMON24625 was cleaved with restriction endonuclease *Bam*HI. The 1161 bp cDNA released by such digestion encodes the first four Kringle (K1-4) domains of human plasminogen fused to the human interleukin 3 signal peptide (24625.seq, SEQ ID NO: 38). The *Pichia pastoris* expression vector pPIC3 was cleaved with *Bam*HI and digested with Shrimp Alkaline Phosphatase to prevent recircularization. The cleaved vector and the cDNA were ligated together. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity of the cDNA.

### Construction of pMON24633

Plasmid pMON24626 was cleaved with restriction endonuclease *Bam*HI. The 873 bp cDNA released by such digestion encodes the first three Kringle domains (K1-3) of human plasminogen fused to the human interleukin 3 signal peptide (24626.seq, SEQ ID NO: 41). The *Pichia pastoris* expression vector pPIC3 was cleaved with *Bam*HI and digested with Shrimp Alkaline Phosphatase to prevent recircularization. The cleaved vector and the cDNA were ligated together. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity of the cDNA.

### Construction of pMON24636

Plasmid pMON24627 was cleaved with restriction endonuclease *Bam*HI. The 909 bp cDNA released by such digestion encodes Kringle domains two through four (K2-4) of human plasminogen fused to the human interleukin 3 signal peptide (24627.seq, SEQ ID NO: 40). The baculovirus expression vector pFastBac1 was cleaved with *Bam*HI and digested with Shrimp Alkaline Phosphatase to prevent recircularization. The cleaved vector and the cDNA were ligated together. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity.

### Construction of pMON24637

Plasmid pMON24628 was cleaved with restriction endonuclease *Bam*HI. The 621 bp cDNA released by such digestion encodes Kringle domains two and three (K2-3) of human plasminogen fused to the human interleukin 3 signal peptide (24628.seq, SEQ ID NO: 41). The baculovirus expression vector pFastBac1 was cleaved with *Bam*HI and digested with Shrimp Alkaline Phosphatase to prevent recircularization. The cleaved vector and the cDNA were ligated together. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity.

### Construction of pMON24638

Plasmid pMON24627 was cleaved with restriction endonuclease *Bam*HI. The 909 bp cDNA released by such digestion encodes Kringle domains two through four (K2-4) of human plasminogen fused to the human interleukin 3 signal peptide (24627.seq, SEQ ID NO: 40). The *Pichia pastoris* expression vector pPIC3 was cleaved with *Bam*HI and digested with Shrimp Alkaline Phosphatase to prevent recircularization. The cleaved vector and the cDNA were ligated together. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity of the cDNA.

### Construction of pMON24639

Plasmid pMON24628 was cleaved with restriction endonuclease *Bam*HI. The 621 bp cDNA released by such digestion encodes Kringle domains two and three (K2-3) of human plasminogen fused to the human interleukin 3 signal peptide (24628.seq, SEQ ID NO: 41). The *Pichia pastoris* expression vector pPIC3 was cleaved with *Bam*HI and digested with Shrimp Alkaline Phosphatase to prevent recircularization. The cleaved vector and the cDNA were ligated together. A portion of the ligation reaction was used to transform E. coli strain DH10B to ampicillin resistance. Plasmid DNA was isolated from bacterial colonies and analyzed by restriction endonuclease cleavage and DNA sequencing to confirm the identity of the cDNA.

### Construction of pMON24642

A cDNA encoding the first four Kringle domains (K1-4*) of human plasminogen (24642.seq, SEQ ID NO: 42) was synthesized by polymerase chain reaction (PCR) using the oligonucleotide primers angec-s1 (SEQ ID NO: 103) and angec-n1 (SEQ ID NO: 100). The resulting DNA was cleaved with the restriction endonucleases *Afl*III and *Hin*dIII and ligated into the *E. coli* expression vector pMON5723 which had been cleaved with *Nco*I and *Hin*dIII. A portion of the ligation reaction was used to transform *E. coli* strain DH10B to spectinomycin resistance. In order to optimize expression in *E. coli,* the 5' PCR primer (angec-s1) contained degeneracies at the third positions of the first few codons. This resulted in a population of plasmids, each encoding the same protein, but with different silent mutations in some of the first few codons. Plasmids were isolated from the DH10B host strain and used to transform *E. coli* strain MON105 to spectinomycin resistance. Individual isolates were screened for protein expression. Plasmid DNA was isolated from bacterial colonies and analyzed by restriction endonuclease cleavage and DNA sequencing to confirm the identity of the cDNA. The protein encoded by pMON24642 is a somewhat longer version of the K1-4 encoded by the cDNA in pMON24643.

### Construction of pMON24643

A cDNA encoding the first four Kringle domains (K1-4) of human plasminogen (24643.seq, SEQ ID NO: 43) was synthesized by polymerase chain reaction (PCR) using the oligonucleotide primers angec-s2 (SEQ ID NO: 102) and angec-n2 (SEQ ID NO: 99). The resulting DNA was cleaved with the restriction endonucleases *Afl*III and *Hin*dIII and ligated into the *E. coli* expression vector pMON5723 which had cleaved with *Nco*I and *Hin*dIII. A portion of the ligation reaction was used to transform *E. coli* strain DH10B to spectinomycin resistance. In order to optimize expression in *E. coli,* the 5' PCR primer (angec-s2) contained degeneracies at the third positions of the first few codons. This resulted in a population of plasmids, each encoding the same protein, but with different silent mutations in some of the first few codons. Plasmids were isolated from the DH10B host strain and used to transform *E. coli* strain MON105 to spectinomycin resistance. Individual isolates were screened for protein expression. Plasmid DNA was isolated from bacterial colonies and analyzed by restriction endonuclease cleavage and DNA sequencing to confirm the identity of the cDNA.

### Construction of pMON24644

A cDNA encoding domains two and three (K2-3) of human plasminogen (24644.seq, SEQ ID NO: 44) was synthesized by polymerase chain reaction (PCR) using the oligonucleotide primers angec-s3 (SEQ ID NO: 101) and angec-n3 (SEQ ID NO: 98). The resulting DNA was cleaved with the restriction endonucleases *Afl*III and *Hin*dIII and ligated into the *E. coli* expression vector pMON5723 which had cleaved with *Nco*I and *Hin*dIII. A portion of the ligation reaction was used to transform *E. coli* strain DH10B to spectinomycin resistance. In order to optimize expression in *E. coli,* the 5' PCR primer (angec-s3 SEQ ID NO: 101) contained degeneracies at the third positions of the first few codons. This resulted in a population of plasmids, each encoding the same protein, but with different silent mutations in some of the first few codons. Plasmids were isolated from the DH10B host strain and used to transform *E. coli* strain MON105 to spectinomycin resistance. Individual isolates were screened for protein expression. Plasmid DNA was isolated from bacterial colonies and analyzed by restriction endonuclease cleavage and DNA sequencing to confirm the identity of the cDNA.

### Construction of pMON24645

A cDNA encoding Kringle domains two through four (K2-4) of human plasminogen (24645.seq, SEQ ID NO: 45) was synthesized by polymerase chain reaction (PCR) using the oligonucleotide primers angec-s3 (SEQ ID NO: 101) and angec-n2 (SEQ ID NO: 99). The resulting DNA was cleaved with the restriction endonucleases *Afl*III and *Hin*dIII and ligated into the *E. coli* expression vector pMON5723 which had cleaved with *Nco*I and *Hin*dIII. A portion of the ligation reaction was used to transform *E. coli* strain DH10B to spectinomycin resistance. In order to optimize expression in *E. coli,* the 5' PCR primer (angec-s3) contained degeneracies at the third positions of the first few codons. This resulted in a population of plasmids, each encoding the same protein, but with different silent mutations in some of the first few codons. Plasmids were isolated from the DH10B host strain and used to transform *E. coli* strain MON105 to spectinomycin resistance. Individual isolates were screened for protein expression. Plasmid DNA was isolated from bacterial colonies and analyzed by restriction endonuclease cleavage and DNA sequencing to confirm the identity of the cDNA.

### Construction of pMON24646

A cDNA encoding the first three Kringle domains (K1-3) of human plasminogen (24646.seq, SEQ ID NO: 46) was synthesized by polymerase chain reaction (PCR) using the oligonucleotide primers angec-s2 (SEQ ID NO: 102) and angec-n3 (SEQ ID NO: 98). The resulting DNA was cleaved with the restriction endonucleases *Afl*III and *Hin*dIII and ligated into the *E. coli* expression vector pMON5723 which had cleaved with *Nco*I and *Hin*dIII. A portion of the ligation reaction was used to transform *E. coli* strain DH10B to spectinomycin resistance. In order to optimize expression in *E. coli,* the 5'PCR primer (angec-s2) contained degeneracies at the third positions of the first few codons. This resulted in a population of plasmids, each encoding the same protein, but with different silent mutations in some of the first few codons. Plasmids were isolated from the DH10B host strain and used to transform *E. coli* strain MON105 to spectinomycin resistance. Individual isolates were screened for protein expression. Plasmid DNA was isolated from bacterial colonies and analyzed by restriction endonuclease cleavage and DNA sequencing to confirm the identity of the cDNA.

### Construction of pMON24648

A cDNA encoding the first Kringle domain (K1) of human plasminogen (24648.seq, SEQ ID NO: 47) was synthesized by polymerase chain reaction (PCR) using the oligonucleotide primers hangec-sk1 (SEQ ID NO: 110) and angec-n3 (SEQ ID NO: 98). The resulting DNA was cleaved with the restriction endonucleases *Nco*I and *Hin*dIII and ligated into the *E. coli* expression vector pMON5723 which had cleaved with *Nco*I and *Hin*dIII. A portion of the ligation reaction was used to transform *E. coli* strain DH10B to spectinomycin resistance. In order to optimize expression in *E. coli,* the 5' PCR primer (hangec-sk1) contained the same nucleotide sequence found in the K1-3 cDNA in pMON24646. Plasmid DNA was isolated from the DH10B host strain and used to transform *E. coli* strain MON105 to spectinomycin resistance. Individual isolates were screened for protein expression. Plasmid DNA was isolated from bacterial colonies and analyzed by restriction endonuclease cleavage and DNA sequencing to confirm the identity of the cDNA.

### Construction of pMON24649

A cDNA encoding the first kringle domain of human angiostatin was synthesized by polymerase chain reaction using the primers Hang-ec-sk1.seq and ang-ec-n4.seq using pMON24646-A7 as template. The resulting DNA was cleaved with the restriction endonucleases *Nco*I and *Hin*dIII and ligated into the *E. coli* expression vector pMON5723 which had cleaved with *Nco*I and *Hin*dIII. A portion of the ligation reaction was used to transform *E. coli* strain DH10B to spectinomycin resistance. Plasmids were isolated from the DH10B host strain and used to transform *E. coli* strain MON105 to spectinomycin resistance. Individual isolates were screened for protein expression. Plasmid DNA was isolated from bacterial colonies and analyzed by restriction endonuclease cleavage and DNA sequencing to confirm the identity of the cDNA.

### Construction of pMON24650

pMON24649 was cleaved with *Nco*I and *Hin*dIII releasing the cDNA encoding the first Kringle (K1) of human plasminogen (24649.seq, SEQ ID NO: 112). The K1 cDNA was ligated into the mammalian cell expression vector pMON3633 which had been similarly cleaved. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity of the cDNA.

### Construction of pMON24652

pMON20412 was cleaved with *Nco*I and *Hin*dIII releasing the cDNA encoding the first three Kringles of human plasminogen fused to the IFN alpha A/D cDNA. The cDNA was ligated into the mammalian cell expression vector pMON3633 which had been similarly cleaved. A portion of the ligation reaction was used to transform *E. coli* strain DH10B to ampicillin resistance. Plasmid DNA was isolated from bacterial colonies and analyzed by restriction endonuclease cleavage and DNA sequencing to confirm the identity of the cDNA.

### Example 5. Purification of angiostatin / interferon alpha 2b chimeras

Plasmid pMON24652, encoding an angiostatin K1-3/interferon A/D chimeric protein, was transfected into BHK/VP16 cells and stable cell lines selected as described above in the materials and methods (Hippenmeyer, P. and Highkin, M. Bio/Technology 11: 1037-1041, 1993). Thirty-six clones were isolated and six clones selected for testing in roller bottles. The concentration of the chimeras were determined by ELISA using a commercially-available human IFN-alpha ELISA kit (Biosource #KHC4012). Clone 15 produced the most chimera, 14.4 ug/ml, while the other five clones produced much less (clones 8, 14, 29, 32, and 36 each produced 1.17, 2.22, 0.04, 0.20, and 1.12 ug/ml, respectively).

A 50 ml lysine sepharose column was pre-equilibrated at 4°C with 50 mM sodium phosphate, pH 7.4. About 500 ml of a 12X concentrate of conditioned serum-free media (SF-BHK) was loaded onto the column at 4°C with a flow rate of about 2.5 ml/min. The column was washed with 50 mM sodium phosphate, pH 7.4, followed by a wash containing 500 mM NaCl in 50 mM sodium phosphate, pH 7.4, to remove non-specific binding proteins. The column was then equilibrated back to a low salt environment with two column volumes of 50 mM sodium phosphate, pH 7.4. The desired chimera was eluted by washing the column with 200 mM epsilon-amino caproic acid in 50 mM sodium phosphate, pH 7.4. Approximately 10.5 ml fractions were collected and the elution monitored by UV absorption. Fractions 4-6 absorbed significant UV and were combined. These fractions were dialyzed three times at 4°C against 4 liters of 50 mM sodium phosphate, pH 7.4, and sterile filtered. Analytical lysine sepharose chromatography yielded a final concentration of about 0.6 mg/ml.

SDS-PAGE analysis of the purified sample on reducing and non-reducing gels indicated that the chimera migrated at a position close to its expected size. Electron spray mass spectrometry indicated a molecular weight of about 51,556 daltons, indicating that the material isolated from BHK cells is partially glycosylated.

The purified chimera was assayed for endothelial cell migration, in triplicate, as described in the materials and methods. The results are shown in the table below:

| **HMEC migration** | |
|---|---|
| **Sample** | **Cells/HPF** |
| Media | 14.33 ± 3.84 |
| VEGF | 190.00 ± 16.46 |
| VEGF + Intron A (500 U/ul) | 111.11 ± 25.47 |
| VEGF + IFN A/D (500 U/ul) | 131.78 ± 19.90 |
| VEGF + IFN A/D (5000 U/ul) | 44.89 ± 18.48 |
| VEGF + Angiostatin K1-K3/IFN A/D (0.3 ug/ul) | 73.22 ± 8.80 |
| VEGF + Angiostatin K1-K3/IFN A/D (30 ug/ul) | 35.11 ± 18.51 |
| VEGF + Angiostatin K1-K3 prepared from *E. coli* (30 ug/ul) | 130.56 ± 7.04 |

Interferon alpha A/D from a commercial source (Genzyme) and the angiostatin/IFN A/D chimera were also tested twice in a Daudi cell proliferation assay using a series of 3-fold dilutions, each beginning at a concentration of 1 nM. The interferon A/D and the angiostatin/interferon A/D chimera had similar inhibition profiles (Figure 5).

All references, patents, or applications cited herein are incorporated by reference in their entirety.

**Tables**

| ***Plasmids*** | | | | |
|---|---|---|---|---|
| **Plasmid** | **SEQ ID NO.** | **Marker** | **Description** | **Source** |
| pFastBac1 | | Amp, Gent | Baculovirus donor plasmid containing multiple cloning site downstream of an AcNPV polyhedrin promoter within a mini-Tn7 transposable element. | Life Technologies, Inc. |
| pPIC3 | | Amp, HIS3 | *Pichia pastoris* expression vector | Invitrogen Company |
| pMON3360 B | | | Mammalian expression vector for use in BHK/VP16 cells | Hippenmeyer, P. and Highkin, M. Bio/Technolog y 11: 1037-1041, 1993 |
| pMON3633 | | | Mammalian expression vector for use in BHK/VP16 cells | This reference |
| pMON5723 | | Spec | Generic Spec-resistant *E. coli* expression vector containing the *E. coli rec*A1 promoter and G10L ribosome binding site | |
| pMON6875 | | Spec | Generic Spec-resistant *E. coli* expression vector containing the *E. coli rec*A1 promoter and G10L ribosome binding site | |
| pMON13180 | | Amp | Intermediate plasmid containing an IL-3 variant fused to a Gly-Ser polypeptide linker (SEQ ID NO: 79). Contains an *Nco*I site at the 5' end of the IL-3 variant coding sequences and *Afl*III and *Hin*dIII cloning sites at the 3' end of the linker region. | WO 97/12985 |
| pMON20405 | | Spec | 3207 bp *Nco*I/*Hind*III frag from pMON30426 + 192 bp frag from pMON30426 (A portion of IFN alpha) + 315 bp frag from 8 synthetic oligos with *Bgl*II/*Hind*III ends (D portion of IFN alpha). Creates IFN alpha A/D hybrid. | This reference |
| pMON20407 | | Spec | 3159 bp *Nco*I/*Hind*III frag from pMON31236 + 507 bp frag from pMON20405 (IFN alpha A/D hybrid) | This reference |
| pMON20408 | | Spec | Removed *Nco*I site in K3 region of angiostatin K1-K3 in pMON24646-A7 | This reference |
| pMON20409 | | Spec | Created *Nco*I site at the 5' end of K1-K3 angiostatin | This reference |
| pMON20410 | | Spec | 3551 bp *Af*lIII, *Hind*III fragment from pMON31250 + 812 bp frag from pMON20409 (creates chimera IL-3 variant K1-K3 angiostatin) | This reference |
| pMON20411 | | Spec | 3551 bp *Afl*III, *Hind*III frag from pMON31250 + 507 bp frag from pMON20407 (creates chimera IL-3 variant/IFN A/D hybrid) | This reference |
| pMON20412 | | Spec | 3695 bp *Nco*I/*Xma*I frag from pMON20411 + 797 bp *Nco*I/*Mfe*I frag from pMON20409 + 1 pair of synthetic oligonucleotides with *Mfe*I/*Xma*I ends (creates K1-K3/IFN A/D hybrid chimera) | This reference |
| pMON20413 | #35 | Spec | 3447 bp *Afl*III/*Hin*dIII fragment from pMON31251 + 507 bp *Nco*I/*Hin*dIII fragment of pMON30426 (creates K1-GlySer-IFN a2b hybrid) | This reference |
| pMON20414 | #31 | Spec | 3447 *Nco*I/*Xma*I from pMON312b2 + 447 bp *Nco*I/*Bsp*HI frag from pMON30426 + synthetic oligos with *Bsp*HI/*Xma*I ends (creates IFN a2b-GlySer-K1 hybrid) | This reference |
| pMON20416 | #33 | Spec | 4017 bp *Afl*III/*Hin*dIII vector from pMON31259 + 507 bp *Nco*I/*Hin*dIII fragment from pMON30426 (creates K1-K3-IFN a2b hybrid) | This reference |
| pMON20420 | #30 | Spec | 4000 bp *Nco*I/*Xma*I frag from pMON20410 + 467 bp *Nco*I, AccI frag from pMON20407 + 1 pair of synthetic oligonucleotides with AccI/*Xma*I ends (creates IFN A/D hybrid/K1-K3 angiostatin chimera | This reference |
| pMON20421 | #34 | Spec | 3480 bp *Afl*III/*Hind*III frag from pMON31251 + 507 bp *Nco*I/*Hind*III frag from pMON20407 creates a chimera of K1/IFN A/D hybrid | This reference |
| pMON20422 | #29 | Spec | 3491 bp *Nco*I/*Xma*I frag from pMON31252 + 467 *Nco*I/AccI bp frag from pMON20407 + 1 pair of synthetic oligonucleotides with AccI/*Xma*I ends (creates IFN A/D hybrid/K1 angiostatin chimera) | This reference |
| pMON24624 | #37 | Amp | Human plasminogen heavy chain including the native plasminogen signal sequence, N-terminal peptide and kringles 1-5. *Bam*HI fragment in pMON3360B. (Amino acids - 19-537 of plasminogen, nucleotides 55-1752) | This reference |
| pMON24625 | #38 | Amp | Human angiostatin(S) including kringles 1-4 of hPlgn. *Afl*III/*Hind*III fragment in pMON3633. Seq file includes hIL3 signal sequence contributed by the vector. (Amino acids 79-440 of plasminogen, nucleotides 346-1431) | This reference |
| pMON24626 | #39 | Amp | K1-3 fragment of human angiostatin(S) including kringles 1-3 of hPlgn. *Afl*III/*Hind*III fragment in pMON 3633. Seq file includes hIL3 signal sequence contributed by the vector. (Amino acids 79-344 of plasminogen, nucleotides 346-1143) | This reference |
| pMON24627 | #40 | Amp | K2-4 fragment of human angiostatin (S) including kringles 2-4 of hPlgn. *Afl*III/*Hind*III fragment in pMON3633. Seq file includes hIL3 signal sequence contributed by the vector. (Amino acids 163-440 of plasminogen, nucleotides 598-1431) | This reference |
| pMON24628 | #41 | Amp | K2-3 fragment of human angiostatin(S) including kringles 2-3 of hPlgn. *Afl*III/*Hind*III fragment in pMON3633. Seq file includes hIL3 signal sequence contributed by the vector. (Amino acids 163-344 of plasminogen, nucleotides 598-1143) | This reference |
| pMON24630 | | Amp | K1-4 cDNA (*Bam*HI fragment) from pMON24625 cloned into baculovirus transfer vector pFastBacl. | This reference |
| pMON24631 | | Amp | K1-3 cDNA (*Bam*HI fragment) from pMON24626 cloned into baculovirus transfer vector pFastBac1. | This reference |
| pMON24632 | | Amp, HIS3 | K1-4 cDNA (*Bam*HI fragment) from pMON24625 cloned into *Pichia pastoris* expression vector pPIC3. | This reference |
| pMON24633 | | Amp, HIS3 | K1-3 cDNA (BamHI fragment) from pMON24626 cloned into *Pichia pastoris* expression vector pPIC3. | This reference |
| pMON24634 | | Kan, Gent | K1-4 cDNA (*Bam*HI fragment) from pMON24625 transposed into the bacmid bMON14272. | This reference |
| pMON24635 | | Kan, Gent | K1-3 cDNA (*Bam*HI fragment) from pMON24626 transposed into the bacmid bMON14272. | This reference |
| pMON24636 | | Amp | K2-4 cDNA (*Bam*HI fragment) from pMON24627 cloned into baculovirus transfer vector pFastBac1. | This reference |
| pMON24637 | | Amp | K2-3 cDNA (*Bam*HI fragment) from pMON24628 cloned into baculovirus transfer vector pFastBac1. | This reference |
| pMON24638 | | Amp, HIS3 | K2-4 cDNA (*Bam*HI fragment) from pMON24627 cloned into *Pichia pastoris* expression vector pPIC3. | This reference |
| pMON24639 | | Amp, HIS3 | K2-3 cDNA (*Bam*HI fragment) from pMON24628 cloned into *Pichia pastoris* expression vector pPIC3. | This reference |
| pMON24640 | | Kan, Gent | K2-4 cDNA (*Bam*HI fragment) from pMON24627 transposed into the bacmid bMON14272. | This reference |
| pMON24641 | | Kan, Gent | K2-3 cDNA (*Bam*HI fragment) from pMON24628 transposed into the bacmid bMON14272. | This reference |
| pMON24642 | #42 | spec | Human angiostatin(E). *Afl*III/*Hind*III fragment in pMON5723. The 5' end has been mutagenized for increased expression. (Amino acids 74-470 of plasminogen, nucleotides 331-1464) | This reference |
| pMON24643 | #43 | spec | Human angiostatin(S) (K1-4) *Afl*III/*Hind*III fragment in pMON5723. (Amino acids 79-440 of plasminogen, nucleotides 346-1431) | This reference |
| pMON24644 | #44 | spec | K2-3 fragment of human angiostatin(S). *Afl*III/*Hind*III fragment in pMON5723. (Amino acids 163-344 of plasminogen, nucleotides 598-1143) | This reference |
| pMON24645 | #45 | spec | K2-4 fragment of human angiostatin(S). *Afl*III/*Hind*III fragment in pMON5723. (Amino acids 163-440 of plasminogen, nucleotides 598-1431) | This reference |
| pMON24646 | #46 | spec | K1-3 fragment of human angiostatin(S). *Afl*III/*Hind*III fragment in pMON5723. (Amino acids 79-344 of plasminogen, nucleotides 346-1143) | This reference |
| pMON24648 | #47 | spec | K1 fragment of human angiostatin(S). *Nco*I/*Hind*III fragment in pMON5723. (Amino acids 79-184 of plasminogen, nucleotides 7 (24644A7.seq) -607) | This reference |
| pMON24649 | #112 | spec | K1 fragment of human angiostatin(S). NcoI/HindIII fragment in pMON5723. (Amino acids 79-184 of plasminogen, nucleotides 1-269 of pMON24646-A7.seq) | This reference |
| pMON24650 | #48 | Amp | K1 fragment of human angiostatin(S). *Nco*I/*Hind*III fragment in pMON3633. (Amino acids 79-184 of plasminogen) | This reference |
| pMON24652 | #49 | Amp | K1-3 fragment of human angiostatin/IFN A/D (*Nco*I/*Hind*III fragment from pMON20412) in pMON3633. | This reference |
| pMON30422 | #27 | spec | Interferon alpha 2b | This reference |
| pMON30426 | #28 | spec | Interferon alpha 2b with optimized N-terminal codons | This reference |
| pMON31236 | | Spec | Similar to pMON5723 with the exception of the *Nco*I/*Hin*dIII insert | This reference |
| pMON31250 | | Spec | 3159 bp *Nco*I/*Hind*III frag from pMON31236 + 413 bp *Nco*I/*Hind*III fragment from pMON13180 (creates an intermediate for making fusion proteins in the new spec vector) | This reference |
| pMON31251 | | Spec | 3208 bp NcoI/XmaI fragment of pMON31250 + 255 NcoI/EarI fragment of pMON24649 + synthetic oligos with EarI/XmaI ends (creates K1-Gly-Ser hybrid intermediate plasmid) | This reference |
| pMON31252 | | Spec | 3552 *Afl*III/*Hin*dIII fragment from pMON31250 + 275 bp *Nco*I/*Hin*dIII fragment from pMON24649 (creates Gly-Ser-angiostatin K1 hybrid) | This reference |
| pMON31259 | | Spec | 3208 bp *Nco*I/*Xma*I fragment of pMON31250 + 798 bp *Nco*I/*Mfe*I fragment of pMON20409 + synthetic oligos with *Mfe*I/*Xma*I ends (creates K1-K3-Gly-Ser hybrid intermediate plasmid) | This reference |

**SEQ ID Correlation table**

| **SEQ ID NO.** | **SEQ ID Name** |
|---|---|
| 1 | Oligo Ifstart |
| 2 | Oligo Ifstop |
| 3 | Oligo NewIF-A |
| 4 | Oligo IFADINTL |
| 5 | Oligo IFNAINTL |
| 6 | Oligo IFNAINTU |
| 7 | Oligo K1NTL |
| 8 | Oligo K1NTU |
| 9 | Oligo K1K3INTL |
| 10 | Oligo K1K3INTU |
| 11 | Oligo INFD1 |
| 12 | Oligo IFND2 |
| 13 | Oligo IFND3X |
| 14 | Oligo IFND4X |
| 15 | Oligo IFND5 |
| 16 | Oligo IFND6 |
| 17 | Oligo IFND7 |
| 18 | Oligo IFND8 |
| 19 | Oligo HPLGN-CD1 |
| 20 | Oligo HPLGN-CD2 |
| 21 | Oligo HANGECSK1 |
| 22 | Oligo ANGECN3 |
| 23 | Oligo K1K3INTU |
| 24 | Oligo KIK3INTL |
| 25 | Oligo IFADINTU |
| 26 | Oligo IFADINTL |
| 27 | 30422.seq |
| 28 | 30426.seq |
| 29 | IFN adk1.seq (20422) |
| 30 | IFN adk1_3.seq (20420) |
| 31 | IFN argk1.seq (20414) |
| 32 | IFN argk1_3.seq ( ) |
| 33 | K1_3IFN arg.seq (20416) |
| 34 | K1IFN ad.seq (20421) |
| 35 | K1IFN arg.seq (20413) |
| 36 | K1-K3IFN ad.seq (20412) |
| 37 | pKON24624.seq |
| 38 | pMON24625_bam.seq |
| 39 | pMON24626_bam.seq |
| 40 | pMON24627.seq |
| 41 | pMON24628.seq |
| 42 | pMON24642_B5.seq |
| 43 | pMON24643-C10.seq |
| 44 | pMON24644-E7.seq |
| 45 | pMON24645-G3.seq |
| 46 | pMON24646-A7.seq |
| 47 | pMON24648_E7.seq |
| 48 | pMON24650.seq |
| 49 | pMON24652.seq |
| 50 | huendostatin.seq |
| 51 | human_plgn.seq |
| 52 | huvasculostatin.seq |
| 53 | 30422.pep |
| 54 | 30426.pep |
| 55 | IFNadk1.pep |
| 56 | IFNadk1_3.pep |
| 57 | IFNargk1.pep |
| 58 | IFNargk1_3.pep |
| 59 | K1_K3IFNarg.pep |
| 60 | K1IFNad.pep |
| 61 | K1IFNarg.pep |
| 62 | K1-K3IFNaD.pep |
| 63 | pMON24624.pep |
| 64 | pMON24625.pep |
| 65 | pMON24626.pep |
| 66 | pMON24627.pep |
| 67 | pHON24628.pep |
| 68 | pMON24642.pep |
| 69 | pMON24643.pep |
| 70 | pMON24644.pep |
| 71 | pMON24645.pep |
| 72 | pMON24646-pep |
| 73 | pMON24648.pep |
| 74 | pMON24650.pep |
| 75 | pMON24652.pep |
| 76 | huendostatin.pep |
| 77 | hplasminogen.pep |
| 78 | Huvasculostatin.pep |
| 79 | Peptide linker, Tyr Val Glu Gly Gly Gly Gly Ser Pro Gly Gly Gly Ser Gly Gly Gly Ser Asn |
| 80 | Peptide linker, Gly Gly Gly Ser |
| 81 | Peptide linker, Gly Gly Gly Ser Gly Gly Gly Ser |
| 82 | Peptide linker, Gly Gly Gly Ser Gly Gly Gly Ser Gly Gly Gly Ser |
| 83 | Peptide linker, Ser Gly Gly Ser Gly Gly Ser |
| 84 | Peptide linker, Glu Phe Gly Asn Met Ala |
| 85 | Peptide linker, Glu Phe Gly Gly Asn Met Ala |
| 6 | Peptide linker, Glu Phe Gly Gly Asn Gly Gly Asn Met Ala |
| 87 | Peptide linker, Gly Gly Ser Asp Met Ala Gly |
| 88 | Peptide linker G3s |
| 89 | Peptide linker G4s |
| 90 | Peptide linker G5s |
| 91 | Peptide linker AGS |
| 92 | Peptide linker G6S |
| 93 | Peptide linker M13 |
| 94 | Peptide linker Hinge1 |
| 95 | Peptide linker Hinge2 |
| 96 | Oligo hang sk1 |
| 97 | Oligo hangec_ec_sk1 |
| 98 | Oligo angec n3 |
| 99 | Oligo angec n2 |
| 100 | Oligo angec n1 |
| 101 | Oligo angec s3 |
| 102 | Oligo angec s2 |
| 103 | Oligo angec s1 |
| 104 | Oligo Hplgn_S1 |
| 105 | Oligo Hplgn_N1 |
| 106 | Oligo angpcr_s1 |
| 107 | Oligo angpcr_n1 |
| 108 | Oligo angpcr_n2 |
| 109 | Oligo angpcr_s2 |
| 110 | Oligo Hang_Ec_Sk1 |
| 111 | Oligo angec_N4 |
| 112 | 24649.seq |
| 113 | 24649.pep |

## Claims

1. A multifunctional protein, comprising an amino acid sequence of the formula:
R₁-L₁-R₂;
R₂-L₁-R₁;
R₁-L₁-R₁;
R₁-R₂;
R₂-R₁;
and
R₁-R₁;
wherein R₁ is angiostatin;
and R₂ is selected from endostatin, human type I interferon,
thrombospondin interferon-inducible protein 10 (IP-10), and platelet factor 4;
and wherein L₁ is a linker capable of linking R₁ to R₂;
and said protein can optionally be immediately preceded by (methionine⁻¹), (alanine⁻¹), (methionine⁻², alanine⁻¹), (serine⁻¹), (methionine⁻², serine⁻¹), (cysteine⁻¹) or (methionine⁻², cysteine⁻¹).

2. The multi-functional protein as recited in claim 1, wherein
R₁ is selected from the group consisting of angiostatin K1, angiostatin K1-K3 angiostatin K1-K4, angiostatin K1-K5, angiostatin K5.

3. The multi-functional protein as recited in claim 1, wherein
said type I interferon is selected from the group consisting of type I interferon variants, interferon alpha 2a, interferon alpha 2b, interferon alpha hybrid A/D, and consensus interferon.

4. The multi-functional protein as recited in claim 1, 2, or 3
wherein said linker (L₁) is one or more peptide sequences selected from the group consisting of;
SEQ ID NO:79, SEQ ID NO:80, SEQ ID NO:81, SEQ ID NO:82, SEQ ID NO:83, SEQ ID NO:84, SEQ ID NO:85, SEQ ID NO:86, and SEQ ID NO:87.

5. The multi-functional protein of claim 1, wherein the amino acid sequence is selected from the group consisting of
SEQ ID NO:53, SEO ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, SEQ ID NO:68, SEQ ID NO.69, SEQ ID NO:70, SEQ ID NO:71 SEQ ID NO:72, SEQ ID NO:73, SEQ ID NO:74, and SEQ ID NO:75.

6. Use of a multi-functional protein fragment of claim 1 for the manufacture of a medicament for therapeutic application to inhibit tumor growth.

7. Use of a multi-functional protein fragment of claim 2 for the manufacture of a medicament for therapeutic application to inhibit tumor growth.

8. Use of a multi-functional protein fragment of claim 3 for the manufacture of a medicament for therapeutic application to inhibit tumor growth.

9. A nucleic acid molecule encoding said multi-functional protein of claim 1.

10. A nucleic acid molecule encoding said multi-functional protein of claim 2

11. A nucleic acid molecule encoding said multi-functional protein of claim 3.

12. A nucleic acid molecule encoding said multi-functional protein of claim 1,
wherein said nucleic acid sequence is selected from the group consisting of
SEQ ID NO:27, SEQ ID NO:28, SEQ ID NO:29, SEQ ID NO:30, SEQ ID NO:31. SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49,
or a combination or a functional homologue thereof.

13. A method of producing a multi-functional protein comprising: growing under suitable nutrient conditions, a host cell transformed or transfected with a replicable vector comprising a nucleic acid molecule of claim 9, 10, 11, or 12 in a manner allowing expression of said multi-functional protein and recovering said multi-functional protein.

14. A pharmaceutical composition comprising a therapeutically effective amount of the multi-functional protein according to claim 1, 2, or 3 and a pharmaceutically acceptable carrier.

15. The pharmaceutical composition of claim 14 further comprising an adjunctive agent, wherein said adjunctive agent is a selected from the group consisting of chemotherapeutic and immunotherapeutic agents.

16. A method of treating a human patient with an angiogenic-mediated disease, comprising administering to the patient with an angiogenic-mediated disease an effective amount of multi-functional protein of claim 1.

17. The method of claim 16, wherein the angiogenic-mediated disease is selected from the group consisting of cancer, diabetic retinopathy, macular degeneration, and arthritis.

18. A method of modulating tumors in a patient comprising the step of; administering an effective amount of the multi-functional protein as recited in claim 1, 2, or 3 to said patient.

19. A method of inhibiting the production of tumor cells in a patient comprising the step of administering an effective amount of the multi-functional protein as recited in claim 1, 2, or 3 to said patient.

20. The method of claim 19, wherein the tumor cell is characteristic of one selected from the group consisting of lung cancer, breast cancer, ovarian cancer, prostate cancer, pancreatic cancer, gastric cancer, colon cancer, renal cancer, bladder cancer, melanoma, hepatoma, sarcoma, and lymphoma.
